# EUROPEAN PATENT APPLICATION

(11) **EP 4 190 772 A1**
(43) Date of publication of application: **07.06.2023**
(21) Application number: 22207948.5
(22) Date of filing: 17.11.2022
(51) Int. Cl.: C07D 307/91, C07C 211/61, C07D 333/76, C07F 7/08, H10K 50/15

(54) **LIGHT EMITTING DEVICE AND AMINE COMPOUND FOR LIGHT EMITTING DEVICE**

(30) Priority: 01.12.2021 KR 20210169655
(71) Applicant: Samsung Display Co., Ltd., Yongin-si, Gyeonggi-do 17113 (KR)
(72) Inventor: Jeong, Eunjae, 17113 Giheung-gu, Yongin-si, Gyeonggi-do (KR); Kim, Minji, 17113 Giheung-gu, Yongin-si, Gyeonggi-do (KR); Park, Hyunbin, 17113 Giheung-gu, Yongin-si, Gyeonggi-do (KR); Lee, Jeongmin, 17113 Giheung-gu, Yongin-si, Gyeonggi-do (KR); Choi, Jiyong, 17113 Giheung-gu, Yongin-si, Gyeonggi-do (KR); Han, Sanghyun, 17113 Giheung-gu, Yongin-si, Gyeonggi-do (KR)
(74) Representative: Dr. Weitzel & Partner

(57) **Abstract**

A light emitting device includes a first electrode, a second electrode disposed on the first electrode, at least one functional layer which is disposed between the first electrode and the second electrode and includes an amine compound represented by Formula 1: 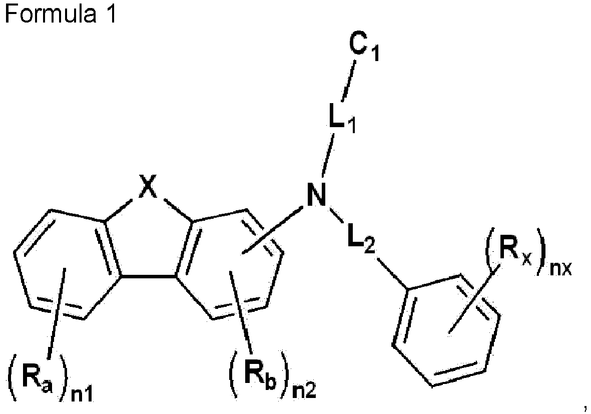 wherein C₁ is a substituted or unsubstituted cycloalkyl group having 6 to 12 ring-forming carbon atoms, and nx is an integer of 2 to 5.

## Description

### BACKGROUND

### 1. Field

The present invention relates to a light emitting device and an amine compound which can be used in a light emitting device, and specifically to an amine compound utilized in a hole transport region and a light emitting device including the same.

### 2. Description of the Related Art

Recently, the development of an organic electroluminescence display apparatus as an image display apparatus is being actively conducted. Unlike liquid crystal display apparatuses and/or the like, the organic electroluminescence display apparatus is a self-luminescent display apparatus in which holes and electrons injected from a first electrode and a second electrode recombine in an emission layer, and thus a luminescent material including an organic compound in the emission layer emits light to implement display of images.

In the application of an organic electroluminescence device to a display apparatus, there is a demand (or desire) for an organic electroluminescence device having low driving voltage, high luminous efficiency, and a long service life, and development of materials for an organic electroluminescence device capable of stably (or suitably) attaining such characteristics is being continuously required (or desired).

It is the object of the present invention to provide an improved material for a hole transport layer in order to realize a highly (or suitably) efficient organic electroluminescence device, and specifically an amine compound and a light emitting device including the same with improved luminous efficiency and improved lifespan characteristics.

### SUMMARY

The object is solved by an amine compound and a light emitting device including the same with the features of claims 1 and 10. The dependent claims describe preferred embodiments.

One or more aspects of embodiments of the present invention are directed toward a light emitting device in which luminous efficiency and a device service life are improved.

One or more aspects of embodiments of the present invention are also directed toward an amine compound capable of improving luminous efficiency and device service life of a light emitting device.

The present invention provides a light emitting device including a first electrode, a second electrode on the first electrode, and at least one functional layer between the first electrode and the second electrode and including an amine compound represented by Formula 1:

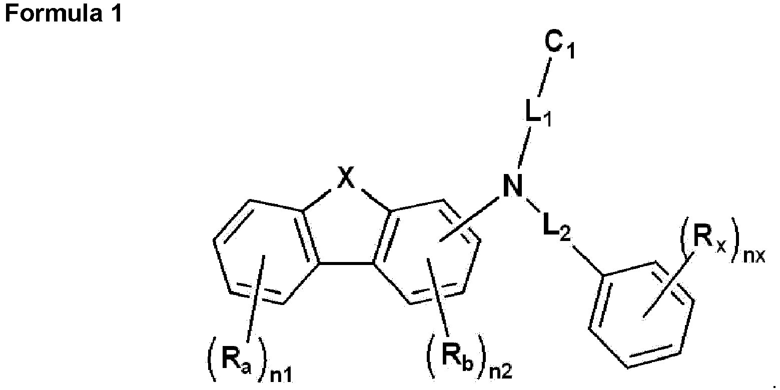

In Formula 1, X is CR₁R₂, O, or S; C₁ is a substituted or unsubstituted cycloalkyl group having 6 to 12 ring-forming carbon atoms; L₁ and L₂ are each independently substituted or unsubstituted arylene group having 6 to 30 ring-forming carbon atoms, or a substituted or unsubstituted heteroarylene group having 2 to 30 ring-forming carbon atoms; R₁, R₂, Rₐ, and R_{b} are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or are bonded to an adjacent group to form a ring; Rₓ is a substituted or unsubstituted silyl group, a substituted or unsubstituted alkyl group having 1 to 20 carbon atoms, a substituted or unsubstituted aryl group having 6 to 30 ring-forming carbon atoms, or a substituted or unsubstituted heteroaryl group 6 to 30 ring-forming carbon atoms; nx is an integer of 2 to 5, n1 is an integer of 0 to 4, and n2 is an integer of 0 to 3.

In one or more embodiments, the at least one functional layer may include an emission layer, a hole transport region between the first electrode and the emission layer, and an electron transport region between the emission layer and the second electrode, and the hole transport region may include the amine compound represented by Formula 1.

In one or more embodiments, the amine compound represented by Formula 1 may be a monoamine compound.

In one or more embodiments, the amine compound represented by Formula 1 may be represented by Formula 1-1:

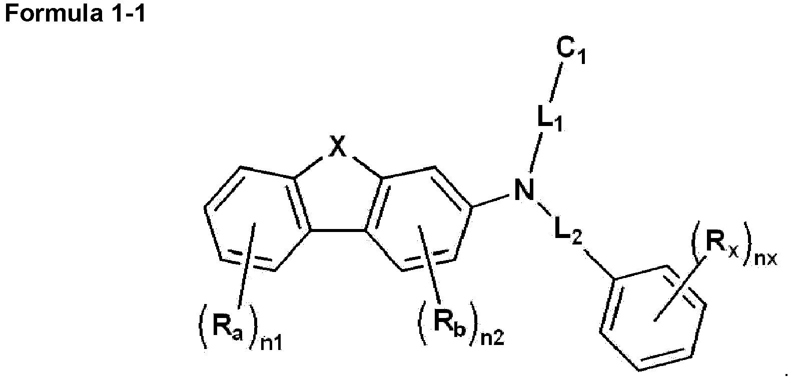

In Formula 1-1, the same definitions as those provided in Formula 1 may be applied to X, C₁, L₁, L₂, R₁, R₂, Rₐ, R_{b}, Rₓ, nx, n1, and n2.

In one or more embodiments, the amine compound represented by Formula 1 may be represented by any one selected from among Formula 2-1 to Formula 2-3:

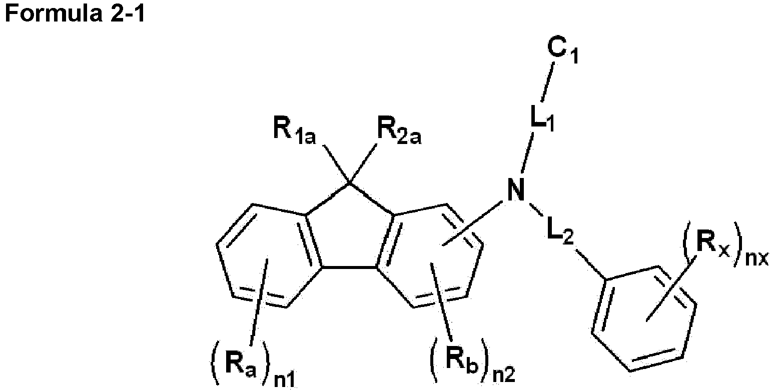

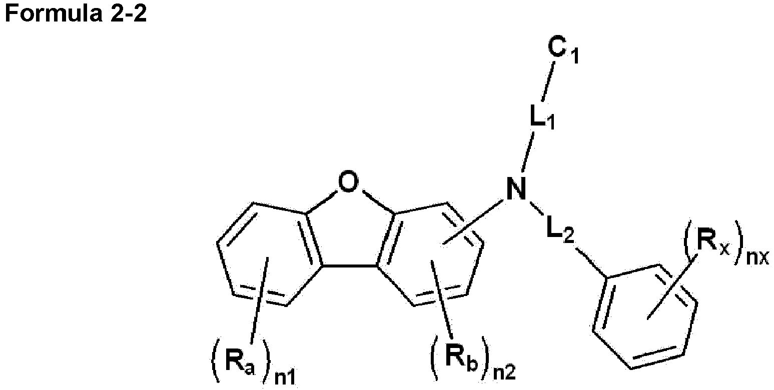

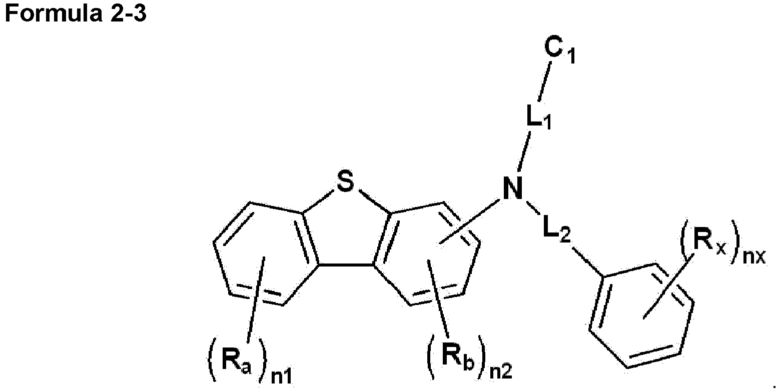

In Formula 2-1 to Formula 2-3, R₁ₐ and R₂ₐ may each independently be a substituted or unsubstituted alkyl group having 1 to 20 carbon atoms, or a substituted or unsubstituted aryl group having 6 to 30 ring-forming carbon atoms, and/or may be bonded to an adjacent group to form a ring.

In Formula 2-1 to Formula 2-3, the same definitions as those provided in Formula 1 may be applied to C₁, L₁, L₂, R₁, R₂, Rₐ, R_{b}, Rₓ, nx, n1, and n2.

In one or more embodiments, the amine compound represented by Formula 1 may be represented by Formula 3:

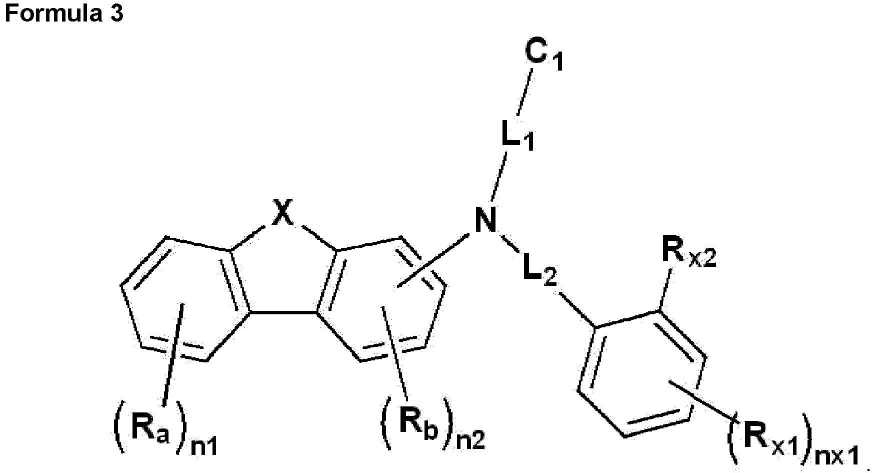

In Formula 3, Rₓ₁ and Rₓ₂ may each independently be a substituted or unsubstituted silyl group, a substituted or unsubstituted alkyl group of 1 to 20 carbon atoms, a substituted or unsubstituted aryl group of 6 to 30 ring-forming carbon atoms, or a substituted or unsubstituted heteroaryl group of 6 to 30 ring-forming carbon atoms, and nx1 is an integer of 1 to 4.

In Formula 3, the same definitions as those provided in Formula 1 may be applied to X, C₁, L₁, L₂, R₁, R₂, Rₐ, R_{b}, n1, and n2.

In one or more embodiments, C₁ may be a substituted or unsubstituted cyclohexyl group, a substituted or unsubstituted bicycloheptanyl group, a substituted or unsubstituted bicyclooctanyl group, a substituted or unsubstituted bicyclononanyl group, or a substituted or unsubstituted adamantyl group.

In one or more embodiments, Rₓ may be a substituted or unsubstituted trimethylsilyl group, a substituted or unsubstituted isopropyl group, a substituted or unsubstituted t-butyl group, a substituted or unsubstituted cyclohexyl group, a substituted or unsubstituted bicycloheptanyl group, a substituted or unsubstituted adamantyl group, a substituted or unsubstituted phenyl group, a substituted or unsubstituted biphenyl group, or a substituted or unsubstituted naphthyl group.

In one or more embodiments, each of L₁ and L₂ may be represented by any one selected from among Formula 4-1 to Formula 4-10:

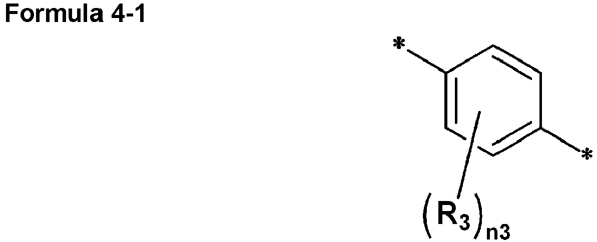

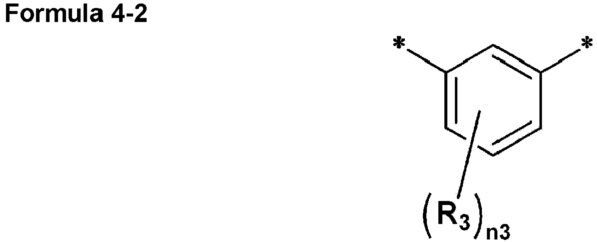

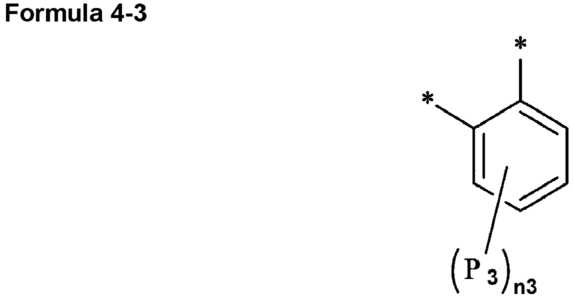

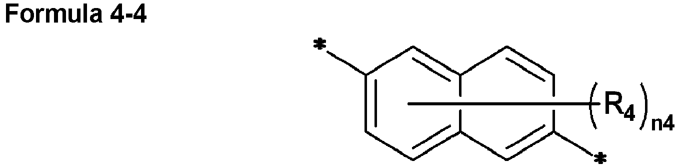

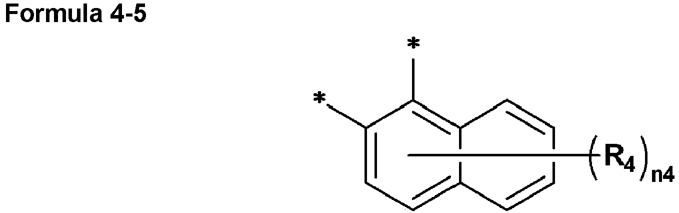

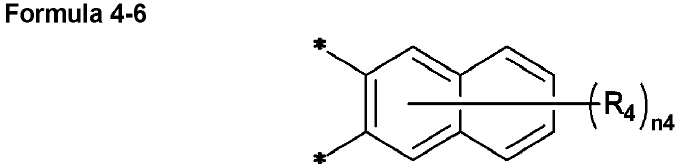

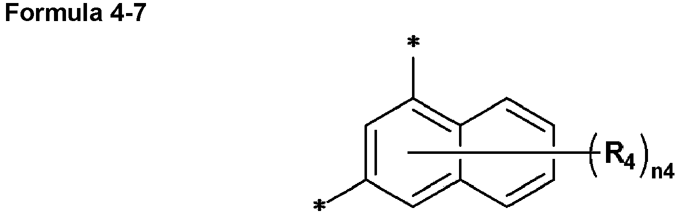

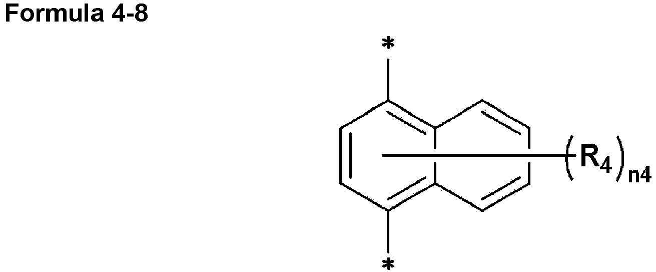

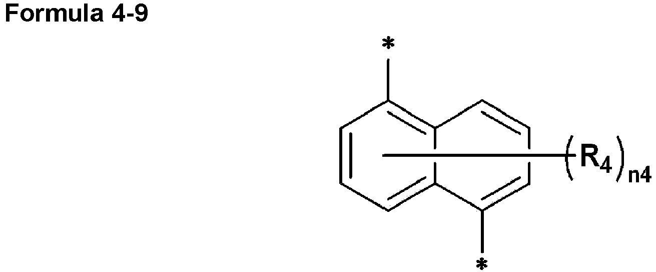

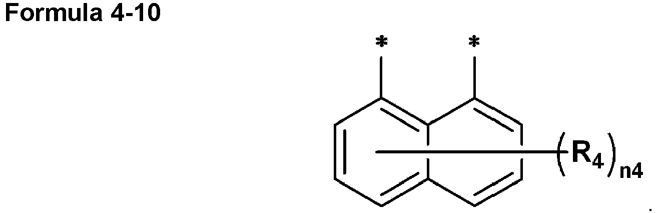

In Formula 4-1 to Formula 4-10, R₃ and R₄ may each independently be a hydrogen atom, a deuterium atom, a halogen atom group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, n3 is an integer of 0 to 4, and n4 is an integer of 0 to 6.

In one or more embodiments, the at least one functional layer may include an emission layer, a first hole transport layer disposed between the first electrode and the emission layer, a second hole transport layer disposed between the first hole transport layer and the emission layer, and an electron transport region disposed between the emission layer and the second electrode, and the first hole transport layer may include the amine compound represented by Formula 1.

In one or more embodiments, the at least one functional layer may further include a third hole transport layer disposed between the second hole transport layer and the emission layer, and the first hole transport layer and the third hole transport layer may each independently include the amine compound represented by Formula 1.

In one or more embodiments, the second hole transport layer may include an amine derivative compound represented by Formula 5:

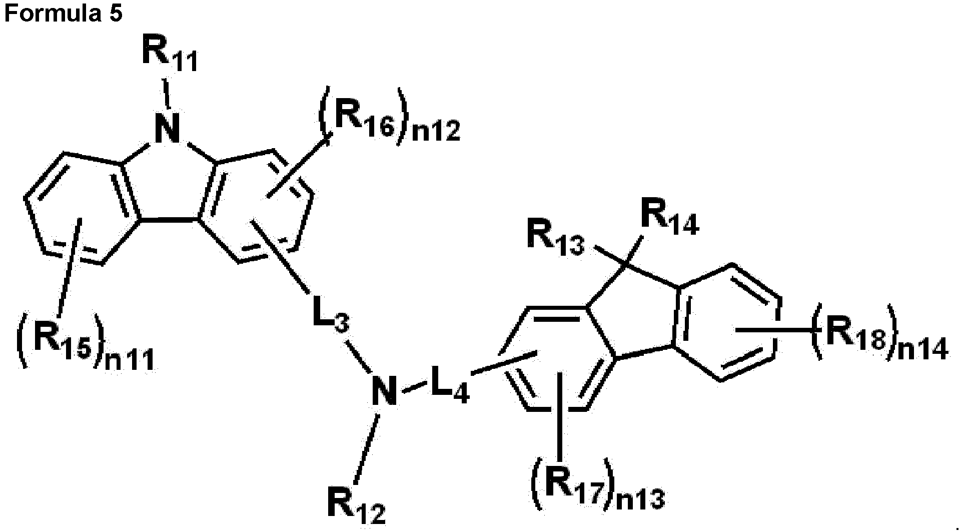

In Formula 5, L₃ and L₄ may each independently be a direct linkage, a substituted or unsubstituted arylene group having 6 to 30 ring-forming carbon atoms, or a substituted or unsubstituted heteroarylene group having 2 to 30 ring-forming carbon atoms; R₁₁ to R₁₄ may each independently be a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, and/or may be bonded to an adjacent group to form a ring; R₁₅ to R₁₈ may each independently be a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or may be bonded to an adjacent group to form a ring; n11 and n14 may each independently be an integer of 0 to 4; and n12 and n13 may each independently be an integer of 0 to 3.

In one or more embodiments, Formula 5 may be represented by Formula 6-1 or Formula 6-2:

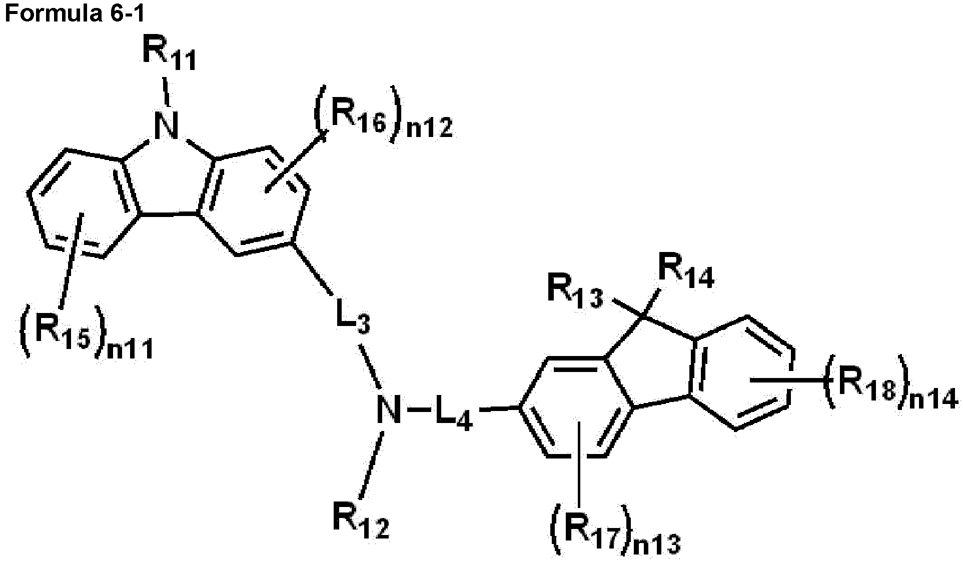

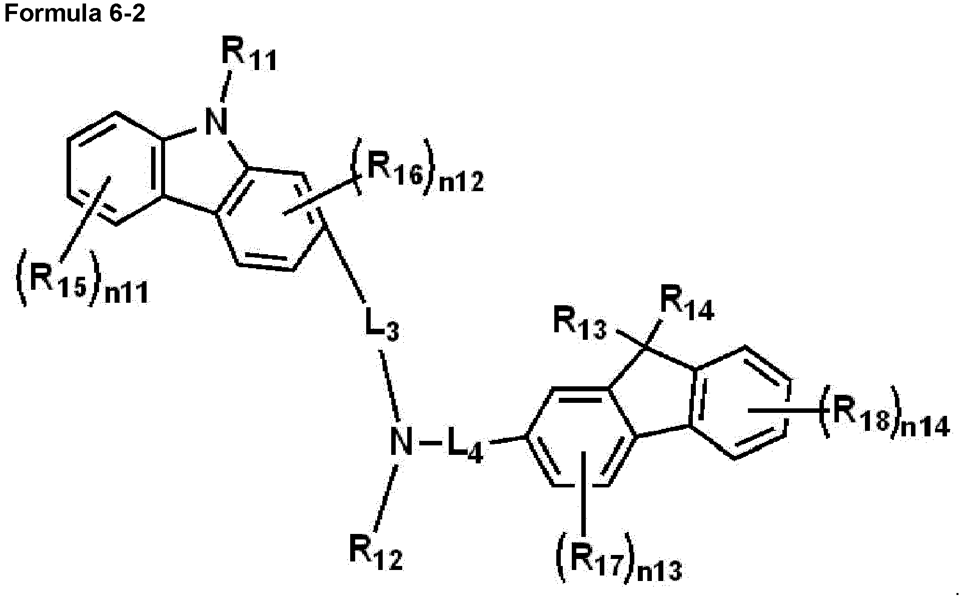

In Formula 6-1 and Formula 6-2, the same definitions as those provided in Formula 5 may be applied to L₃, L₄, R₁₁ to R₁₄, R₁₅ to R₁₈, and n11 to n13.

In one or more embodiments of the present disclosure, an amine compound is represented by Formula 1.

### BRIEF DESCRIPTION OF THE DRAWINGS

The accompanying drawings are included to provide a further understanding of the present disclosure, and are incorporated in and constitute a part of this specification. The drawings illustrate example embodiments of the present disclosure and, together with the description, serve to explain principles of the present disclosure. In the drawings:
FIG. 1 is a plan view of a display apparatus according to one or more embodiments of the present disclosure;
FIG. 2 is a cross-sectional view of a display apparatus according to one or more embodiments of the present disclosure;
FIG. 3 is a cross-sectional view schematically illustrating a light emitting device according to one or more embodiments of the present disclosure;
FIG. 4 is a cross-sectional view schematically illustrating a light emitting device according to one or more embodiments of the present disclosure;
FIG. 5 is a cross-sectional view schematically illustrating a light emitting device according to one or more embodiments of the present disclosure;
FIG. 6 is a cross-sectional view schematically illustrating a light emitting device according to one or more embodiments of the present disclosure;
FIG. 7 is a cross-sectional view schematically illustrating a light emitting device according to one or more embodiments of the present disclosure;
Each of FIGS. 8 and 9 is a cross-sectional view of a display apparatus according to one or more embodiments of the present disclosure;
FIG. 10 is a cross-sectional view illustrating a display apparatus according to one or more embodiments of the present disclosure; and
FIG. 11 is a cross-sectional view illustrating a display apparatus according to one or more embodiments of the present disclosure.

### DETAILED DESCRIPTION

The present disclosure may be modified in many alternate forms, and thus certain embodiments will be exemplified in the drawings and described in more detail herein below. It should be understood, however, that it is not intended to limit the present disclosure to the particular forms disclosed, but rather, is intended to cover all modifications and alternatives falling within the scope of the present claims.

When explaining each of the drawings, like reference numerals are used for referring to like elements. In the accompanying drawings, the dimensions of each structure are exaggeratingly illustrated for clarity of the present disclosure. It will be understood that, although the terms "first", "second", *etc.* may be used herein to describe one or more suitable elements, these elements should not be limited by these terms. These terms are only used to distinguish one element from another. For example, a first element could be termed a second element, and, similarly, a second element could be termed a first element, without departing from the scope of the present disclosure. As used herein, the singular forms, "a," "an," and "the" are intended to include the plural forms as well, unless the context clearly indicates otherwise.

In the present application, it will be understood that the terms "include," "have" *etc.,* specify the presence of a feature, a fixed number, a step, an operation, an element, a component, or a combination thereof disclosed in the specification, but do not exclude the possibility of presence or addition of one or more other features, fixed numbers, steps, operations, elements, components, or combination thereof.

In the present application, when a part such as a layer, a film, a region, or a plate is referred to as being "on" or "above" another part, it can be directly on the other part (e.g., without any intervening parts therebetween), or one or more intervening parts may also be present. Similarly, when a part such as a layer, a film, a region, or a plate is referred to as being "under" or "below" another part, it can be directly under the other part (e.g., without any intervening parts therebetween), or one or more intervening parts may also be present. In some embodiments, it will be understood that when a part is referred to as being "on" another part, it can be disposed on the other part, or disposed under the other part as well.

In the specification, the term "disposed" may refer to being provided and/or positioned.

As used herein, the terms "use," "using," and "used" may be considered synonymous with the terms "utilize," "utilizing," and "utilized," respectively.

As used herein, expressions such as "at least one of", "one of", and "selected from", when preceding a list of elements, modify the entire list of elements and do not modify the individual elements of the list. For example, "at least one selected from a, b and c", "at least one of a, b or c", and "at least one of a, b and/or c" may indicate only a, only b, only c, both (e.g., simultaneously) a and b, both (e.g., simultaneously) a and c, both (e.g., simultaneously) b and c, all of a, b, and c, or variations thereof.

As used herein, the term "and/or" includes any and all combinations of one or more of the associated listed items.

Further, the use of "may" when describing embodiments of the present disclosure refers to "one or more embodiments of the present disclosure".

As used herein, the terms "substantially", "about", and similar terms are used as terms of approximation and not as terms of degree, and are intended to account for the inherent deviations in measured or calculated values that would be recognized by those of ordinary skill in the art. "About" or "approximately," as used herein, is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, 5% of the stated value.

Any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein

In the specification, the term "substituted or unsubstituted" may refer to a group that is unsubstituted or that is substituted with at least one substituent selected from the group consisting of a deuterium atom, a halogen atom, a cyano group, a nitro group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, an alkoxy group, a hydrocarbon ring group, an aryl group, and a heterocyclic group. In some embodiments, each of the substituents exemplified may be substituted or unsubstituted. For example, a biphenyl group may be interpreted as an aryl group and/or a phenyl group substituted with a phenyl group.

In the specification, the phrase "bonded to an adjacent group to form a ring" may indicate that one is bonded to an adjacent group to form a substituted or unsubstituted hydrocarbon ring, or a substituted or unsubstituted heterocycle. The hydrocarbon ring includes an aliphatic hydrocarbon ring and an aromatic hydrocarbon ring. The heterocycle includes an aliphatic heterocycle and an aromatic heterocycle. The hydrocarbon ring and the heterocycle may each independently be monocyclic or polycyclic. In some embodiments, the rings formed by being bonded to each other may be connected to another ring to form a spiro structure.

In the specification, the term "adjacent group" may refer to a pair of substituent groups where the first substituent is connected to an atom which is directly connected to another atom substituted with the second substituent; a pair of substituent groups connected to the same atom; or a pair of substituent groups where the first substituent is sterically positioned at the nearest position to the second substituent. For example, two methyl groups in 1,2-dimethylbenzene may be interpreted as "adjacent groups" to each other and two ethyl groups in 1,1-diethylcyclopentane may be interpreted as "adjacent groups" to each other. In some embodiments, two methyl groups in 4,5-dimethylphenanthrene may be interpreted as "adjacent groups" to each other.

In the specification, examples of the halogen atom may include a fluorine atom, a chlorine atom, a bromine atom, and an iodine atom.

In the specification, the alkyl group may be a linear, branched or cyclic alkyl group. The number of carbons in the alkyl group may be 1 to 50, 1 to 30, 1 to 20, 1 to 10, or 1 to 6. Examples of the alkyl group may include a methyl group, an ethyl group, an n-propyl group, an isopropyl group, an n-butyl group, an s-butyl group, a t-butyl group, an i-butyl group, a 2-ethylbutyl group, a 3,3-dimethylbutyl group, an n-pentyl group, an i-pentyl group, a neopentyl group, a t-pentyl group, a cyclopentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, a cyclohexyl group, a 4-methylcyclohexyl group, a 4-t-butylcyclohexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, a cyclooctyl group, an n-nonyl group, an n-decyl group, an adamantyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldocecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-eicosyl group, a 2-ethyleicosyl group, a 2-butyleicosyl group, a 2-hexyleicosyl group, a 2-octyleicosyl group, an n-henicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, *etc.,* but the embodiment of the present disclosure is not limited thereto.

In the specification, a cycloalkyl group may refer to a cyclic alkyl group. The number of carbons in the cycloalkyl group may be 3 to 50, 3 to 30, 3 to 20, or 3 to 12. Examples of the cycloalkyl group may include a cyclopropyl group, a cyclobutyl group, a cyclopentyl group, a cyclohexyl group, a 4-methylcyclohexyl group, a 4-t-butylcyclohexyl group, a cycloheptyl group, a cyclooctyl group, a cyclononyl group, a cyclodecyl group, a norbornyl group, a 1-adamantyl group, a 2-adamantyl group, an isobornyl group, a bicycloheptanyl group, a bicyclooctanyl group, a bicyclononanyl group, *etc.,* but the embodiment of the present disclosure is not limited thereto.

In the specification, an aryl group refers to any functional group or substituent derived from an aromatic hydrocarbon ring. The aryl group may be a monocyclic aryl group or a polycyclic aryl group. The number of ring-forming carbon atoms in the aryl group may be 6 to 30, 6 to 20, or 6 to 15. Examples of the aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylenyl group, a pyrenyl group, a benzofluoranthenyl group, a chrysenyl group, *etc.,* but the embodiment of the present disclosure is not limited thereto.

In the specification, the fluorenyl group may be substituted, and two substituents may be bonded to each other to form a spiro structure. Examples of cases where the fluorenyl group is substituted are as follows. However, the embodiment of the present disclosure is not limited thereto:

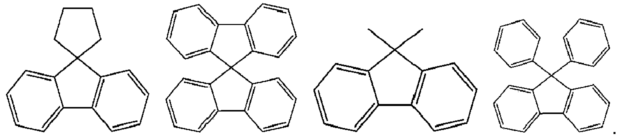

In the specification, the heteroaryl group may include at least one of B, O, N, P, Si, or S as a heteroatom. When the heteroaryl group contains two or more heteroatoms, the two or more heteroatoms may be the same as or different from each other. The heteroaryl group may be a monocyclic heteroaryl group or a polycyclic heteroaryl group. The number of ring-forming carbon atoms in the heteroaryl group may be 2 to 30, 2 to 20, or 2 to 10. Examples of the heteroaryl group may include a thiophene group, a furan group, a pyrrole group, an imidazole group, a pyridine group, a bipyridine group, a pyrimidine group, a triazine group, a triazole group, an acridine group, a pyridazine group, a pyrazine group, a quinoline group, a quinazoline group, a quinoxaline group, a phenoxazine group, a phthalazine group, a pyrido pyrimidine group, a pyrido pyrazine group, a pyrazino pyrazine group, an isoquinoline group, an indole group, a carbazole group, an N-arylcarbazole group, an N-heteroarylcarbazole group, an N-alkylcarbazole group, a benzoxazole group, a benzimidazole group, a benzothiazole group, a benzocarbazole group, a benzothiophene group, a dibenzothiophene group, a thienothiophene group, a benzofuran group, a phenanthroline group, a thiazole group, an isoxazole group, an oxazole group, an oxadiazole group, a thiadiazole group, a phenothiazine group, a dibenzosilole group, a dibenzofuran group, *etc.,* but the embodiment of the present disclosure is not limited thereto.

In the specification, the above description of the aryl group may be applied to an arylene group except that the arylene group is a divalent group. The above description of the heteroaryl group may be applied to a heteroarylene group except that the heteroarylene group is a divalent group.

In the description, the silyl group includes an alkylsilyl group and an arylsilyl group. Examples of the silyl group may include trimethylsilyl, triethylsilyl, t-butyldimethylsilyl, vinyldimethylsilyl, propyldimethylsilyl, triphenylsilyl, diphenylsilyl, phenylsilyl, *etc.* However, one or more embodiments of the present disclosure are not limited thereto.

In the specification, a thio group may include an alkylthio group and an arylthio group. The thio group may mean that a sulfur atom is bonded to the alkyl group or the aryl group as defined above. Examples of the thio group may include a methylthio group, an ethylthio group, a propylthio group, a pentylthio group, a hexylthio group, an octylthio group, a dodecylthio group, a cyclopentylthio group, a cyclohexylthio group, a phenylthio group, a naphthylthio group, but the embodiment of the present disclosure is not limited thereto.

In the specification, an oxy group may mean that an oxygen atom is bonded to the alkyl group or the aryl group as defined above. The oxy group may include an alkoxy group and an aryl oxy group. The alkoxy group may be a linear chain, a branched chain or a ring chain. The number of carbon atoms in the alkoxy group is not specifically limited, but may be, for example, 1 to 20 or 1 to 10. Examples of the oxy group include methoxy, ethoxy, n-propoxy, isopropoxy, butoxy, pentyloxy, hexyloxy, octyloxy, nonyloxy, decyloxy, benzyloxy, *etc.,* but the embodiment of the present disclosure is not limited thereto.

In the specification, the number of carbon atoms in an amine group is not specifically limited, but may be 1 to 30. The amine group may include an alkyl amine group and an aryl amine group. Examples of the amine group may include a methylamine group, a dimethylamine group, a phenylamine group, a diphenylamine group, a naphthylamine group, a 9-methyl-anthracenylamine group, a triphenylamine group, *etc.,* but the embodiment of the present disclosure is not limited thereto.

In the specification, a direct linkage may refer to a single bond.

In some embodiments, in the description, " ^{∗-}" refers to a position to be connected (e.g., a binding site).

Hereinafter, embodiments of the present disclosure will be described with reference to the accompanying drawings.

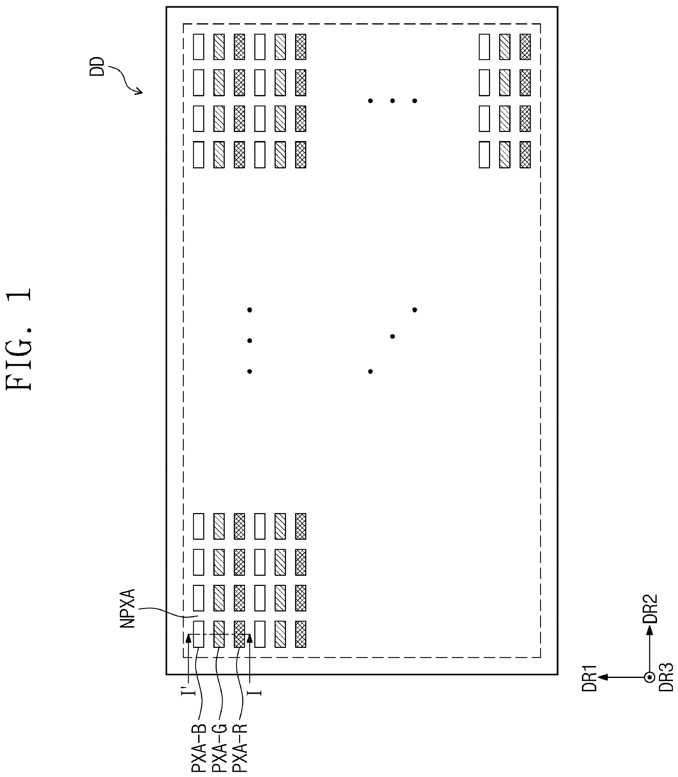

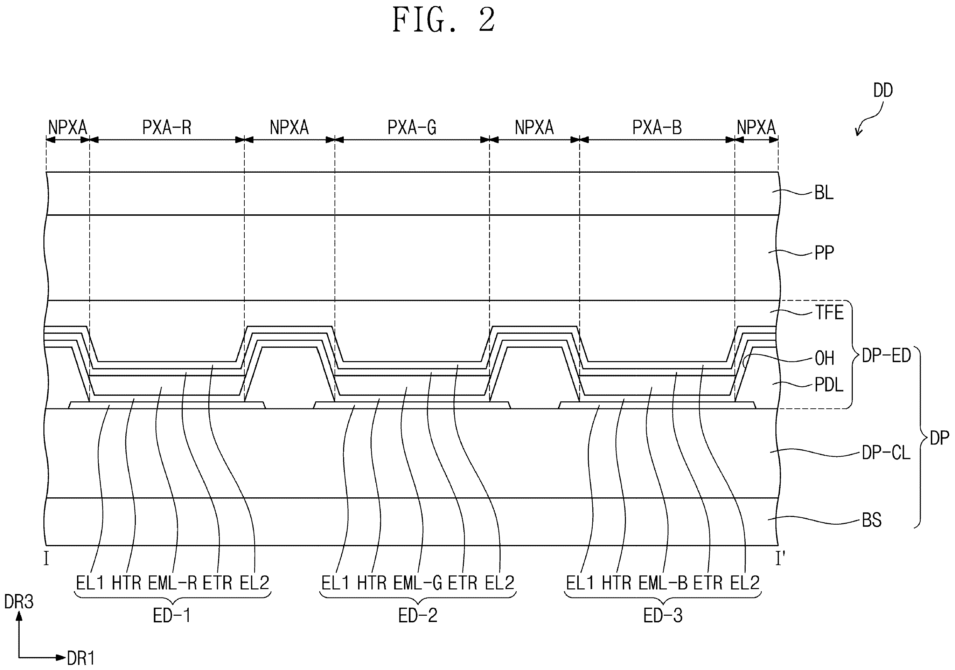

FIG. 1 is a plan view illustrating one or more embodiments of a display apparatus DD. FIG. 2 is a cross-sectional view of the display apparatus DD of one or more embodiments. FIG. 2 is a cross-sectional view illustrating a part taken along line I-I' of FIG. 1.

The display apparatus DD may include a display panel DP and an optical layer PP disposed on the display panel DP. The display panel DP includes light emitting devices ED-1, ED-2, and ED-3. The display apparatus DD may include a plurality of light emitting devices ED-1, ED-2, and ED-3. The optical layer PP may be disposed on the display panel DP and control reflected light in the display panel DP due to external light. The optical layer PP may include, for example, a polarization layer and/or a color filter layer. In some embodiments, the optical layer PP may not be provided in the display apparatus DD.

A base substrate BL may be disposed on the optical layer PP. The base substrate BL may be a member which provides a base surface on which the optical layer PP is disposed. The base substrate BL may be a glass substrate, a metal substrate, a plastic substrate, *etc.* However, the embodiment of the present disclosure is not limited thereto, and the base substrate BL may be an inorganic layer, an organic layer, or a composite material layer (e.g., including an organic material and an inorganic material). In some embodiments, the base substrate BL may not be provided.

The display apparatus DD according to one or more embodiments may further include a filling layer. The filling layer may be disposed between a display device layer DP-ED and the base substrate BL. The filling layer may be an organic material layer. The filling layer may include at least one of an acrylic-based resin, a silicone-based resin, or an epoxy-based resin.

The display panel DP may include a base layer BS, a circuit layer DP-CL provided on the base layer BS, and the display device layer DP-ED. The display device layer DP-ED may include a pixel defining film PDL, the light emitting devices ED-1, ED-2, and ED-3 disposed between portions of the pixel defining film PDL, and an encapsulation layer TFE disposed on the light emitting devices ED-1, ED-2, and ED-3.

The base layer BS may be a member which provides a base surface on which the display device layer DP-ED is disposed. The base layer BS may be a glass substrate, a metal substrate, a plastic substrate, *etc.* However, the embodiment is not limited thereto, and the base layer BS may be an inorganic layer, an organic layer, or a composite material layer.

In one or more embodiments, the circuit layer DP-CL is disposed on the base layer BS, and the circuit layer DP-CL may include a plurality of transistors. Each of the transistors may include a control electrode, an input electrode, and an output electrode. For example, the circuit layer DP-CL may include a switching transistor and a driving transistor for driving the light emitting devices ED-1, ED-2, and ED-3 of the display device layer DP-ED.

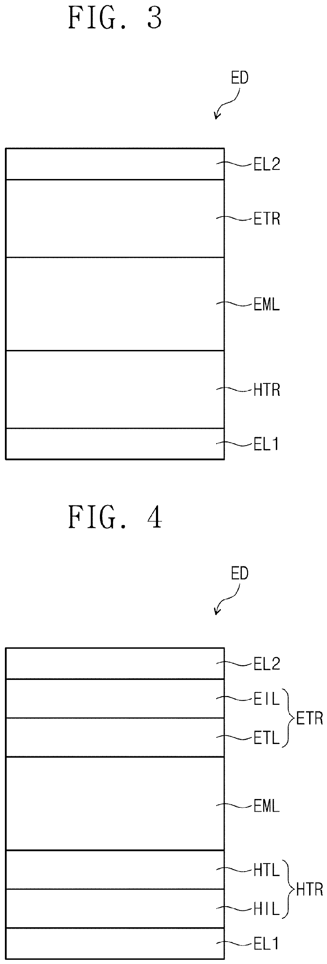

Each of the light emitting devices ED-1, ED-2, and ED-3 may have a structure of a light emitting device ED of one or more embodiments according to FIGS. 3 to 6, which will be described in more detail herein below. Each of the light emitting devices ED-1, ED-2 and ED-3 may include a first electrode EL1, a hole transport region HTR, emission layers EML-R, EML-G and EML-B (respectively), an electron transport region ETR, and a second electrode EL2.

FIG. 2 illustrates one or more embodiments in which the emission layers EML-R, EML-G, and EML-B of the light emitting devices ED-1, ED-2, and ED-3 are disposed in openings OH defined in the pixel defining film PDL, and the hole transport region HTR, the electron transport region ETR, and the second electrode EL2 are provided as a common layer in the entire light emitting devices ED-1, ED-2, and ED-3. However, the embodiment of the present disclosure is not limited thereto, and in some embodiments, the hole transport region HTR and the electron transport region ETR in one or more embodiments may be provided by being patterned inside the openings OH defined in the pixel defining film PDL. For example, the hole transport region HTR, the emission layers EML-R, EML-G, and EML-B, and the electron transport region ETR of the light emitting devices ED-1, ED-2, and ED-3 in one or more embodiments may be provided by being patterned in an inkjet printing method.

The encapsulation layer TFE may cover the light emitting devices ED-1, ED-2 and ED-3. The encapsulation layer TFE may seal the display device layer DP-ED. The encapsulation layer TFE may be a thin film encapsulation layer. The encapsulation layer TFE may be formed by laminating one layer or a plurality of layers. The encapsulation layer TFE includes at least one insulation layer. The encapsulation layer TFE according to one or more embodiments may include at least one inorganic film (hereinafter, an encapsulation-inorganic film). The encapsulation layer TFE according to one or more embodiments may also include at least one organic film (hereinafter, an encapsulation-organic film) and at least one encapsulation-inorganic film.

The encapsulation-inorganic film protects the display device layer DP-ED from moisture/oxygen, and the encapsulation-organic film protects the display device layer DP-ED from foreign substances such as dust particles. The encapsulation-inorganic film may include silicon nitride, silicon oxynitride, silicon oxide, titanium oxide, aluminum oxide, and/or the like, but the embodiment of the present disclosure is not particularly limited thereto. The encapsulation-organic film may include an acrylic-based compound, an epoxy-based compound, and/or the like. The encapsulation-organic film may include a photopolymerizable organic material, but the embodiment of the present disclosure is not particularly limited thereto.

The encapsulation layer TFE may be disposed on the second electrode EL2 and may be disposed filling the opening OH.

Referring to FIGS. 1 and 2, the display apparatus DD may include a non-light emitting region NPXA and light emitting regions PXA-R, PXA-G and PXA-B. The light emitting regions PXA-R, PXA-G and PXA-B may be regions in which light generated by the respective light emitting devices ED-1, ED-2 and ED-3 is emitted. The light emitting regions PXA-R, PXA-G, and PXA-B may be spaced apart from each other on a plane.

Each of the light emitting regions PXA-R, PXA-G, and PXA-B may be a region divided by the pixel defining film PDL. The non-light emitting regions NPXA may be regions between the adjacent light emitting regions PXA-R, PXA-G, and PXA-B, which correspond to portions of the pixel defining film PDL. In some embodiments, in the specification, the light emitting regions PXA-R, PXA-G, and PXA-B may respectively correspond to pixels. The pixel defining film PDL may divide the light emitting devices ED-1, ED-2, and ED-3. The emission layers EML-R, EML-G and EML-B of the light emitting devices ED-1, ED-2 and ED-3 may be disposed in openings OH defined in the pixel defining film PDL and separated from each other.

The light emitting regions PXA-R, PXA-G and PXA-B may be divided into a plurality of groups according to the color of light generated from the light emitting devices ED-1, ED-2 and ED-3. In the display apparatus DD of one or more embodiments shown in FIGS. 1 and 2, three light emitting regions PXA-R, PXA-G, and PXA-B which emit red light, green light, and blue light, respectively, are illustrated. For example, the display apparatus DD of one or more embodiments may include the red light emitting region PXA-R, the green light emitting region PXA-G, and the blue light emitting region PXA-B that are separated from each other.

In the display apparatus DD according to one or more embodiments, the plurality of light emitting devices ED-1, ED-2 and ED-3 may be to emit light beams having wavelengths different from each other. For example, in one or more embodiments, the display apparatus DD may include a first light emitting device ED-1 that emits (e.g., is to emit) red light, a second light emitting device ED-2 that emits (e.g., is to emit) green light, and a third light emitting device ED-3 that emits (e.g., is to emit) blue light. For example, the red light emitting region PXA-R, the green light emitting region PXA-G, and the blue light emitting region PXA-B of the display apparatus DD may correspond to the first light emitting device ED-1, the second light emitting device ED-2, and the third light emitting device ED-3, respectively.

However, the embodiment of the present disclosure is not limited thereto, and the first to third light emitting devices ED-1, ED-2, and ED-3 may be to emit light beams in substantially the same wavelength range or at least one light emitting device may be to emit a light beam in a wavelength range different from the others. For example, the first to third light emitting devices ED-1, ED-2, and ED-3 may all emit blue light.

The light emitting regions PXA-R, PXA-G, and PXA-B in the display apparatus DD according to one or more embodiments may be arranged in a stripe form. Referring to FIG. 1, the plurality of red light emitting regions PXA-R, the plurality of green light emitting regions PXA-G, and the plurality of blue light emitting regions PXA-B each may be arranged along a second directional axis DR2. In some embodiments, the red light emitting region PXA-R, the green light emitting region PXA-G, and the blue light emitting region PXA-B may be alternately arranged in this order along a first directional axis DR1.

FIGS. 1 and 2 illustrate that all the light emitting regions PXA-R, PXA-G, and PXA-B have similar area, but the embodiment of the present disclosure is not limited thereto. Thus, the light emitting regions PXA-R, PXA-G, and PXA-B may have different areas from each other according to the wavelength range of the emitted light. In this case, the areas of the light emitting regions PXA-R, PXA-G, and PXA-B may refer to areas when viewed on a plane defined by the first directional axis DR1 and the second directional axis DR2.

In some embodiments, an arrangement form of the light emitting regions PXA-R, PXA-G, and PXA-B is not limited to the configuration illustrated in FIG. 1, and the order in which the red light emitting region PXA-R, the green light emitting region PXA-G, and the blue light emitting region PXA-B are arranged may be provided in one or more suitable combinations according to the characteristics of display quality required in the display apparatus DD. For example, the arrangement form of the light emitting regions PXA-R, PXA-G, and PXA-B may be a PENTILE^{®} arrangement form (PENTILE^{®} is a registered trademark owned by Samsung Display Co., Ltd.) or a DIAMOND OLED^{™} arrangement form (DIAMOND OLED^{™} is a trademark of Samsung Display Co., Ltd.).

In some embodiments, the areas of the light emitting regions PXA-R, PXA-G, and PXA-B may be different from each other. For example, in one or more embodiments, the area of the green light emitting region PXA-G may be smaller than that of the blue light emitting region PXA-B, but the embodiment of the present disclosure is not limited thereto.

Hereinafter, FIGS. 3 to 7 are cross-sectional views schematically illustrating light emitting devices according to embodiments. The light emitting devices ED according to embodiments each may include a first electrode EL1, a second electrode EL2 facing the first electrode EL1, and at least one functional layer disposed between the first electrode EL1 and the second electrode EL2. The at least one functional layer may include a hole transport region HTR, an emission layer EML, and an electron transport region ETR that are sequentially stacked. For example, each of the light emitting devices ED of embodiments may include the first electrode EL1, the hole transport region HTR, the emission layer EML, the electron transport region ETR, and the second electrode EL2 that are sequentially stacked.

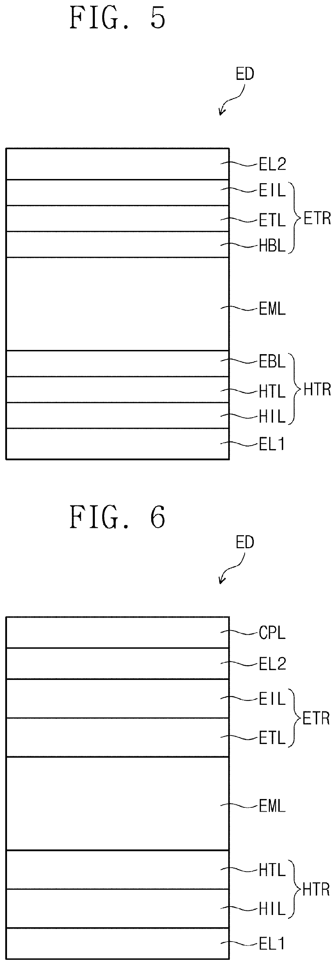

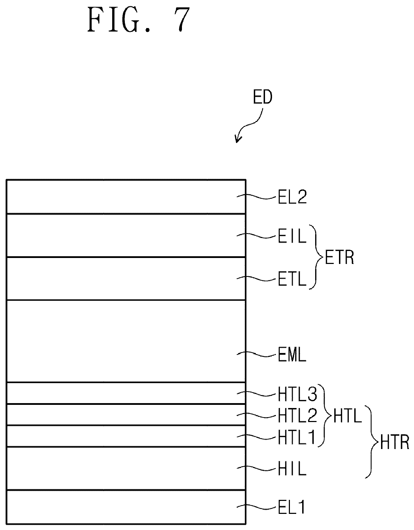

Compared with FIG. 3, FIG. 4 illustrates a cross-sectional view of a light emitting device ED of one or more embodiments, in which a hole transport region HTR includes a hole injection layer HIL and a hole transport layer HTL, and an electron transport region ETR includes an electron injection layer EIL and an electron transport layer ETL. In some embodiments, compared with FIG. 3, FIG. 5 illustrates a cross-sectional view of a light emitting device ED of one or more embodiments, in which a hole transport region HTR includes a hole injection layer HIL, a hole transport layer HTL, and an electron blocking layer EBL, and an electron transport region ETR includes an electron injection layer EIL, an electron transport layer ETL, and a hole blocking layer HBL. Compared with FIG. 4, FIG. 6 illustrates a cross-sectional view of a light emitting device ED of one or more embodiments including a capping layer CPL disposed on a second electrode EL2. Compared with FIG. 4, FIG. 7 illustrates a cross-sectional view of a light emitting device ED of one or more embodiments, in which a hole transport region HTR includes a plurality of hole transport layers HTL1, HTL2, and HTL3.

The light emitting device ED of one or more embodiments may include the amine compound of one or more embodiments, which will be described in more detail herein below, in at least one functional layer of the hole transport region HTR, the emission layer EML, the electron transport region ETR, and/or the like.

The first electrode EL1 has conductivity (e.g., is a conductor). The first electrode EL1 may be formed of a metal material, a metal alloy, or a conductive compound. The first electrode EL1 may be an anode or a cathode. However, the embodiment of the present disclosure is not limited thereto. In some embodiments, the first electrode EL1 may be a pixel electrode. The first electrode EL1 may be a transmissive electrode, a transflective electrode, or a reflective electrode. The first electrode EL1 may include at least one selected from among Ag, Mg, Cu, Al, Pt, Pd, Au, Ni, Nd, Ir, Cr, Li, Ca, LiF, Mo, Ti, W, In, Sn, and Zn, a compound of two or more selected therefrom, a mixture of two or more selected therefrom, and/or an oxide thereof.

When the first electrode EL1 is the transmissive electrode, the first electrode EL1 may include a transparent metal oxide such as indium tin oxide (ITO), indium zinc oxide (IZO), zinc oxide (ZnO), and/or indium tin zinc oxide (ITZO). When the first electrode EL1 is the transflective electrode or the reflective electrode, the first electrode EL1 may include Ag, Mg, Cu, Al, Pt, Pd, Au, Ni, Nd, Ir, Cr, Li, Ca, LiF/Ca (a stacked structure of LiF and Ca), LiF/AI (a stacked structure of LiF and Al), Mo, Ti, W, and/or a compound or mixture thereof (e.g., a mixture of Ag and Mg). In some embodiments, the first electrode EL1 may have a multilayer structure including a reflective film or a transflective film formed of any of the above-described materials, and a transparent conductive film formed of ITO, IZO, ZnO, ITZO, *etc.* For example, the first electrode EL1 may have a three-layer structure of ITO/Ag/ITO, but the embodiment of the present disclosure is not limited thereto. In some embodiments, the embodiment of the present disclosure is not limited thereto, and the first electrode EL1 may include one or more of the above-described metal materials, combinations of at least two metal materials of the above-described metal materials, oxides of the above-described metal materials, and/or the like. The thickness of the first electrode EL1 may be from about 70 nm (700 Å) to about 1,000 nm (10,000 Å). For example, the thickness of the first electrode EL1 may be from about 100 nm (1,000 Å) to about 300 nm (3,000 Å).

The hole transport region HTR is provided on the first electrode EL1. The hole transport region HTR may include at least one of a hole injection layer HIL, a hole transport layer HTL, a buffer layer, an emission-auxiliary layer, or an electron blocking layer EBL. The thickness of the hole transport region HTR may be, for example, from about 5 nm (50 Å) to about 1,500 nm (15,000 Å).

The hole transport region HTR may have a single layer formed of a single material, a single layer formed of a plurality of different materials, or a multilayer structure including a plurality of layers formed of a plurality of different materials.

For example, the hole transport region HTR may have a single layer structure of the hole injection layer HIL or the hole transport layer HTL, or may have a single layer structure formed of a hole injection material and a hole transport material. In some embodiments, the hole transport region HTR may have a single layer structure formed of a plurality of different materials, or a structure in which a hole injection layer HIL/hole transport layer HTL, a hole injection layer HIL/hole transport layer HTL/buffer layer, a hole injection layer HIL/buffer layer, a hole transport layer HTL/buffer layer, or a hole injection layer HIL/hole transport layer HTL/electron blocking layer EBL, are stacked in order from the first electrode EL1, but the embodiment of the present disclosure is not limited thereto.

When the hole transport region HTR includes the hole transport layer HTL, the hole transport layer HTL may have a single layer formed of a single material, a single layer formed of a plurality of different materials, or a multilayer structure including a plurality of layers formed of a plurality of different materials. When the hole transport layer HTL has the multilayer structure, as in FIG. 7, the hole transport layer HTL may include first to third hole transport layers HTL1, HTL2, and HTL3. The hole transport layer HTL may include a first hole transport layer HTL1 disposed on the first electrode EL1, a second hole transport layer HTL2 disposed on the first hole transport layer HTL1, and a third hole transport layer HTL3 disposed on the second hole transport layer HTL2. In the light emitting device ED of one or more embodiments, the hole transport layer HTL may include the plurality of hole transport layers HTL1, HTL2, and HTL3 disposed in order of the first hole transport layer HTL1/second hole transport layer HTL2/third hole transport layer HTL3 in the thickness direction.

In the light emitting device ED of one or more embodiments, the hole transport region HTR may include the first hole transport layer HTL1 disposed to be adjacent to the hole injection layer HIL, the third hole transport layer HTL3 disposed to be adjacent to the emission layer EML, and the second hole transport layer HTL2 disposed between the first hole transport layer HTL1 and the third hole transport layer HTL3. Each of the first hole transport layer HTL1 and the third hole transport layer HTL3 may include the amine compound represented by Formula 1 of one or more embodiments. The second hole transport layer HTL2 may include an amine derivative compound represented by Formula 5 of one or more embodiments.

The first hole transport layer HTL1 may include a first amine compound having a first refractive index. The second hole transport layer HTL2 may include a second amine compound having a second refractive index greater than the first refractive index. The third hole transport layer HTL3 may include a third amine compound having a third refractive index less than the second refractive index. For example, the second refractive index may be larger than each of the first refractive index and the third refractive index. The first refractive index may be the same as or different from the third refractive index.

The first to third hole transport layers HTL1, HTL2, and HTL3 may include the first to third amine compound to have the first to third refractive indexes, respectively.

In the light emitting device ED of one or more embodiments, the first hole transport layer HTL1 and the third hole transport layer HTL3 having relatively lower refractive indexes may be respectively disposed on the upper side and lower side with respect to the second hole transport layer HTL2 having a relatively higher refractive index than other hole transport layers HTL1 and HTL3. In the light emitting device ED of one or more embodiments, the hole transport region HTR may include three hole transport layers HTL1, HTL2, and HTL3 disposed in order of the low refractive hole transport layer/high refractive hole transport layer/low refractive hole transport layer in the thickness direction.

The difference between the first refractive index of the first hole transport layer HTL1 and the second refractive index of the second hole transport layer HTL2 may be about 0.1 to about 1.0. The difference between the third refractive index of the third hole transport layer HTL3 and the second refractive index of the second hole transport layer HTL2 may be about 0.1 to about 1.0. The difference between the first refractive index and the second refractive index may be the same as or different from the difference between the third refractive index and the second refractive index. In one or more embodiments, the difference between the first refractive index of the first hole transport layer HTL1 and the second refractive index of the second hole transport layer HTL2 may be about 0.1 to about 0.5.

The first refractive index and the third refractive index may each independently be about 1.4 to about 1.8. The first refractive index and the third refractive index may be the same as or different from each other. The second refractive index may be about 1.8 to about 2.4. For example, the first refractive index and the third refractive index may each independently be about 1.4 to about 1.8, and the second refractive index may be about 1.8 to about 2.4. The light emitting device ED of one or more embodiments may include the three hole transport layers HTL1, HTL2, and HTL3 disposed in order of the low refractive hole transport layer/high refractive hole transport layer/low refractive hole transport layer, thereby having an increase in light extraction efficiency due to the occurrence of the constructive interference of light by the hole transport layers.

The hole transport region HTR may be formed utilizing one or more suitable methods such as a vacuum deposition method, a spin coating method, a cast method, a Langmuir-Blodgett (LB) method, an inkjet printing method, a laser printing method, and/or a laser induced thermal imaging (LITI) method.

The hole transport region HTR in the light emitting device ED of one or more embodiments may include an amine compound of one or more embodiments. The hole transport layer HTL in the light emitting device ED of one or more embodiments may include the amine compound represented by Formula 1. In some embodiments, as in FIG. 7, when the light emitting device ED includes the plurality of hole transport layers HTL1, HTL2, and HTL3, the first hole transport layer HTL1 and/or the third hole transport layer HTL3 may include the amine compound represented by Formula 1.

The amine compound of one or more embodiments includes a structure in which a first substituent, a second substituent, and a third substituent are linked to a core (e.g., central) nitrogen atom. In one or more embodiments, the first substituent has a fluorenyl moiety, a dibenzofuranyl moiety, or a dibenzothiophenyl moiety. The second substituent has a structure in which an aliphatic cycloalkyl group, such as a cyclohexyl group, a bicycloheptanyl group, a bicyclooctanyl group, a bicyclononanyl group, and/or an adamantyl group, is linked via an arylene or heteroarylene linker. The third substituent has a structure in which at least two additional substituents are bonded to a phenyl group, and the additional substituent bonded to the phenyl group is a silyl group, an alkyl group, an aryl group, and/or a heteroaryl group. The amine compound of one or more embodiments may include an amine group, and the first to third substituents may be bonded to the amine group (e.g., to the central N atom) of the amine compound of one or more embodiments. The amine compound of one or more embodiments having such a structure exhibits high thermal characteristics, and thus the application of the amine compound to the light emitting device ED of one or more embodiments contributes to an improvement in device service life and efficiency, and the excellent or improved charge transport capability may allow to balance holes and electrons in the emission layer EML. In some embodiments, the amine compound of one or more embodiments has low refractive characteristics (e.g., refraction of light may be reduced), and thus the application of the amine compound to the light emitting device ED may reduce a proportion of light progressing downwards, and the amount of light loss inside the light emitting device may be reduced and the amount of light emitted to the outside may be increased, thereby achieving an effect of improving the luminous efficiency of the light emitting device ED.

The amine compound of one or more embodiments is represented by Formula 1:

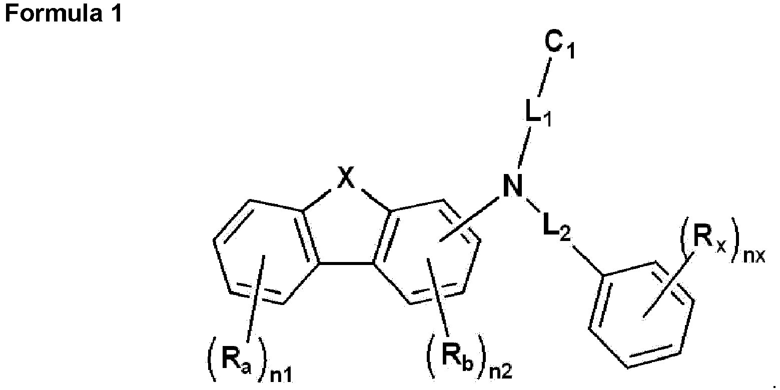

In Formula 1, X may be CR₁R₂, O, or S. For example, the amine compound represented by Formula 1 may have a fluorenyl moiety, a dibenzofuranyl moiety, or a dibenzothiophenyl moiety in the molecular structure.

In Formula 1, C₁ is a substituted or unsubstituted cycloalkyl group having 6 to 12 ring-forming carbon atoms. For example, C₁ may be a substituted or unsubstituted cyclohexyl group, a substituted or unsubstituted bicycloheptanyl group, a substituted or unsubstituted bicyclooctanyl group, a substituted or unsubstituted bicyclononanyl group, or a substituted or unsubstituted adamantyl group. For example, C₁ may be a substituted or unsubstituted cyclohexyl group, a substituted or unsubstituted bicyclo[2,2,1]heptanyl group, a substituted or unsubstituted bicyclo[2,2,2]octanyl group, a substituted or unsubstituted bicyclo[3,2,2]nonanyl group, or a substituted or unsubstituted adamantyl group.

In Formula 1, L₁ and L₂ may each independently be a substituted or unsubstituted arylene group having 6 to 30 ring-forming carbon atoms, or a substituted or unsubstituted heteroarylene group having 2 to 30 ring-forming carbon hetero. For example, L₁ and L₂ may each independently be a substituted or unsubstituted phenylene group, a substituted or unsubstituted bivalent biphenylene group, or a substituted or unsubstituted bivalent naphthylene group.

In Formula 1, R₁, R₂, Rₐ and R_{b} may each independently be a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. In some embodiments, R₁, R₂, Rₐ and R_{b} may each independently be bonded to an adjacent group to form a ring. For example, Rₐ and R_{b} may each independently be a hydrogen atom, a deuterium atom, or a halogen atom. For example, R₁ and R₂ may each independently be a substituted or unsubstituted methyl group, a substituted or unsubstituted ethyl group, a substituted or unsubstituted phenyl group, and/or may be bonded to an adjacent group to form a ring.

In Formula 1, Rₓ is a substituted or unsubstituted silyl group, a substituted or unsubstituted alkyl group having 1 to 20 carbon atoms, a substituted or unsubstituted aryl group having 6 to 30 ring-forming carbon atoms, or a substituted or unsubstituted heteroaryl group having 6 to 30 ring-forming carbon atoms. For example, Rₓ may be a substituted or unsubstituted trimethylsilyl group, a substituted or unsubstituted isopropyl group, a substituted or unsubstituted t-butyl group, a substituted or unsubstituted cyclohexyl group, a substituted or unsubstituted bicycloheptanyl group, a substituted or unsubstituted adamantyl group, a substituted or unsubstituted phenyl group, a substituted or unsubstituted biphenyl group, or a substituted or unsubstituted naphthyl group.

In Formula 1, nx is an integer of 2 to 5. For example, the amine compound represented by Formula 1 includes at least two Rₓ substituents in the molecular structure. nx is an integer of 2 or more, and thus a plurality of Rₓ's may all be the same, or at least one of the plurality of Rₓ's may be different from the others.

In Formula 1, n1 refers to the number of R₁'s, and is an integer of 0 to 4. n2 refers to the number of R₂'s, and is an integer of 0 to 3.

When n1 is 0, the amine compound of one or more embodiments may not be substituted with Rₐ. In Formula 1, the case where n1 is 4 and Rₐ's are all hydrogen atoms may be the same as the case where n1 is 0 in Formula 1. When n1 is an integer of 2 or more, a plurality of Rₐ's may all be the same, or at least one of the plurality of Rₐ's may be different from the others.

When n2 is 0, the amine compound of one or more embodiments may not be substituted with R_{b}. In Formula 1, the case where n2 is 3 and R_{b}'s are all hydrogen atoms may be the same as the case where n2 is 0 in Formula 1. When n2 is an integer of 2 or more, a plurality of R_{b}'s may all be the same, or at least one of the plurality of R_{b}'s may be different from the others.

In one or more embodiments, the amine compound represented by Formula 1 may be represented by Formula 1-1:

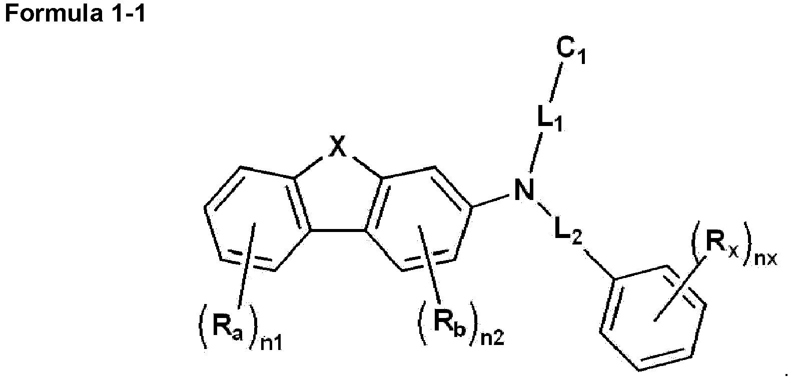

Formula 1-1 represents the case where the carbon position, in which the first substituent corresponding to the fluorenyl moiety, dibenzofuranyl moiety, or dibenzothiophenyl moiety is linked to the amine core, is specified in Formula 1 structure. For example, Formula 1-1 represents the case where the first substituent is bonded to the amine core at the meta-position with respect to X position in Formula 1 structure.

In some embodiments, in Formula 1-1, the same descriptions as those provided in Formula 1 may be applied to X, C₁, L₁, L₂, R₁, R₂, Rₐ, R_{b}, Rₓ, nx, n1, and n2.

In one or more embodiments, the amine compound represented by Formula 1 may be represented by any one selected from among Formula 2-1 to Formula 2-3:

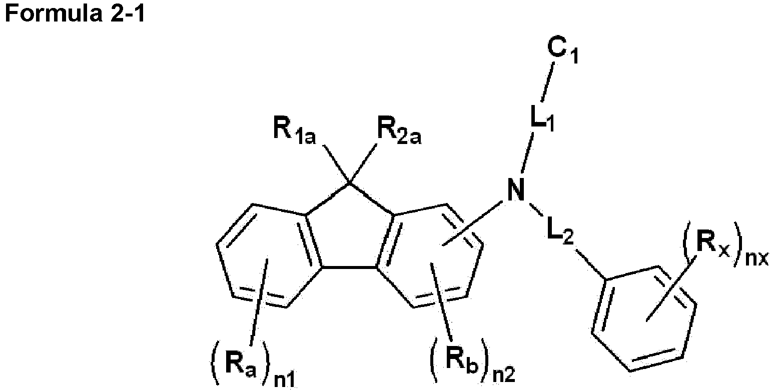

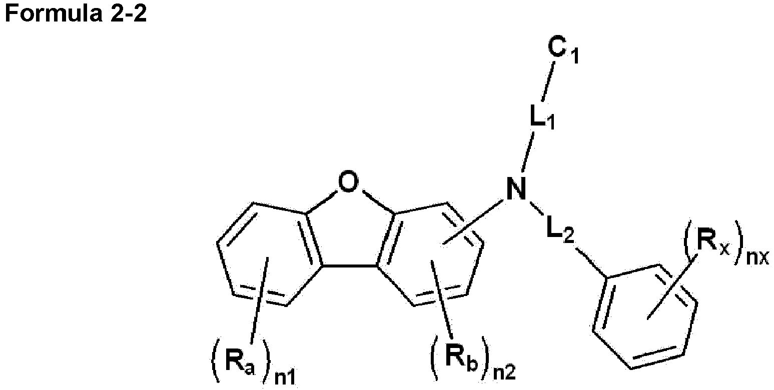

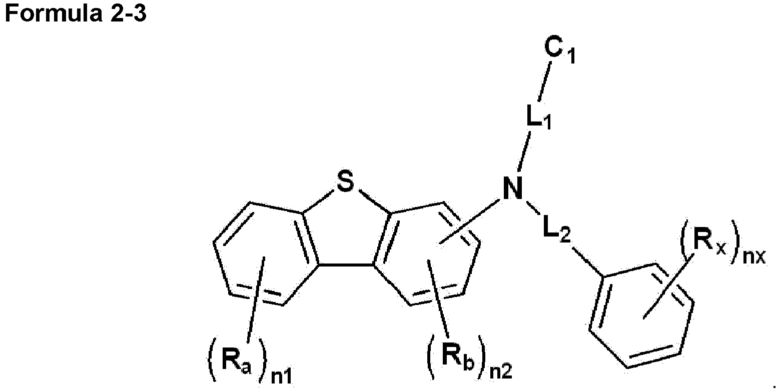

Formula 2-1 to Formula 2-3 represent the cases where in Formula 1 structure, X is specified so that the amine compounds of embodiments are specified to include the fluorenyl moiety, dibenzofuranyl moiety, and dibenzothiophenyl moiety, respectively. Formula 2-1 represents the case where the amine compound represented by Formula 1 is specified to include the fluorenyl moiety, Formula 2-2 represents the case where the amine compound represented by Formula 1 is specified to include the dibenzofuranyl moiety, and Formula 2-3 represents the case where the amine compound represented by Formula 1 is specified to include the dibenzothiophenyl moiety.

In Formula 2-1, R₁ₐ and R₂ₐ may each independently be a substituted or unsubstituted alkyl group having 1 to 20 carbon atoms, or a substituted or unsubstituted aryl group having 6 to 30 ring-forming carbon atoms. In some embodiments, R₁ₐ and R₂ₐ may each independently be bonded to an adjacent group to form a ring. For example, R₁ₐ and R₂ₐ may each independently be a substituted or unsubstituted methyl group, a substituted or unsubstituted ethyl group, or a substituted or unsubstituted phenyl group. In some embodiments, R₁ₐ and R₂ₐ may each independently be a substituted or unsubstituted phenyl group, and R₁ₐ and R₂ₐ may be bonded to each other to form a ring.

In some embodiments, in Formula 2-1 to Formula 2-3, the same descriptions as those provided in Formula 1 may be applied to C₁, L₁, L₂, R₁, R₂, Rₐ, R_{b}, Rₓ, nx, n1, and n2.

In one or more embodiments, the amine compound represented by Formula 1 may be represented by Formula 3:

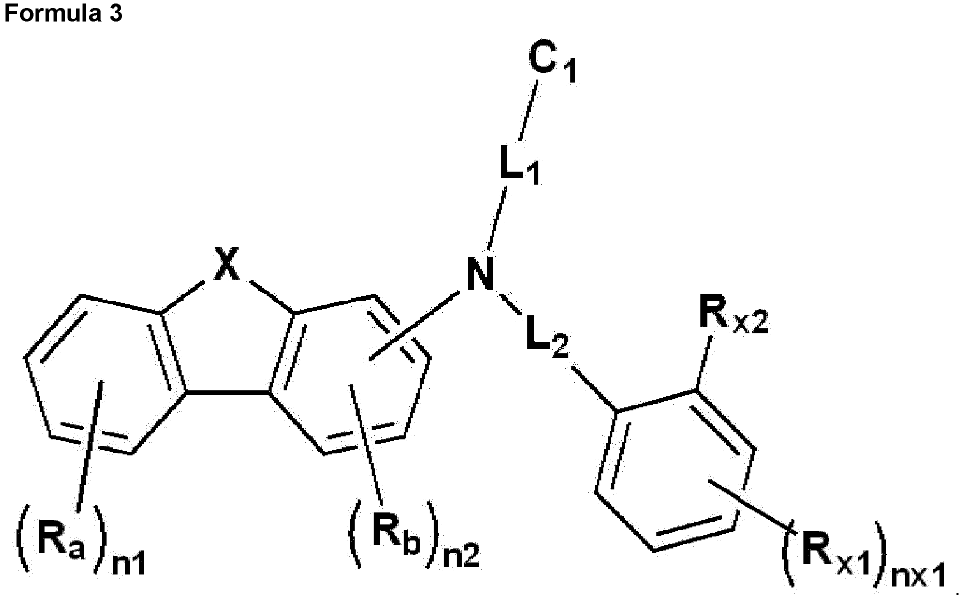

Formula 3 represents the case where in Formula 1 structure, the position, at which one among Rₓ's (i.e., Rₓ(s)) is substituted, is specified. For example, Formula 3 represents the case where at least one among Rₓ's is specified in the phenyl group moiety at which Rₓ is substituted so that at least one among Rₓ's is substituted at the ortho position with respect to the position of L₂, a linker.

In Formula 3, Rₓ₁ and Rₓ₂ may each independently be a substituted or unsubstituted silyl group, a substituted or unsubstituted alkyl group of 1 to 20 carbon atoms, a substituted or unsubstituted aryl group of 6 to 30 ring-forming carbon atoms, or a substituted or unsubstituted heteroaryl group of 6 to 30 ring-forming carbon atoms. For example, Rₓ₁ and Rₓ₂ may each independently be a substituted or unsubstituted trimethylsilyl group, a substituted or unsubstituted isopropyl group, a substituted or unsubstituted t-butyl group, a substituted or unsubstituted cyclohexyl group, a substituted or unsubstituted bicycloheptanyl group, a substituted or unsubstituted adamantyl group, a substituted or unsubstituted phenyl group, a substituted or unsubstituted biphenyl group, or a substituted or unsubstituted naphthyl group.

In Formula 3, nx1 is an integer of 1 to 4. For example, the amine compound represented by Formula 3 further includes at least one Rₓ₁ substituent in the molecular structure in addition to including Rₓ₂. nx1 is an integer of 1 or more, and thus a plurality of Rₓ₁'s may all be the same, or at least one of the plurality of Rₓ₁'s may be different from the others.

In some embodiments, in Formula 3, the same descriptions as those provided in Formula 1 may be applied to X, C₁, L₁, L₂, R₁, R₂, Rₐ, R_{b}, n1, and n2.

In one or more embodiments, the amine compound represented by Formula 3 may be represented by any of Formula 3-1 to Formula 3-6:

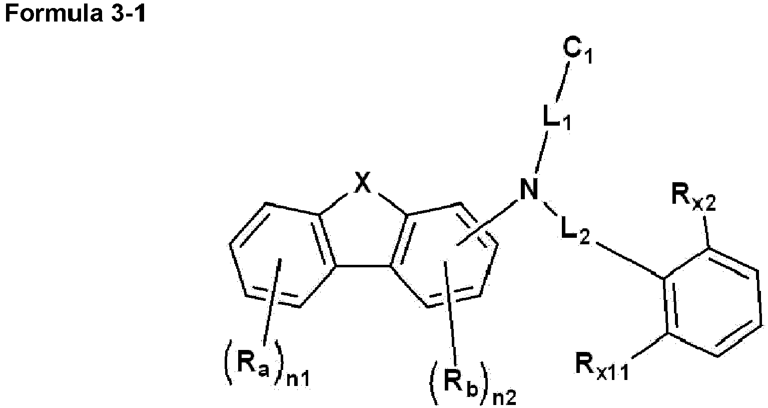

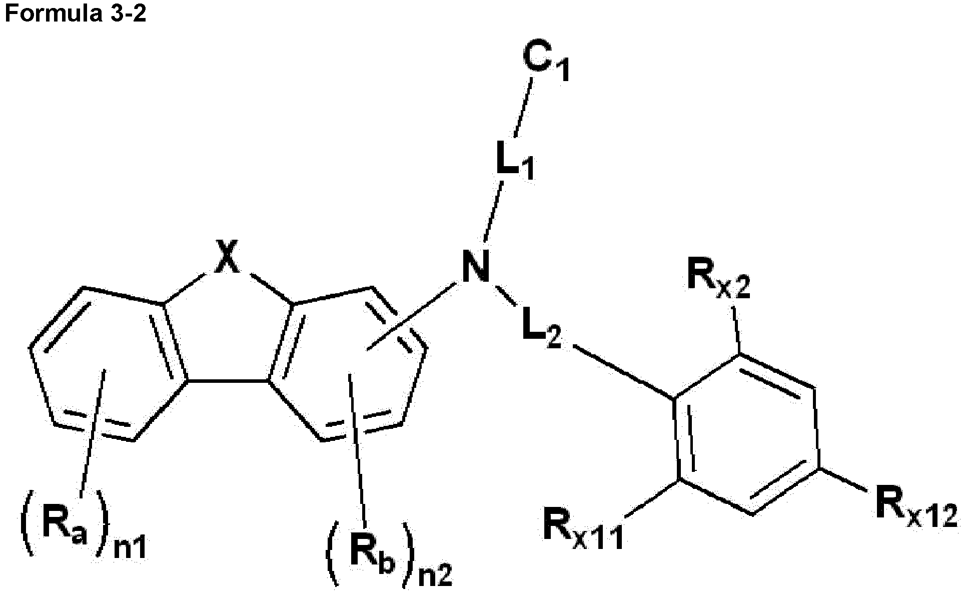

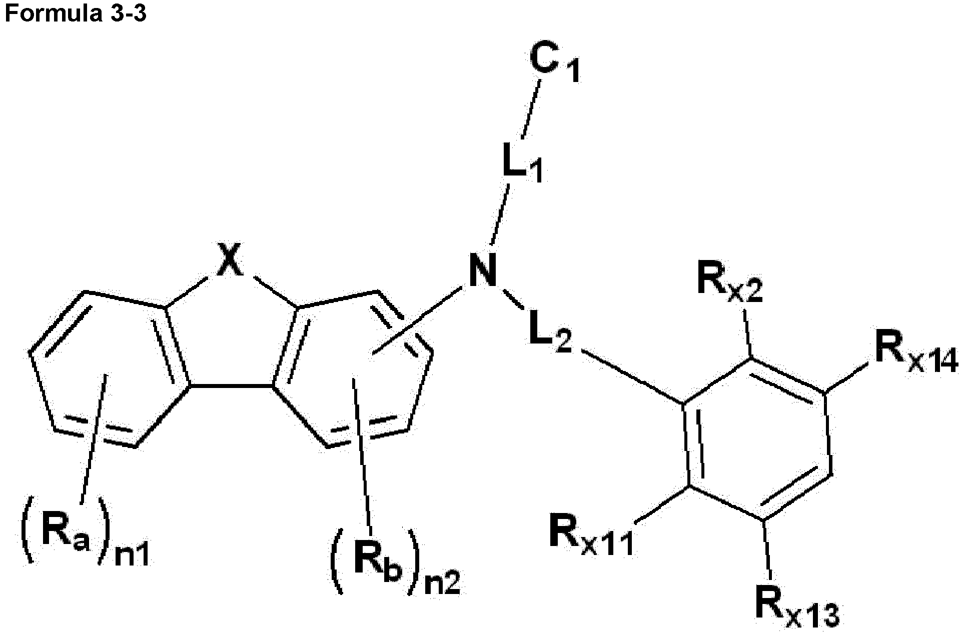

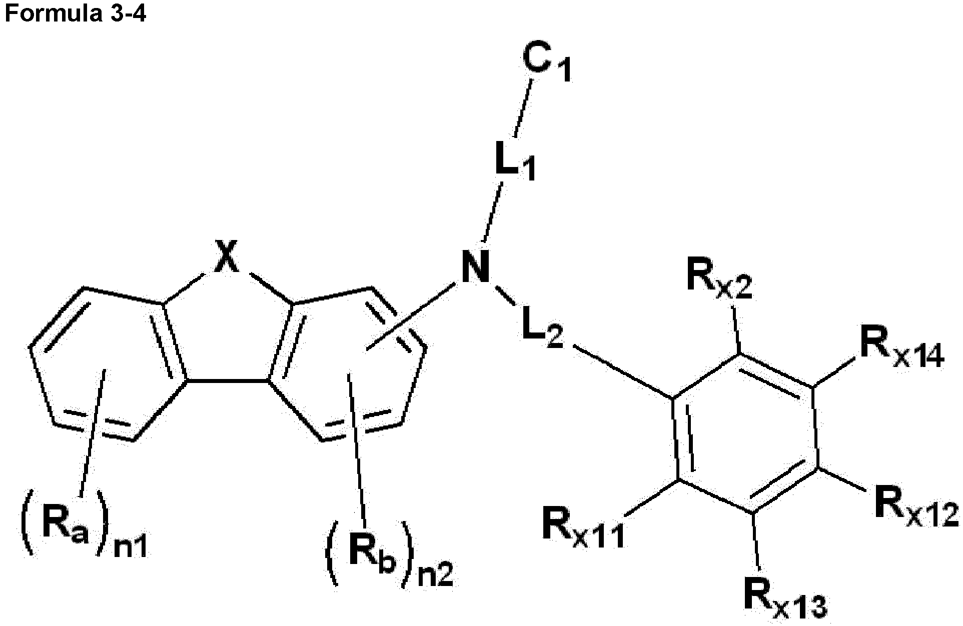

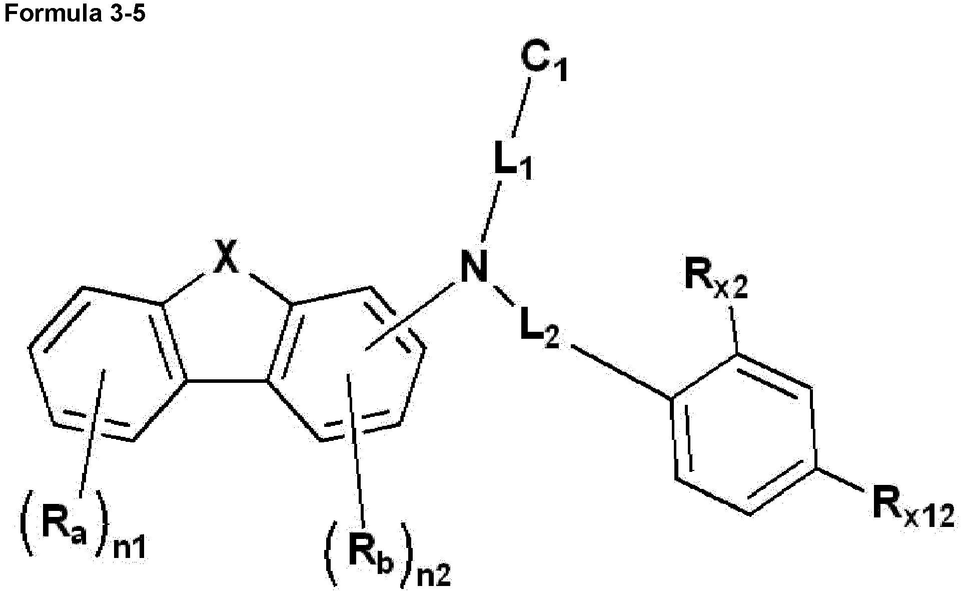

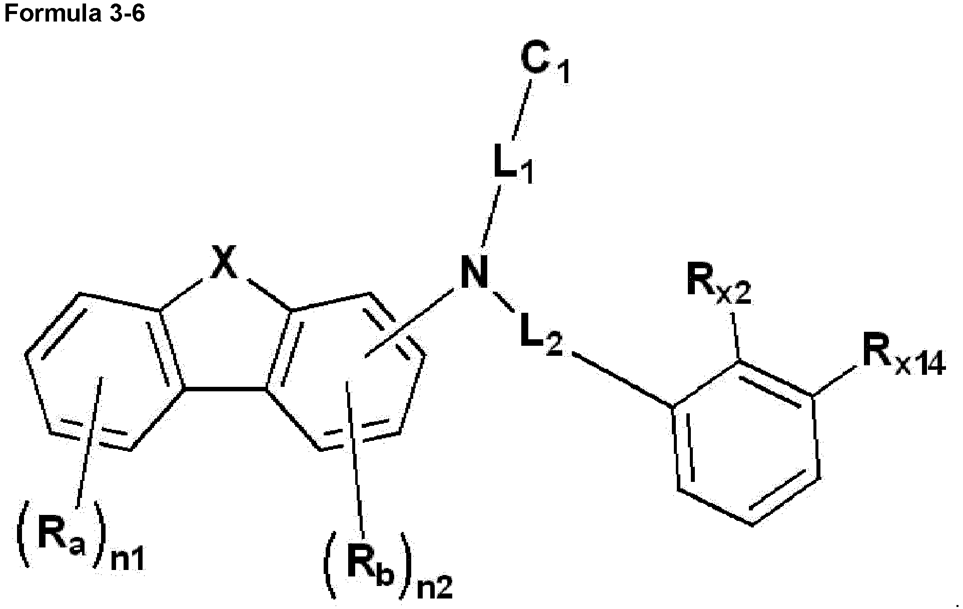

Formula 3-1 to Formula 3-6 represent the case where the number of substituents represented by Rₓ₁ and substituted position thereof are specified in Formula 3 structure.

In Formula 3-1 to Formula 3-6, Rₓ₁₁ to Rₓ₁₄ may each independently be a substituted or unsubstituted silyl group, a substituted or unsubstituted alkyl group of 1 to 20 carbon atoms, a substituted or unsubstituted aryl group of 6 to 30 ring-forming carbon atoms, or a substituted or unsubstituted heteroaryl group of 6 to 30 ring-forming carbon atoms. For example, Rₓ₁₁ and Rₓ₁₄ may each independently be a substituted or unsubstituted trimethylsilyl group, a substituted or unsubstituted isopropyl group, a substituted or unsubstituted t-butyl group, a substituted or unsubstituted cyclohexyl group, a substituted or unsubstituted bicycloheptanyl group, a substituted or unsubstituted adamantyl group, a substituted or unsubstituted phenyl group, a substituted or unsubstituted biphenyl group, or a substituted or unsubstituted naphthyl group.

In some embodiments, in Formula 3-1 to Formula 3-6, the same descriptions as those provided in Formula 3 may be applied to X, C₁, L₁, L₂, R₁, R₂, Rₐ, R_{b}, n1, n2, and Rₓ₂.

Referring to Formula 1 again, each of L₁ and L₂ in Formula 1 may be represented by any one selected from among Formula 4-1 to Formula 4-10:

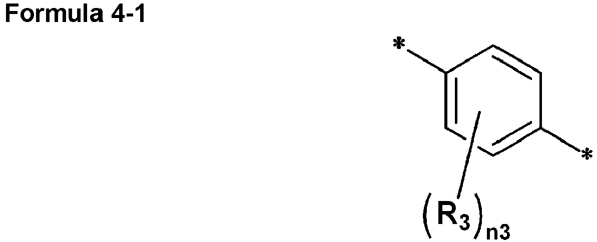

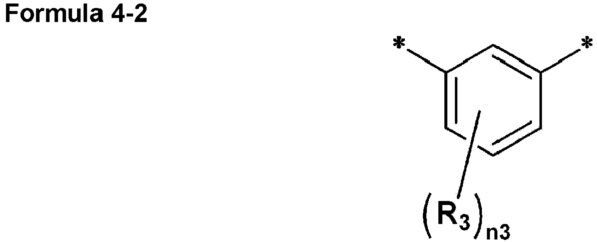

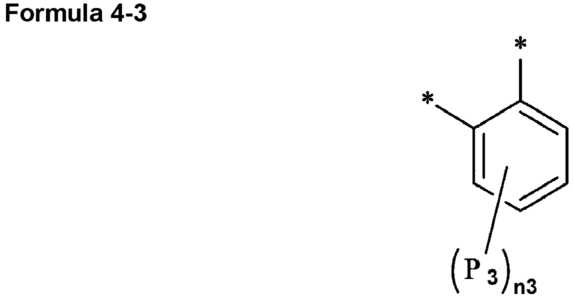

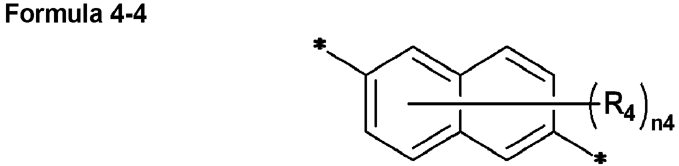

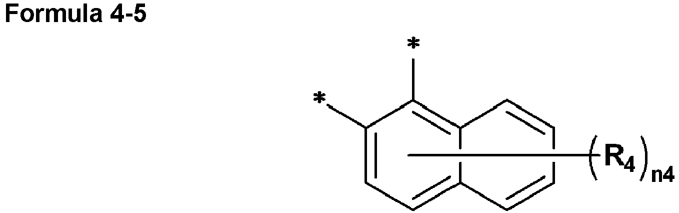

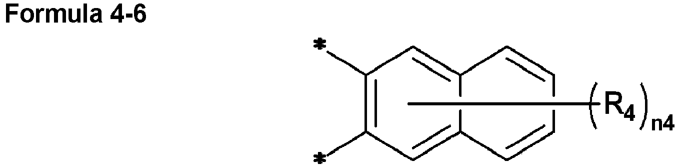

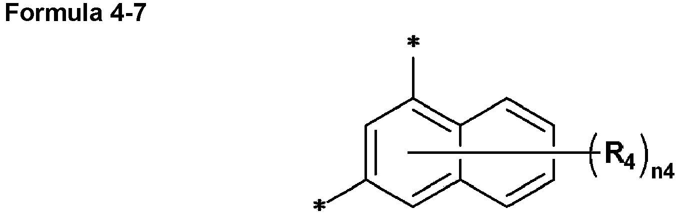

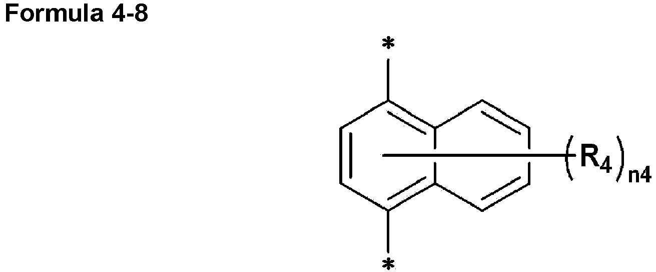

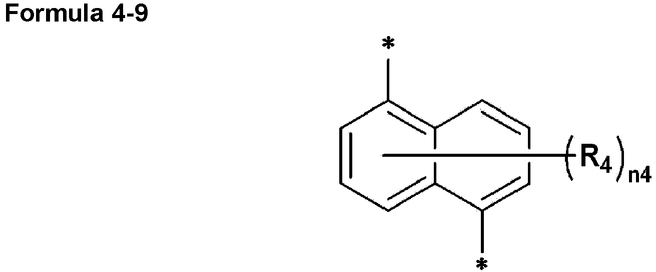

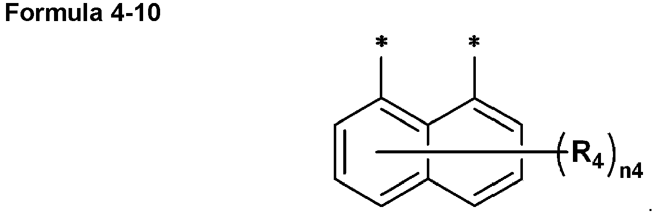

In Formula 4-1 to Formula 4-10, R₃ and R₄ may each independently be a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. For example, R₃ and R₄ may each independently be a hydrogen atom, a deuterium atom, a halogen atom, a substituted or unsubstituted methyl group, or a substituted or unsubstituted phenyl group.

In Formula 4-1 to Formula 4-3, n3 refers to the number of R₃'s, and is an integer of 0 to 4. In Formula 4-4 to Formula 4-10, n4 refers to the number of R₄'s, and is an integer of 0 to 6.

When n3 is 0, the amine compound of one or more embodiments may not be substituted with R₃. In Formula 4-1 to Formula 4-3, the case where n3 is 4 and R₃'s are all hydrogen atoms may be the same as the case where n3 is 0 in Formula 4-1 to Formula 4-3. When n3 is an integer of 2 or more, a plurality of R₃'s may all be the same, or at least one of the plurality of R₃'s may be different from the others.

When n4 is 0, the amine compound of one or more embodiments may not be substituted with R₄. In Formula 4-1 to Formula 4-10, the case where n4 is 6 and R₄'s are all hydrogen atoms may be the same as the case where n4 is 0 in Formula 4-4 to Formula 4-10. When n4 is an integer of 2 or more, a plurality of R₄'s may all be the same, or at least one of the plurality of R₄'s may be different from the others.

In one or more embodiments, the amine compound represented by Formula 1 may be represented by any one selected from among the compounds in Compound Group 1. The hole transport region HTR may include at least one selected from among the compounds represented by Compound Group 1:

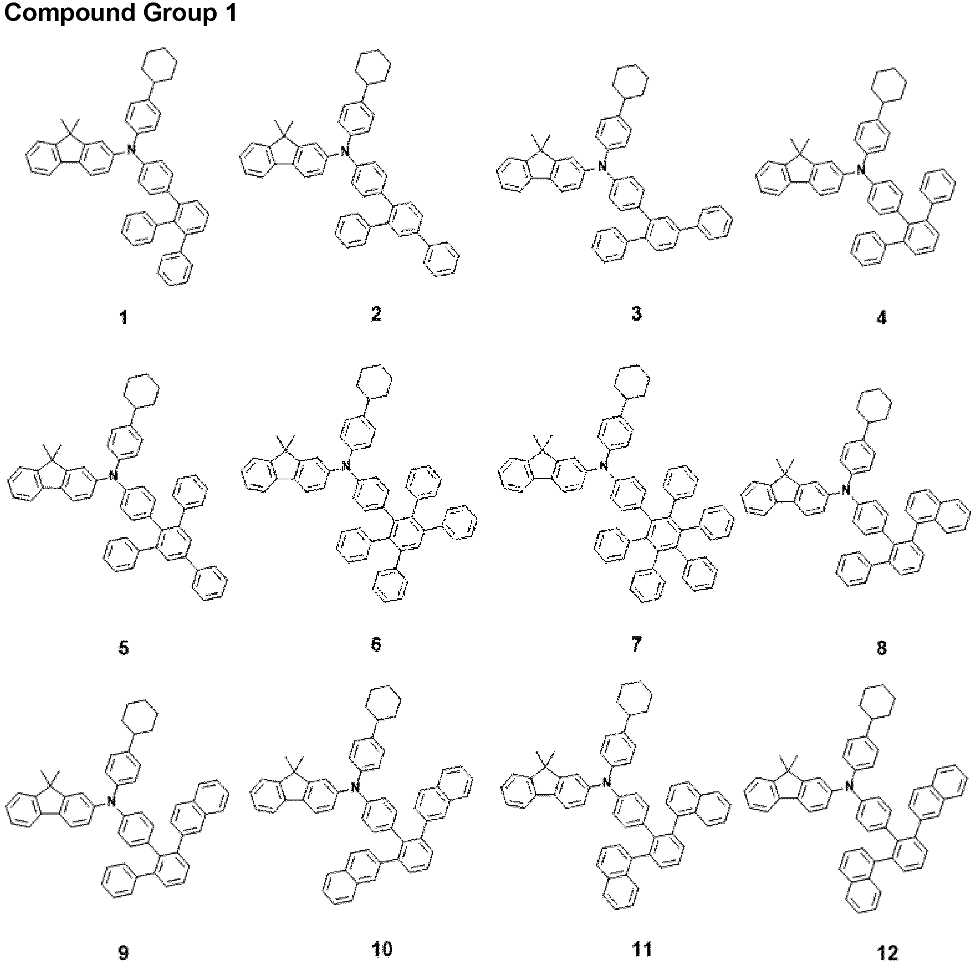

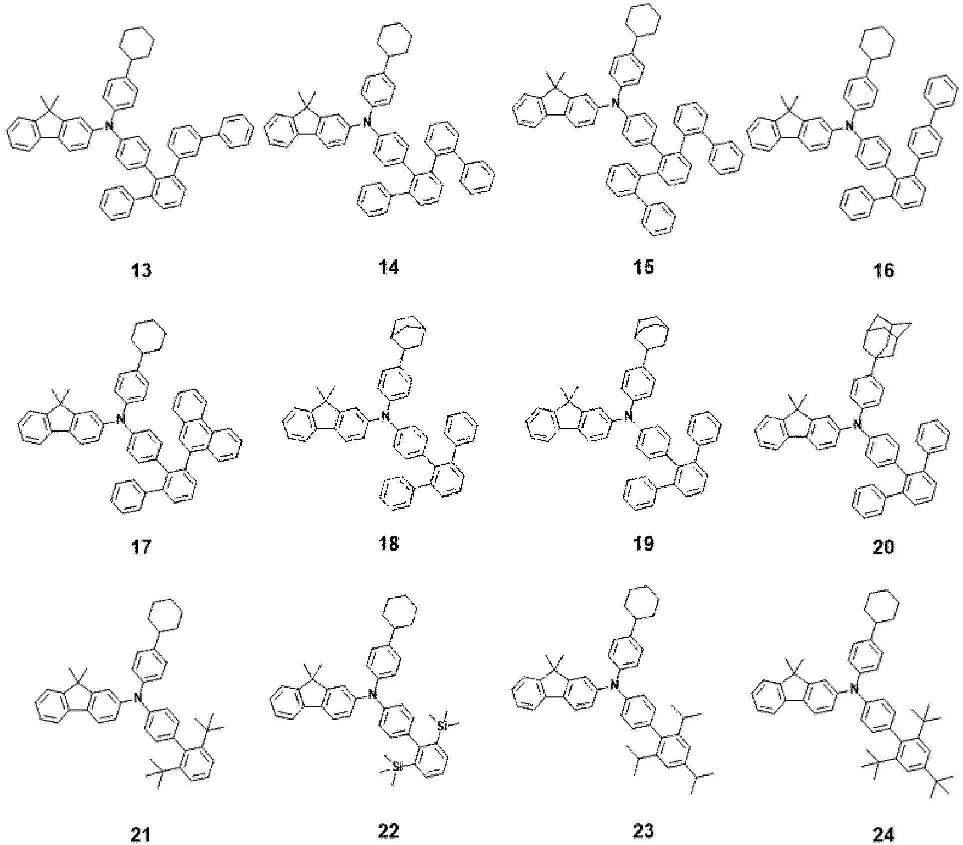

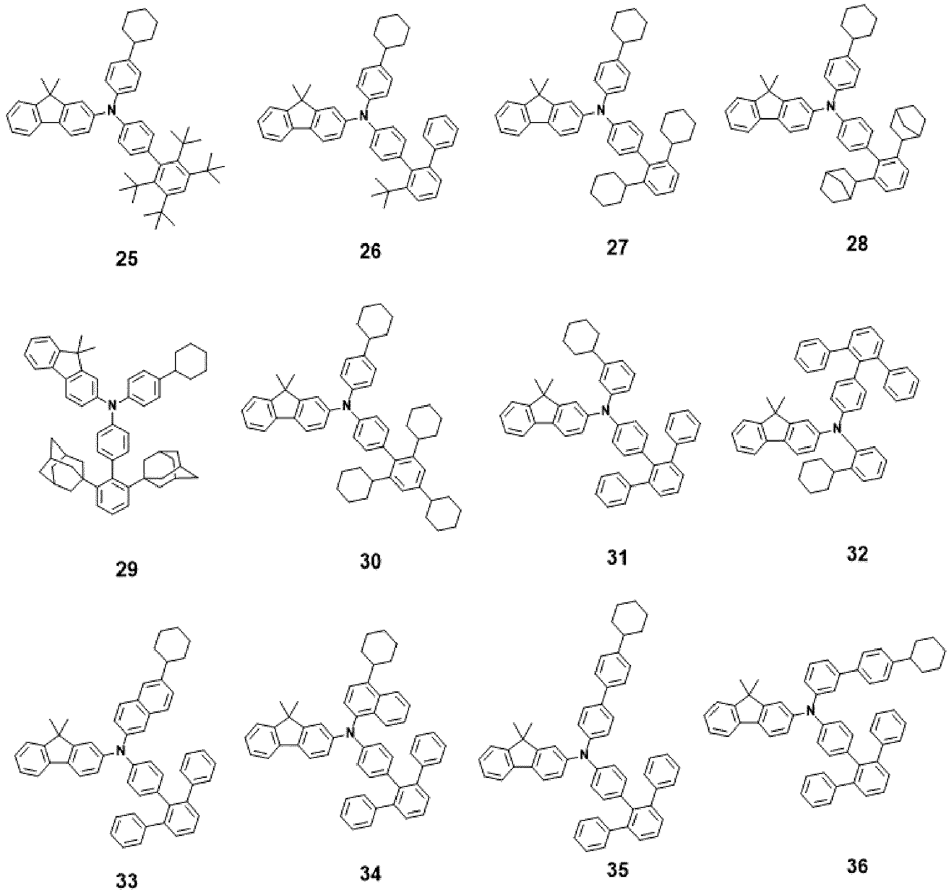

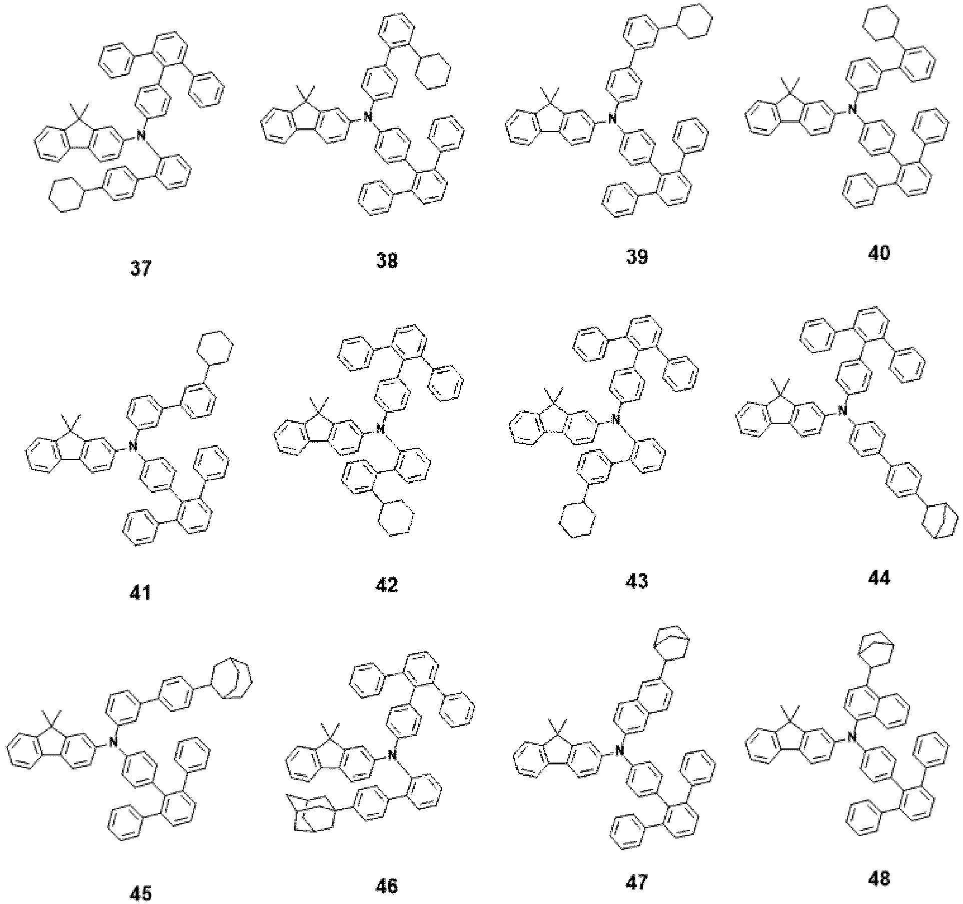

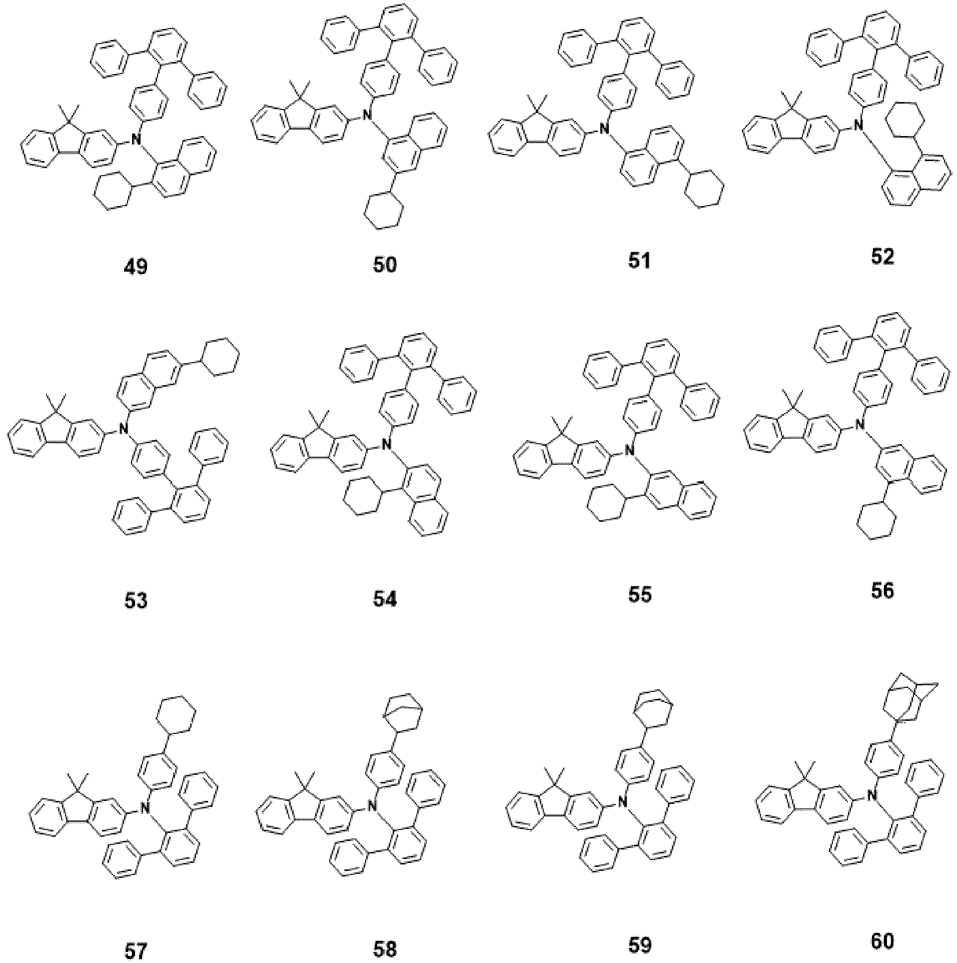

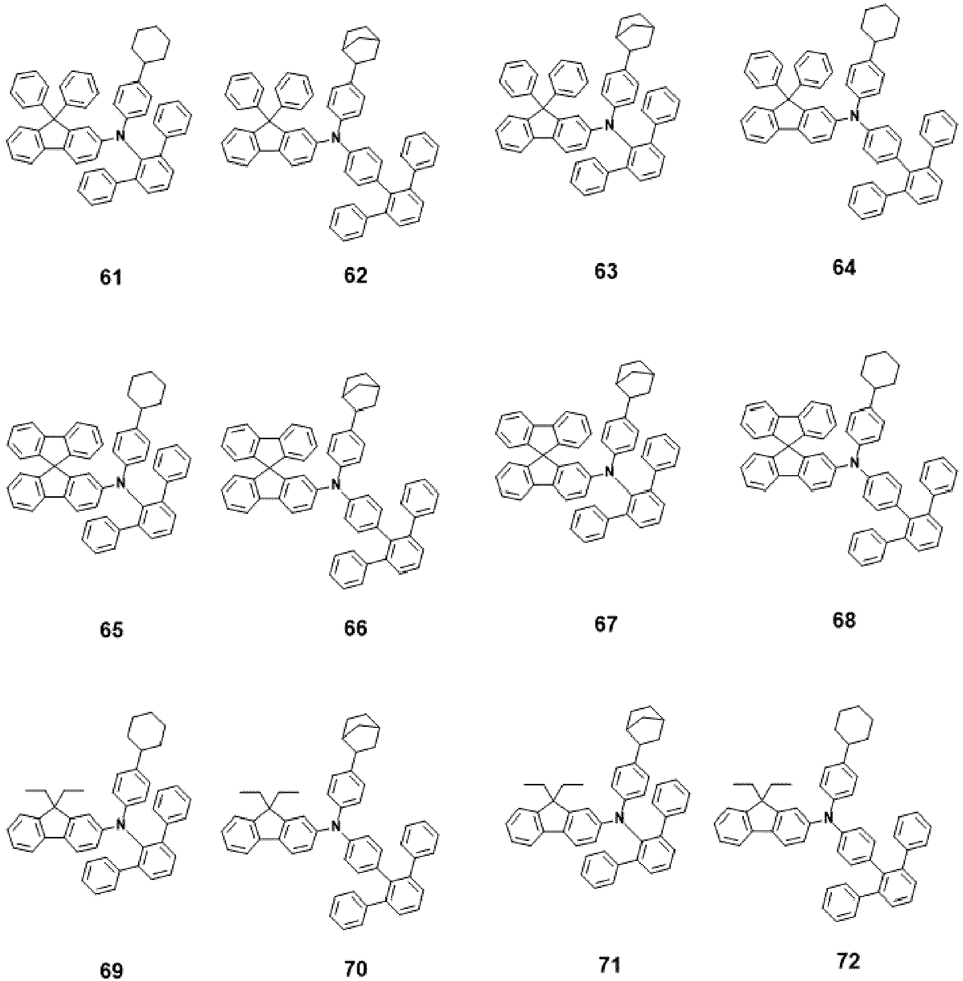

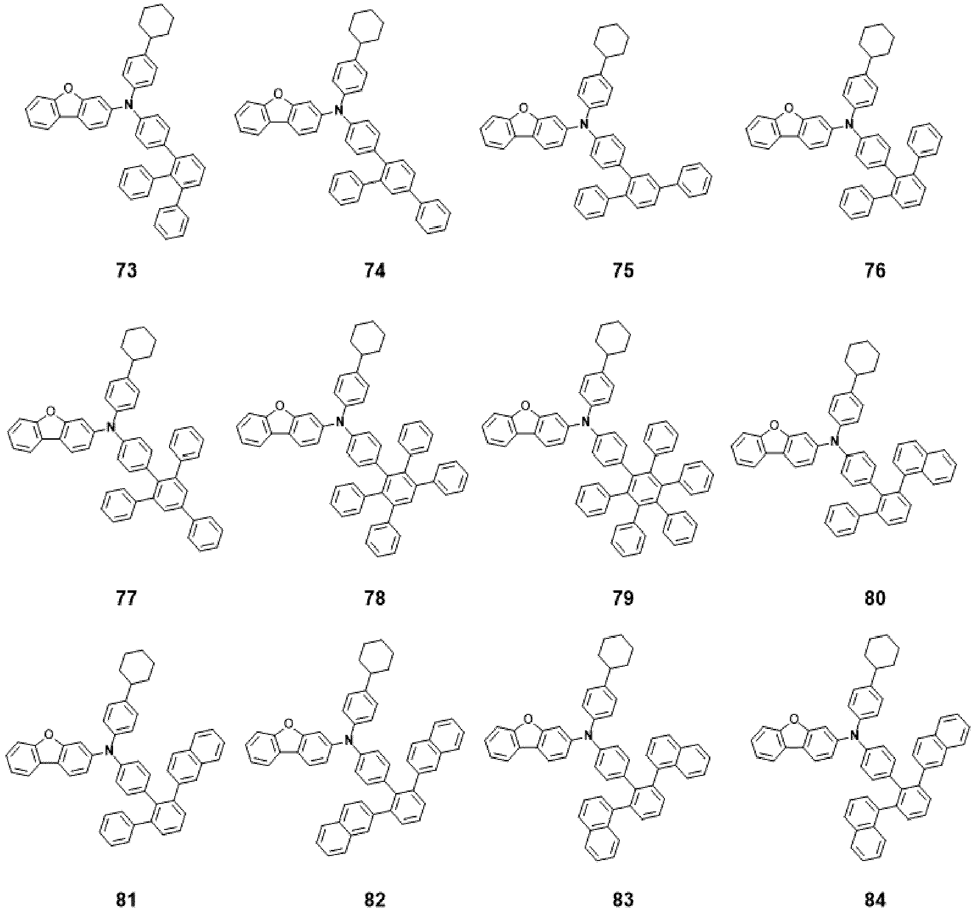

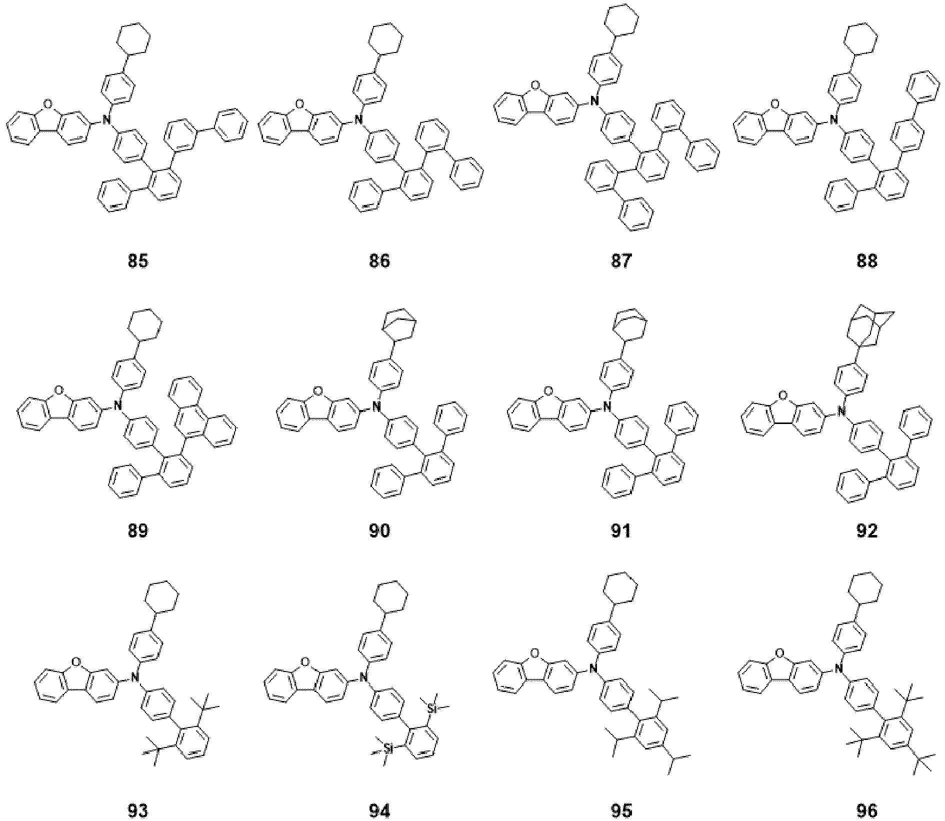

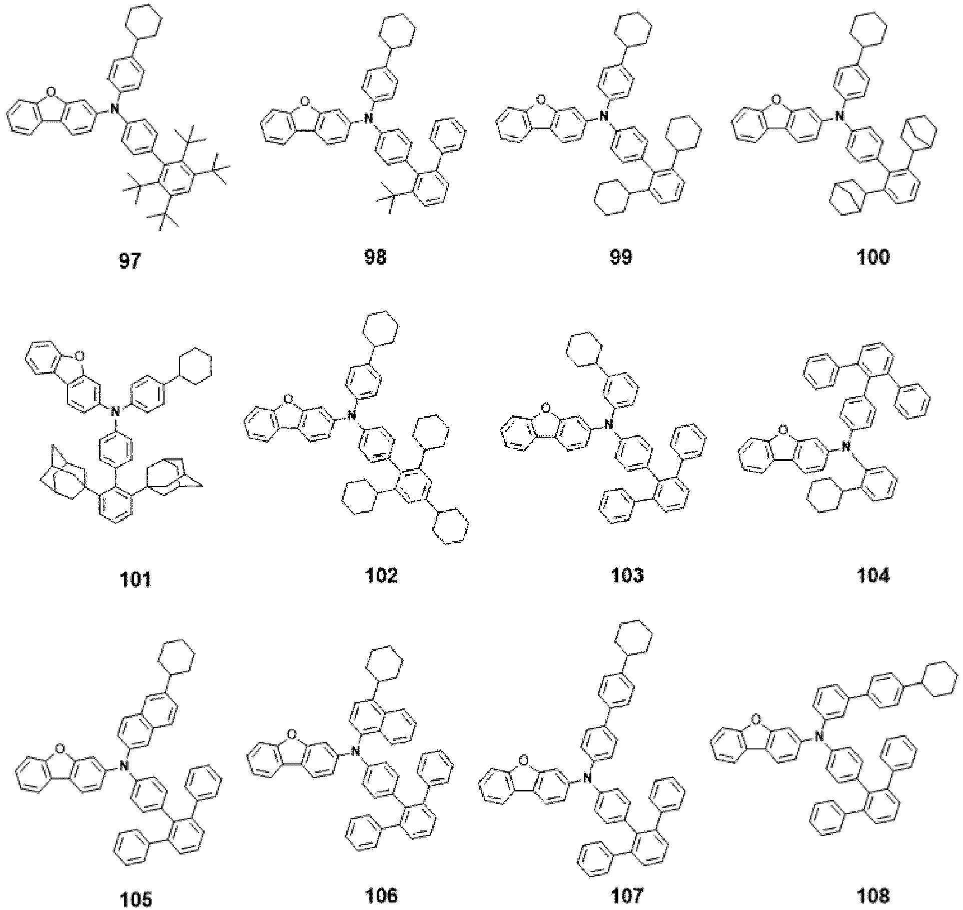

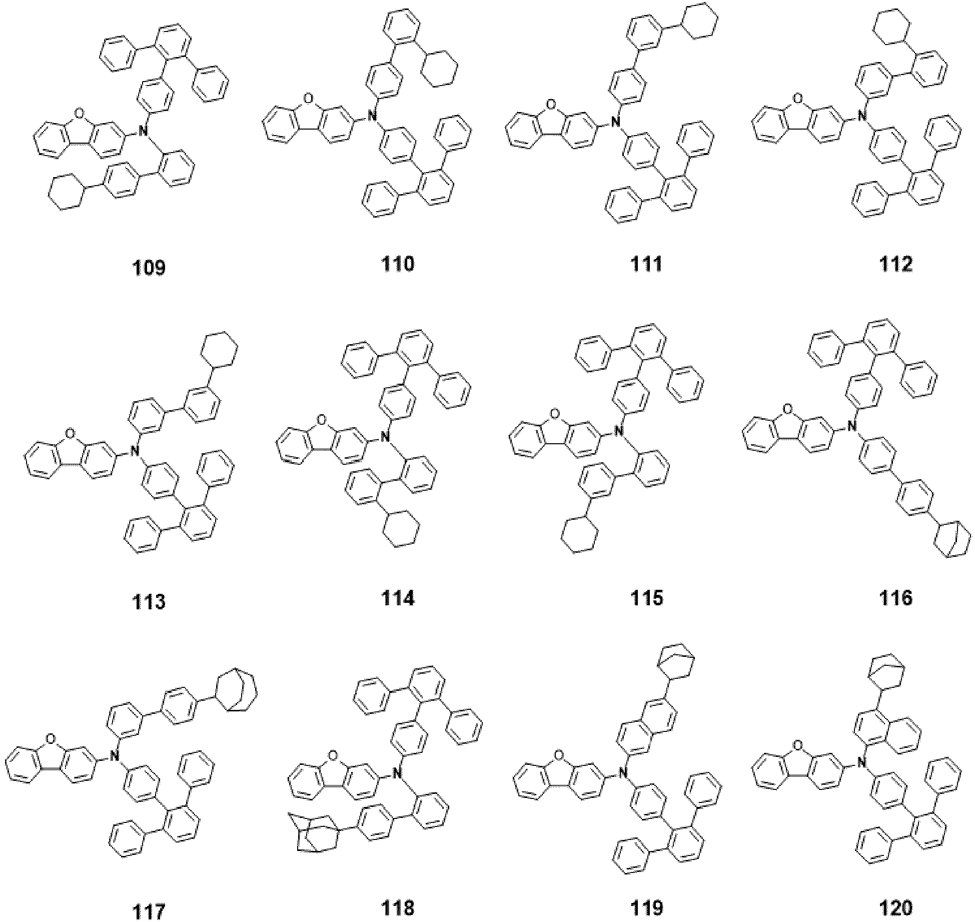

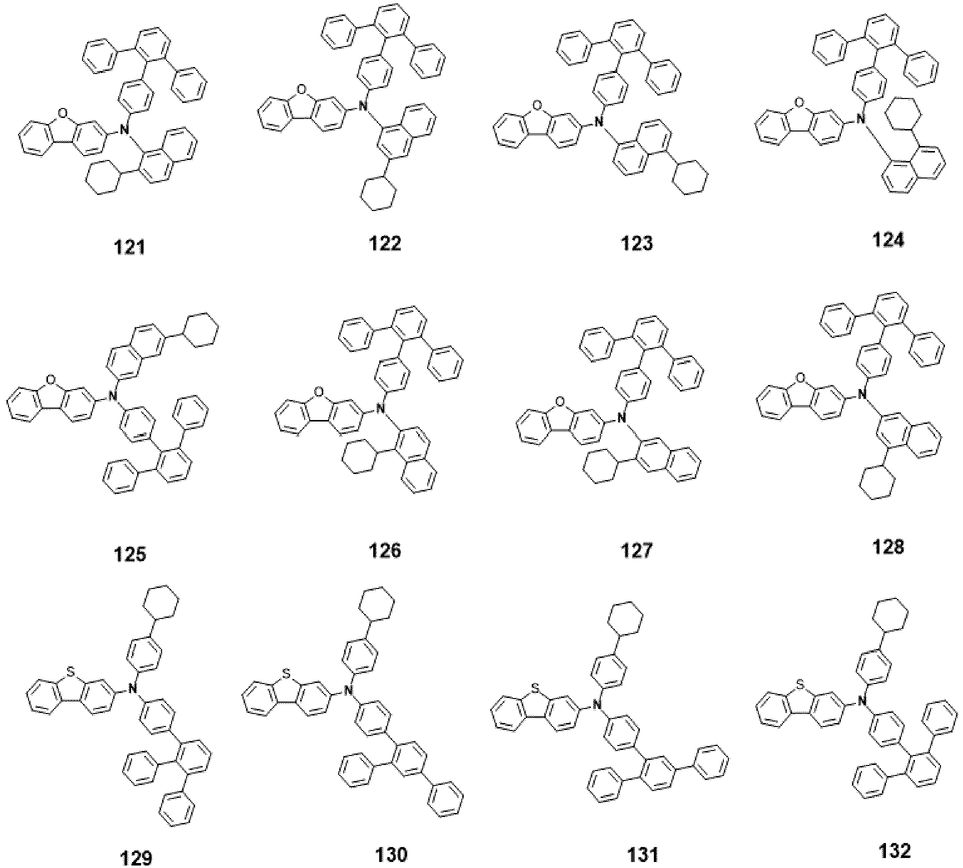

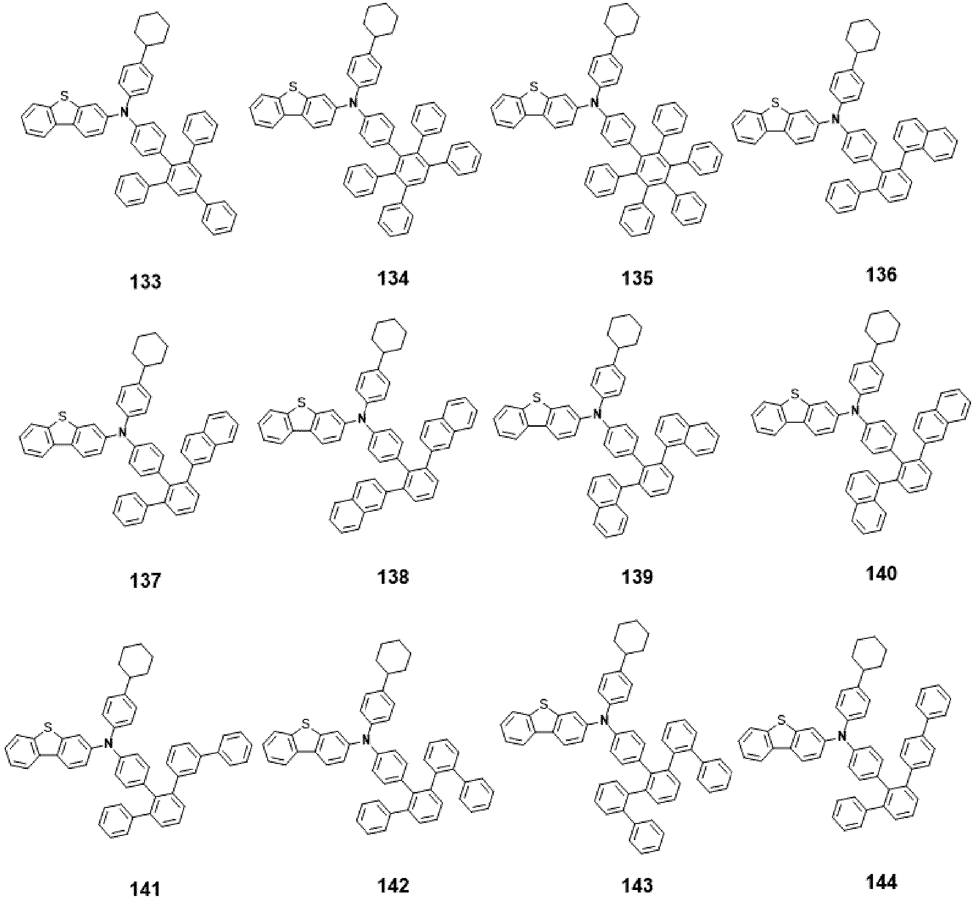

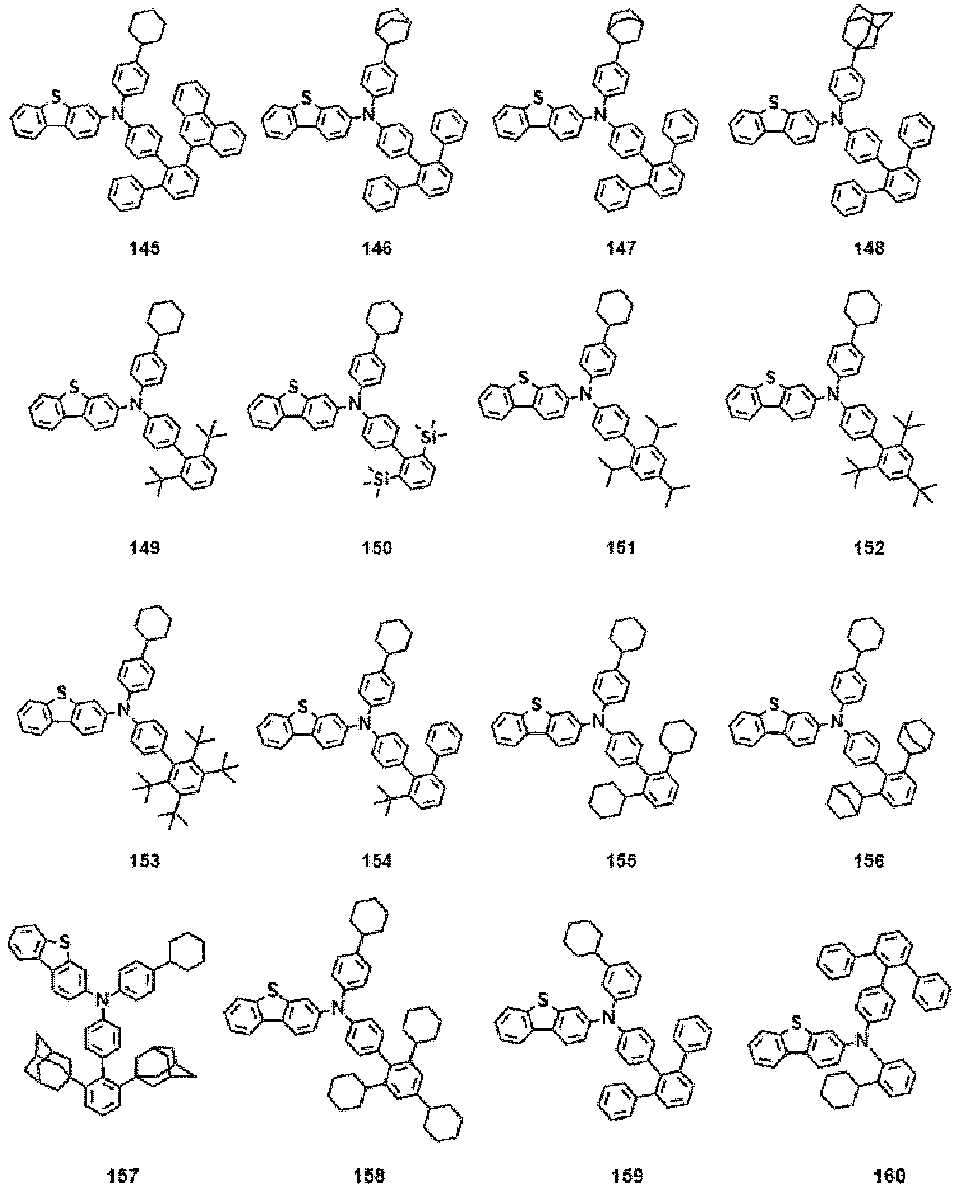

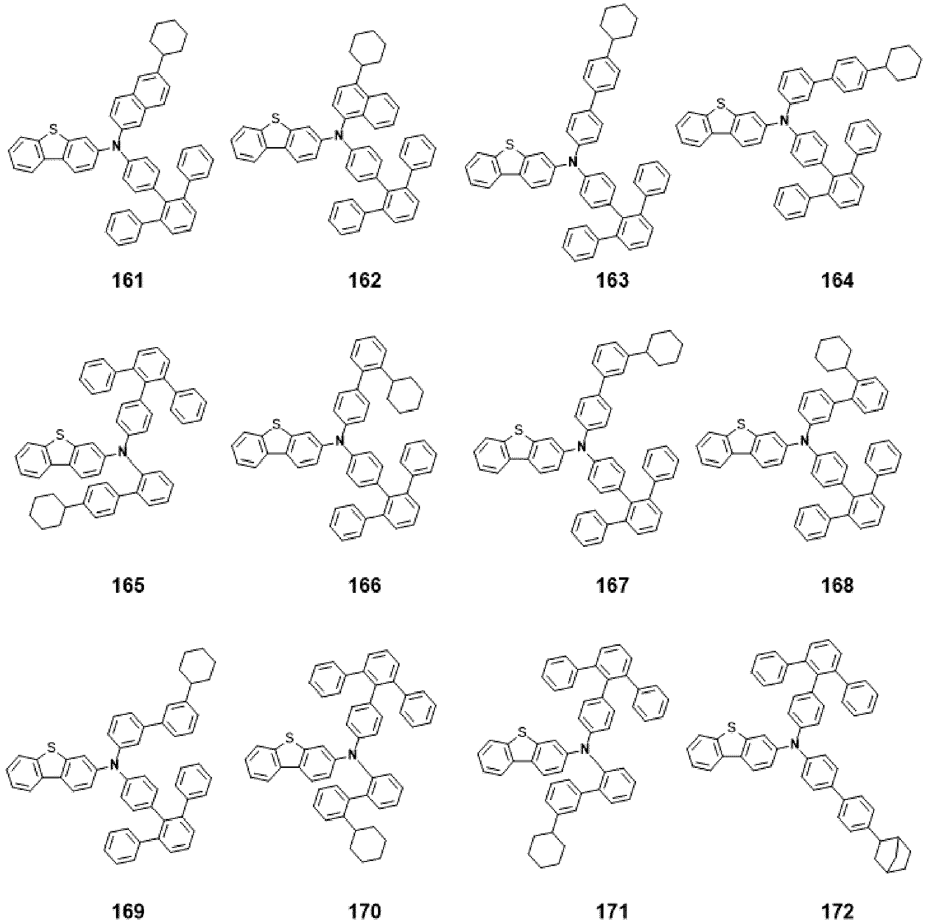

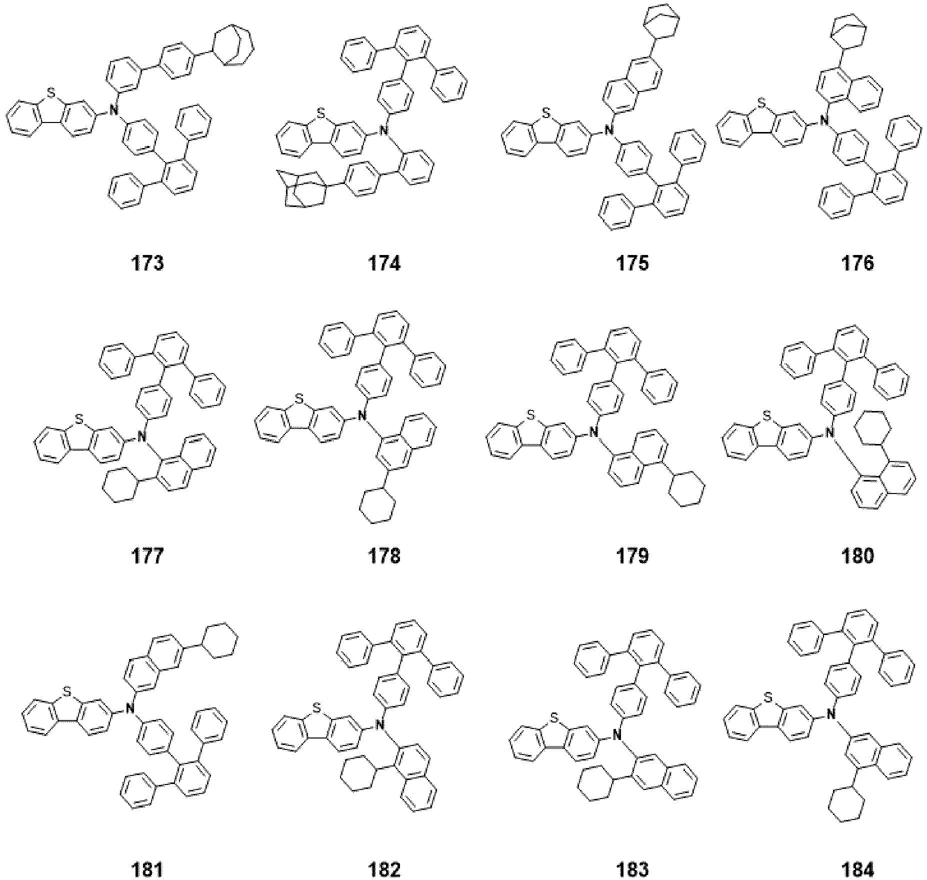

The amine compound according to one or more embodiments has a structure in which a first substituent, a second substituent, and a third substituent are linked to a core nitrogen atom, the first substituent has a fluorenyl moiety, a dibenzofuranyl moiety, or a dibenzothiophenyl moiety, the second substituent has a structure in which an aliphatic cycloalkyl group (such as a cyclohexyl group, a bicycloheptanyl group, a bicyclooctanyl group, a bicyclononanyl group, and/or an adamantyl group) is linked via an arylene or heteroarylene linker, and the third substituent has a structure in which at least two additional substituents are bonded to a phenyl group, and the additional substituent bonded to the phenyl group is a silyl group, an alkyl group, an aryl group, or a heteroaryl group. The amine compound of one or more embodiments has the first to third substituents, and thus may have an improvement in hole transport property and stability of the molecule while having a low refractive characteristic when applied to the hole transport layer. Thus, when the amine compound of one or more embodiments is applied to the light emitting device ED, the improvement of luminous efficiency and a long service life may be achieved.

When the amine compound of one or more embodiments is utilized in the hole transport region, the refractive index and the light extraction mode may be changed between the first electrode and the second electrode, and thus the external quantum efficiency may be increased. Accordingly, when the amine compound of one or more embodiments is utilized in the hole transport region, the luminous efficiency of the light emitting device may be increased and the service life of the light emitting device may be improved. In some embodiments, the amine compound of one or more embodiments has excellent or suitable heat resistance and durability, and thus the light emitting device of one or more embodiments may have an improvement in service life and luminous efficiency by including the amine compound of one or more embodiments as a material of the light emitting device.

In some embodiments, when the light emitting device ED of one or more embodiments includes the plurality of hole transport layers HTL1, HTL2, and HTL3, each of the first hole transport layer HTL1 adjacent to the first electrode EL1 and the third hole transport layer HTL3 adjacent to the emission layer EML may include the above-described amine compound represented by Formula 1 of one or more embodiments. In some embodiments, the second hole transport layer HTL2 disposed between the first hole transport layer HTL1 and the third hole transport layer HTL3 may include the amine derivative compound represented by Formula 5:

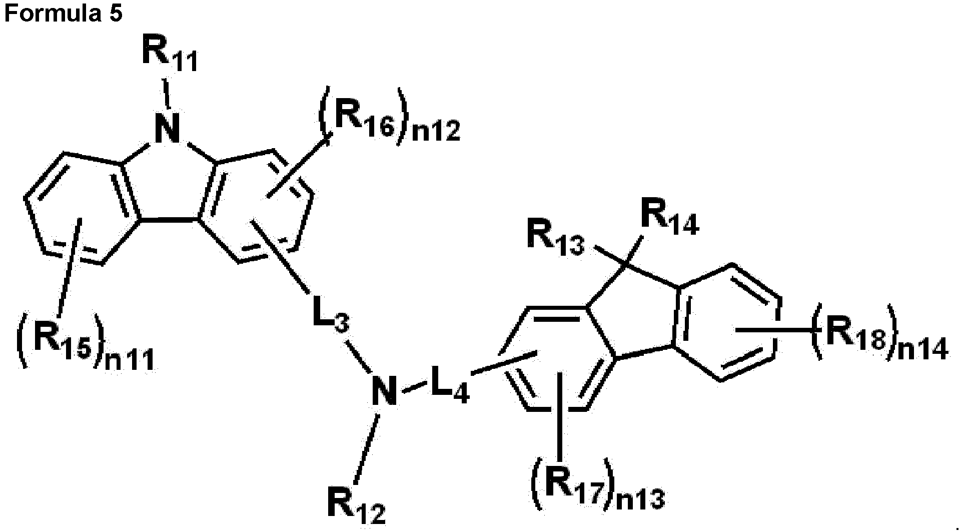

In Formula 5, L₃ and L₄ may each independently be a direct linkage, a substituted or unsubstituted arylene group having 6 to 30 ring-forming carbon atoms, or a substituted or unsubstituted heteroarylene group having 2 to 30 ring-forming carbon atoms.

In Formula 5, R₁₁ to R₁₄ may each independently be a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms. In some embodiments, each of R₁₁ to R₁₄ may be bonded to an adjacent group to form a ring. For example, R₁₁ to R₁₄ may each independently be a substituted or unsubstituted methyl group, or a substituted or unsubstituted phenyl group.

In Formula 5, R₁₅ to R₁₈ may each independently be a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. In some embodiments, R₁₅ to R₁₈ may each independently be bonded to an adjacent group to form a ring. For example, R₁₅ and R₁₈ may each independently be a hydrogen atom.

In Formula 5, n11 and n14 may each independently be an integer of 0 to 4, and n12 and n13 may each independently be an integer of 0 to 3.

When each of n11 and n14 is 0, the amine compound of one or more embodiments may not be substituted with R₁₅ or R₁₈. In Formula 1, the case where each of n11 and n14 is 4 and R₁₅'s and R₁₈'s are each hydrogen atoms may be the same as the case where each of n11 and n14 is 0. When each of n11 and n14 is an integer of 2 or more, a plurality of R₁₅'s and R₁₈'s may each be the same or at least one among the plurality of R₁₅'s and R₁₈'s may be different from the others.

When each of n12 and n13 is 0, the amine compound of one or more embodiments may not be substituted with R₁₆ or R₁₇. In Formula 1, the case where each of n12 and n13 is 3 and R₁₆'s and R₁₇'s are each hydrogen atoms may be the same as the case where each of n12 and n13 is 0. When each of n12 and n13 is an integer of 2 or more, a plurality of R₁₆'s and R₁₇'s may each be the same or at least one among the plurality of R₁₆'s and R₁₇'s may be different from the others.

In the amine derivative represented by Formula 5, R₁₂ may be an aryl group or a heteroaryl group. For example, R₁₂ may be a substituted or unsubstituted phenyl group, a substituted or unsubstituted naphthyl group, a substituted or unsubstituted biphenyl group, a substituted or unsubstituted dibenzofuran group, or a substituted or unsubstituted dibenzothiophene group.

In one or more embodiments, the amine derivative compound represented by Formula 5 may be represented by Formula 6-1 or Formula 6-2:

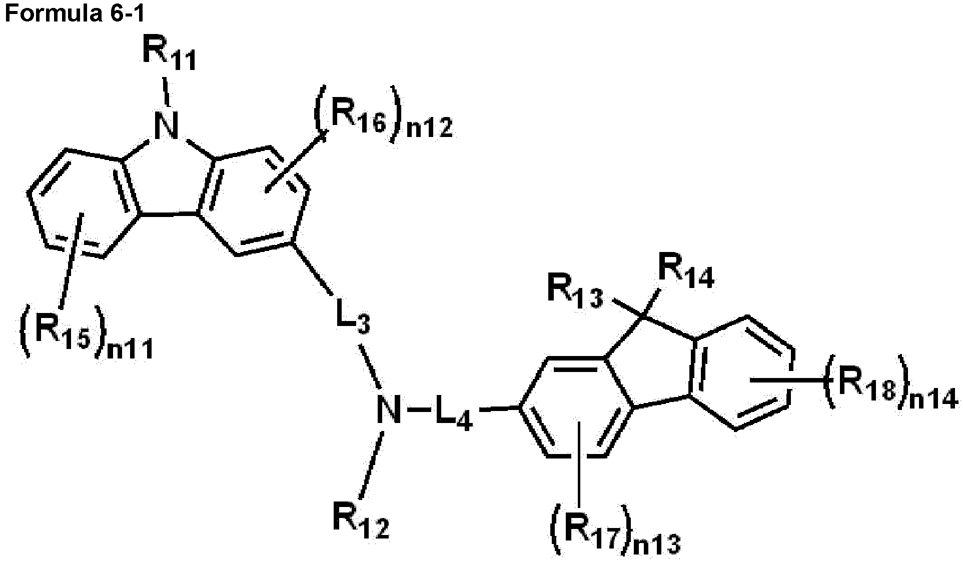

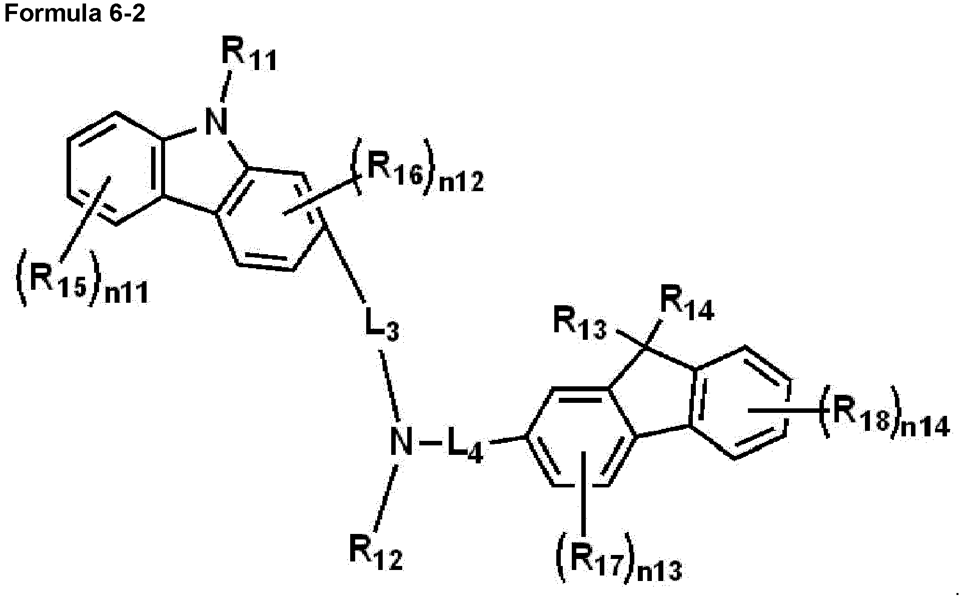

Formula 6-1 and Formula 6-2 represent the cases where in Formula 5, the position at which the carbazole moiety is linked to L₃ and the position at which the fluorene moiety is linked to L₄ are specified. Formula 6-1 corresponds to the case where in Formula 5, the third carbon position of the carbazole moiety is the position linked to (e.g., the binding site for) L₃. Formula 6-2 corresponds to the case where in Formula 5, the second carbon position of the carbazole moiety is the position linked to (e.g., the binding site for) L₃. Formulae 6-1 and 6-2 each correspond to the case where in Formula 5, the second carbon position of the fluorene moiety is the position linked to (e.g., the binding site for) L₄.

In some embodiments, in Formula 6-1 and Formula 6-2, the same descriptions as those provided in Formula 5 may be applied to L₃, L₄, R₁₁ to R₁₄, R₁₅ to R₁₈, and n11 to n13.

The amine derivative compound represented by Formula 5 may be represented by any one selected from among the compounds represented by Compound Group 2. For example, the second hole transport layer HTL2 may include at least one selected from among the compounds represented by Compound Group 2:

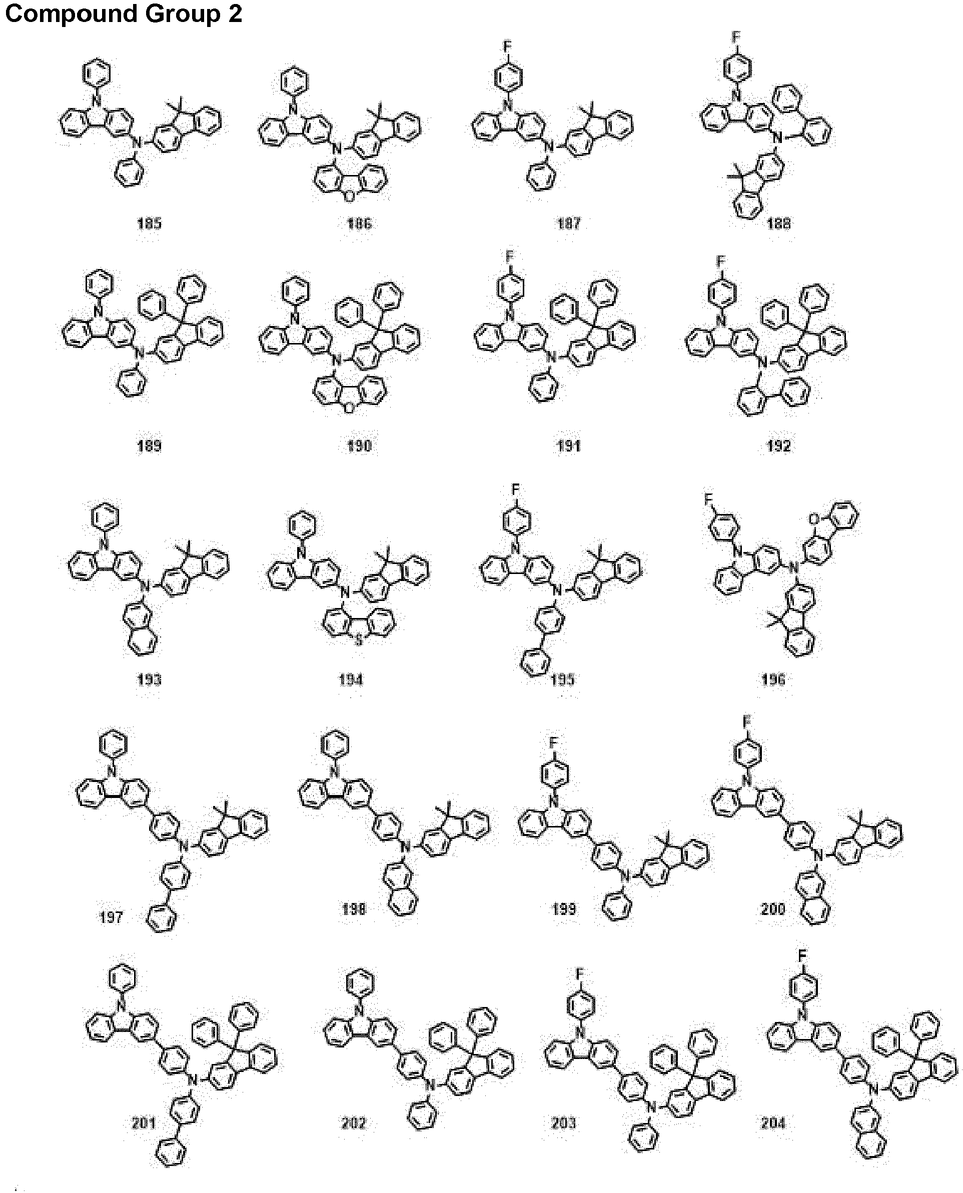

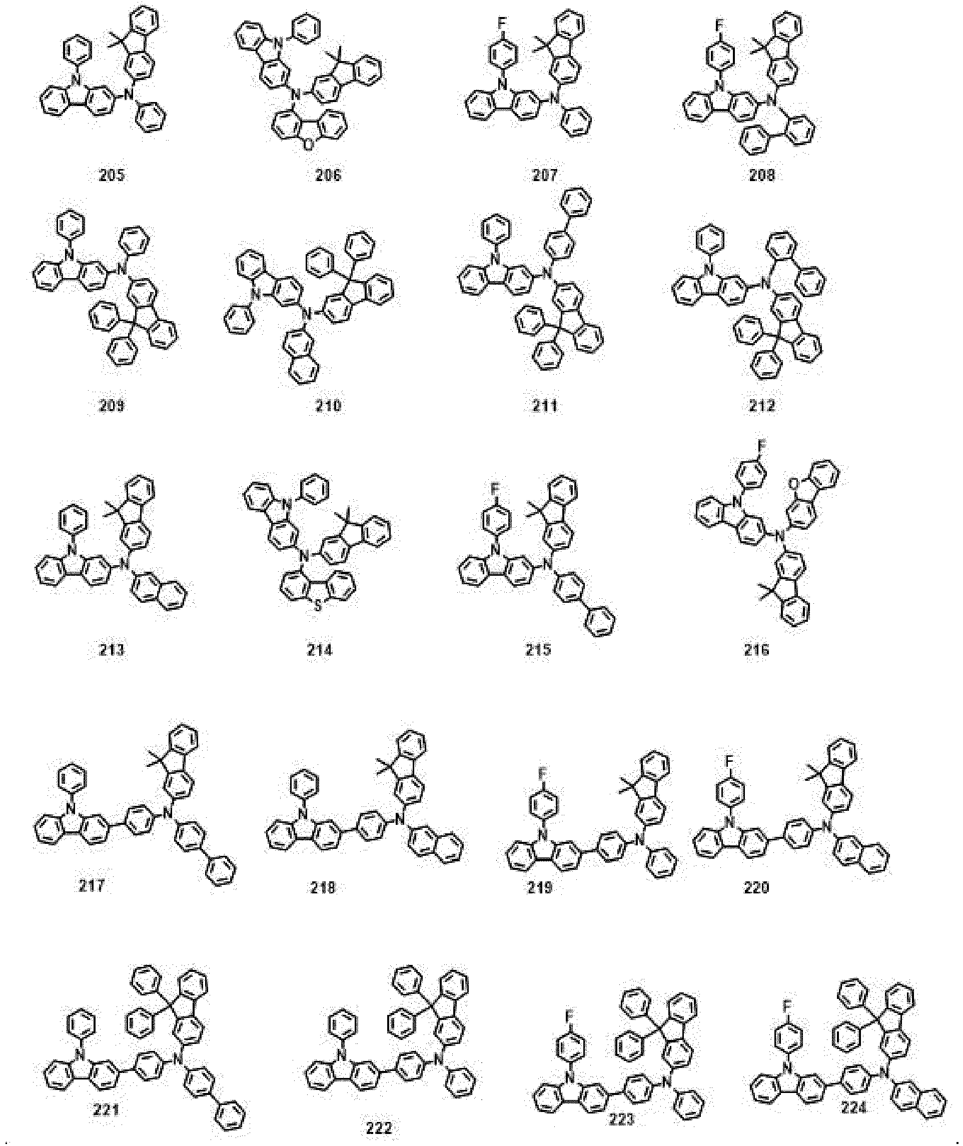

In some embodiments, the light emitting device ED of one or more embodiments may further include materials for the hole transport region, which will be described in more detail herein below, in the hole transport region HTR in addition to the above-described amine compound of one or more embodiments and the amine derivative compound represented by Formula 5.

The hole transport region HTR may include a compound represented by Formula H-1:

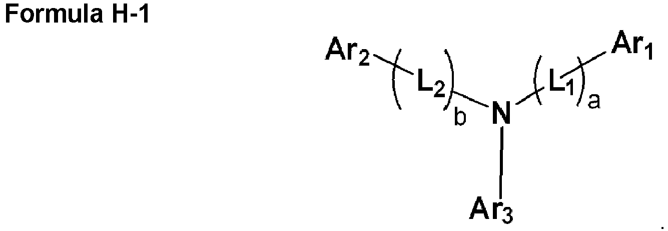

In Formula H-1, L₁ and L₂ may each independently be a direct linkage, a substituted or unsubstituted arylene group having 6 to 30 ring-forming carbon atoms, or a substituted or unsubstituted heteroarylene group having 2 to 30 ring-forming carbon atoms. a and b may each independently be an integer of 0 to 10. In some embodiments, when a or b is an integer of 2 or greater, a plurality of L₁'s and L₂'s may each independently be a substituted or unsubstituted arylene group having 6 to 30 ring-forming carbon atoms, or a substituted or unsubstituted heteroarylene group having 2 to 30 ring-forming carbon atoms.

In Formula H-1, Ar₁ and Ar₂ may each independently be a substituted or unsubstituted aryl group having 6 to 30 ring-forming carbon atoms, or a substituted or unsubstituted heteroaryl group having 2 to 30 ring-forming carbon atoms. In some embodiments, in Formula H-1, Ar₃ may be a substituted or unsubstituted aryl group having 6 to 30 ring-forming carbon atoms.

The compound represented by Formula H-1 may be a monoamine compound. In some embodiments, the compound represented by Formula H-1 may be a diamine compound in which at least one selected from among Ar₁ to Ar₃ includes the amine group as a substituent. In some embodiments, the compound represented by Formula H-1 may be a carbazole-based compound including a substituted or unsubstituted carbazole group in at least one of Ar₁ or Ar₂, or a fluorene-based compound including a substituted or unsubstituted fluorene group in at least one of Ar₁ or Ar₂.

The compound represented by Formula H-1 may be represented by any one selected from among the compounds of Compound Group H. However, the compounds listed in Compound Group H are examples, and the compounds represented by Formula H-1 are not limited to those represented by Compound Group H:

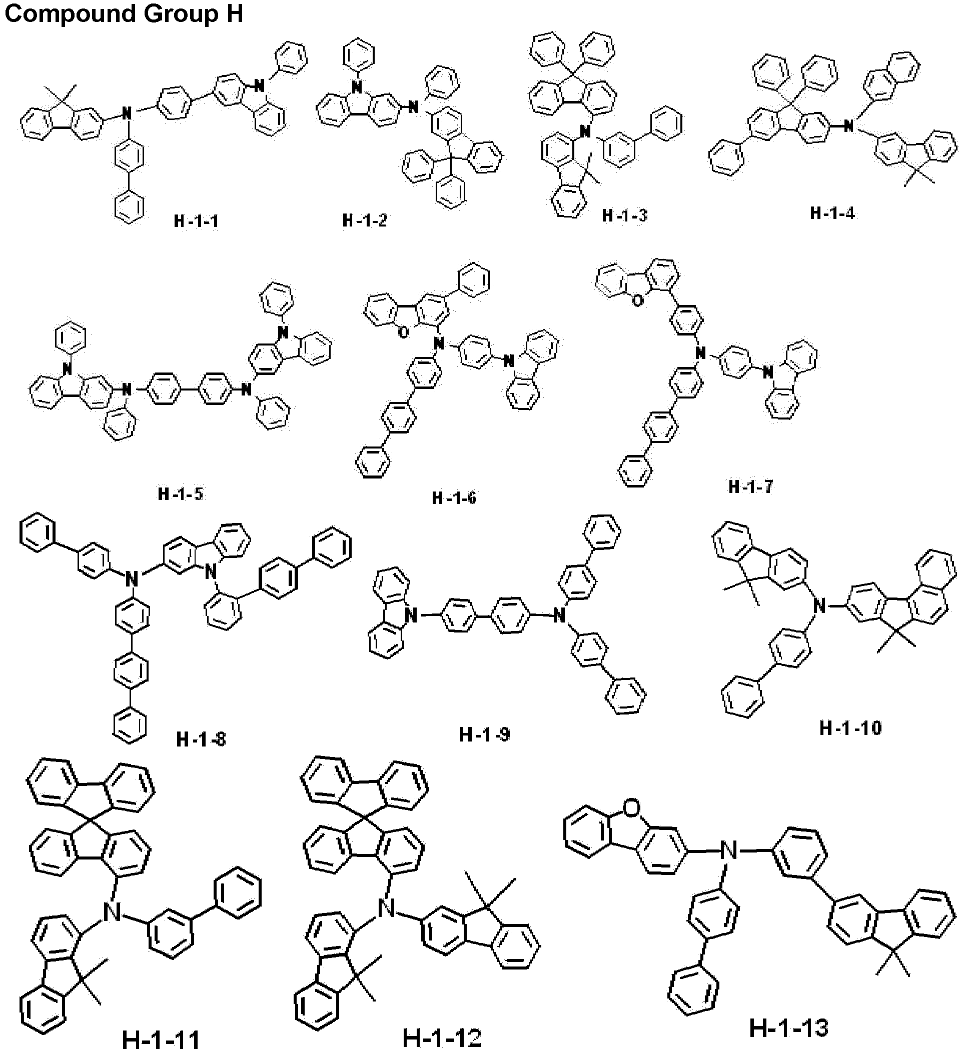

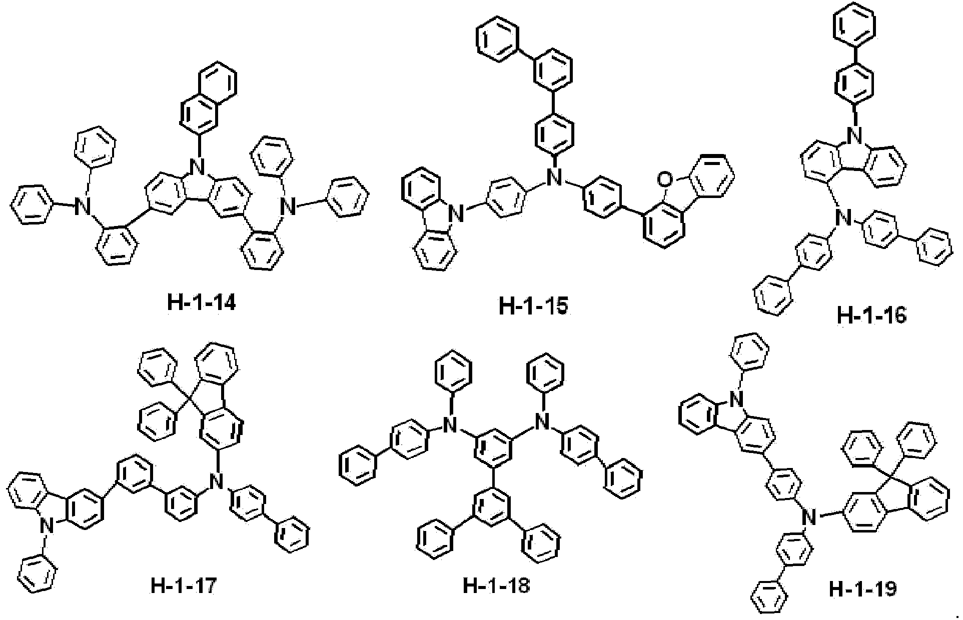

The hole transport region HTR may include a phthalocyanine compound (such as copper phthalocyanine), N¹,N^{1'}-([1,1'-biphenyl]-4,4'-diyl)bis(N¹-phenyl-N⁴,N⁴-di-m-tolylbenzene-1,4-diamine) (DNTPD), 4,4',4"-[tris(3-methylphenyl)phenylamino] triphenylamine (m-MTDATA), 4,4'4"-tris(N,N-diphenylamino)triphenylamine (TDATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonic acid (PANI/CSA), polyaniline/poly(4-styrenesulfonate) (PANI/PSS), N,N'-di(naphthalene-1-yl)-N,N'-diphenyl-benzidine (NPB), triphenylamine-containing polyetherketone (TPAPEK), 4-isopropyl-4'-methyldiphenyliodonium [tetrakis(pentafluorophenyl)borate], dipyrazino[2,3-f: 2',3'-h]quinoxaline-2,3,6,7,10,11-hexacarbonitrile (HATCN), *etc.*

The hole transport region HTR may include a carbazole-based derivative (such as N-phenyl carbazole and/or polyvinyl carbazole), a fluorene-based derivative, a triphenylamine-based derivative (such as N,N'-bis(3-methylphenyl)-N,N'-diphenyl-[1,1-biphenyl]-4,4'-diamine (TPD) and/or 4,4',4"-tris(N-carbazolyl)triphenylamine (TCTA)), N,N'-di(naphthalene-l-yl)-N,N'-diphenyl-benzidine (NPB), 4,4'-cyclohexylidene bis[N,N-bis(4-methylphenyl]benzenamine] (TAPC), 4,4'-bis[N,N'-(3-tolyl)amino]-3,3'-dimethylbiphenyl (HMTPD), 1,3-bis(N-carbazolyl)benzene (mCP), *etc.*

In some embodiments, the hole transport region HTR may include 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), *etc.*

The hole transport region HTR may include the above-described compounds of the hole transport region in at least one of a hole injection layer HIL, a hole transport layer HTL, or an electron blocking layer EBL.

The thickness of the hole transport region HTR may be from about 10 nm (100 Å) to about 1,000 nm (10,000 Å), for example, from about 10 nm (100 Å) to about 500 nm (5,000 Å). When the hole transport region HTR includes the hole injection layer HIL, the hole injection layer HIL may have, for example, a thickness of about 3 nm (30 Å) to about 100 nm (1,000 Å). When the hole transport region HTR includes the hole transport layer HTL, the hole transport layer HTL may have a thickness of about 3 nm (30 Å) to about 100 nm (1,000 Å). For example, when the hole transport region HTR includes the electron blocking layer EBL, the electron blocking layer EBL may have a thickness of about 1 nm (10 Å) to about 100 nm (1,000 Å). When the thicknesses of the hole transport region HTR, the hole injection layer HIL, the hole transport layer HTL and the electron blocking layer EBL satisfy the above-described ranges, satisfactory or suitable hole transport properties may be achieved without a substantial increase in driving voltage.

The hole transport region HTR may further include a charge generating material to increase conductivity in addition to the-described materials. The charge generating material may be dispersed uniformly or non-uniformly in the hole transport region HTR. The charge generating material may be, for example, a p-dopant. The p-dopant may include at least one of a halogenated metal compound, a quinone derivative, a metal oxide, or a cyano group-containing compound, but the embodiment of the present disclosure is not limited thereto. For example, the p-dopant may include a metal halide compound (such as Cul and/or RbI), a quinone derivative (such as tetracyanoquinodimethane (TCNQ) and/or
2,3,5,6-tetrafluoro-7,7'8,8-tetracyanoquinodimethane (F4-TCNQ)), a metal oxide (such as tungsten oxide and/or molybdenum oxide), a cyano group-containing compound (such as dipyrazino[2,3-f: 2',3'-h] quinoxaline-2,3,6,7,10,11-hexacarbonitrile (HATCN) and/or
4-[[2,3-bis[cyano-(4-cyano-2,3,5,6-tetrafluorophenyl)methylidene]cyclopropylidene]-cya nomethyl]-2,3,5,6-tetrafluorobenzonitrile (NDP9)), *etc.,* but the embodiment of the present disclosure is not limited thereto.

As described above, the hole transport region HTR may further include at least one of the buffer layer or the electron blocking layer EBL, in addition to the hole injection layer HIL and the hole transport layer HTL. The buffer layer may compensate for a resonance distance according to the wavelength of light emitted from the emission layer EML and may thus increase light emission efficiency. A material that may be contained in the hole transport region HTR may be utilized as a material to be contained in the buffer layer. The electron blocking layer EBL is a layer that serves to prevent or reduce the electron injection from the electron transport region ETR to the hole transport region HTR.

The emission layer EML is provided on the hole transport region HTR. The emission layer EML may have a thickness of, for example, about 10 nm (100 Å) to about 100 nm (1,000 Å) or about 10 nm (100 Å) to about 30 nm (300 Å). The emission layer EML may have a single layer formed of a single material, a single layer formed of a plurality of different materials, or a multilayer structure having a plurality of layers formed of a plurality of different materials.

In the light emitting device ED of one or more embodiments, the emission layer EML may include an anthracene derivative, a pyrene derivative, a fluoranthene derivative, a chrysene derivative, a dehydrobenzanthracene derivative, and/or a triphenylene derivative. For example, the emission layer EML may include the anthracene derivative and/or the pyrene derivative.

In each light emitting device ED of embodiments illustrated in FIGS. 3 to 6, the emission layer EML may include a host and a dopant, and the emission layer EML may include a compound represented by Formula E-1. The compound represented by Formula E-1 may be utilized as a fluorescent host material.

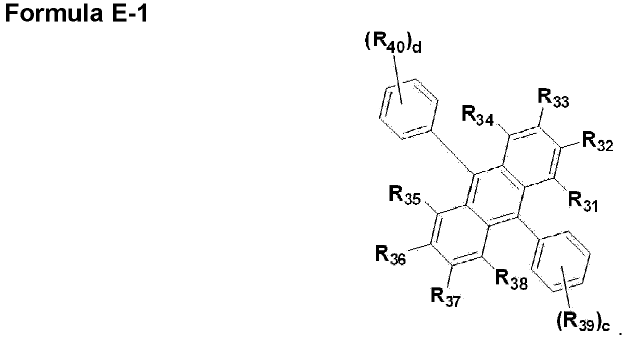

In Formula E-1, R₃₁ to R₄₀ may each independently be a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 1 to 10 carbon atoms, a substituted or unsubstituted aryl group having 6 to 30 ring-forming carbon atoms, or a substituted or unsubstituted heteroaryl group having 2 to 30 ring-forming carbon atoms, and/or may be bonded to an adjacent group to form a ring. In some embodiments, R₃₁ to R₄₀ may be bonded to an adjacent group to form a saturated hydrocarbon ring, an unsaturated hydrocarbon ring, a saturated heterocycle, or an unsaturated heterocycle.

In Formula E-1, c and d may each independently be an integer of 0 to 5.

Formula E-1 may be represented by any one selected from among Compound E1 to Compound E19:

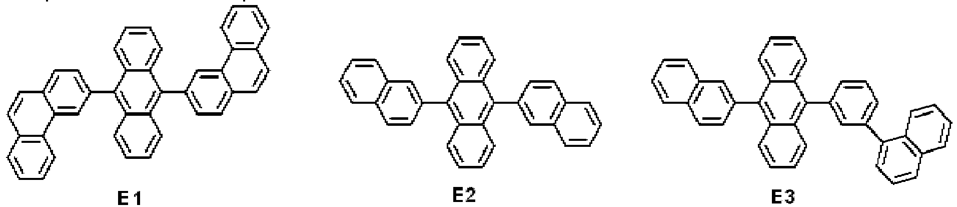

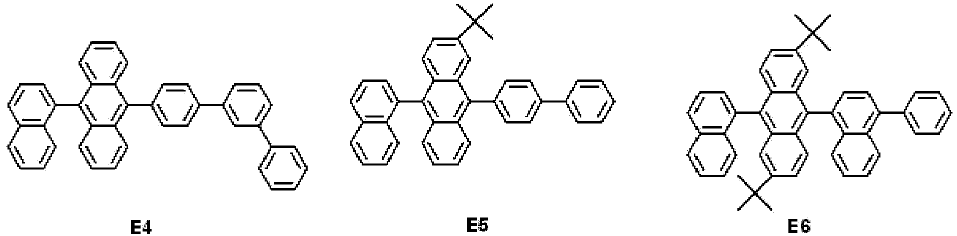

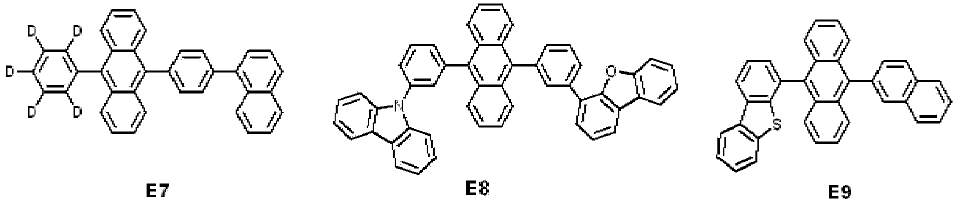

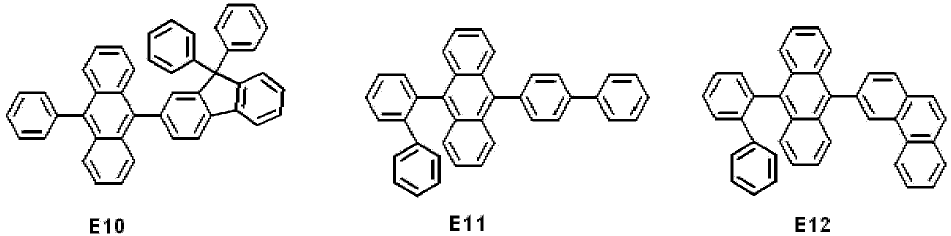

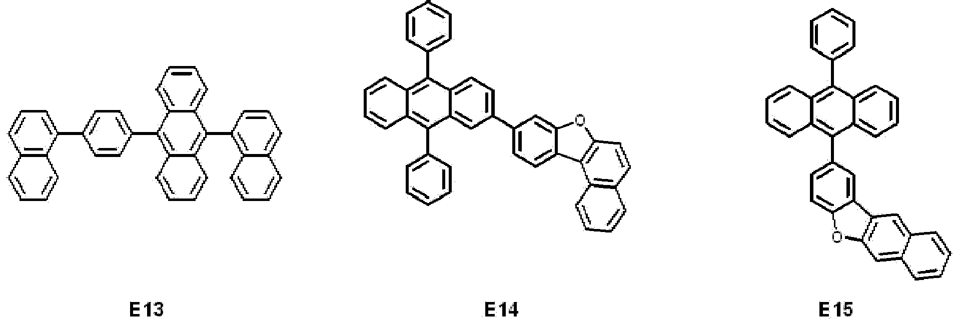

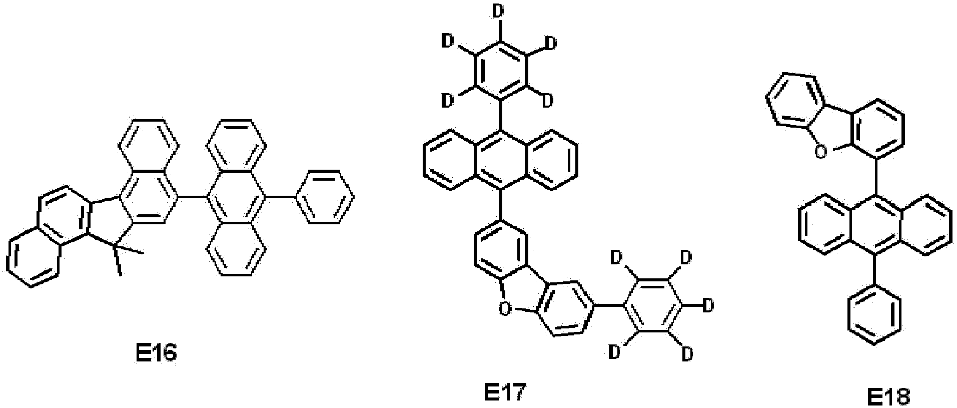

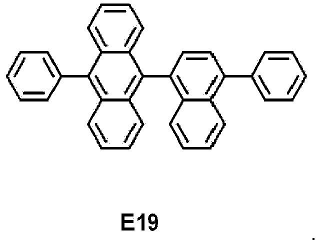

In one or more embodiments, the emission layer EML may include a compound represented by Formula E-2a or Formula E-2b. The compound represented by Formula E-2a or Formula E-2b may be utilized as a phosphorescent host material.

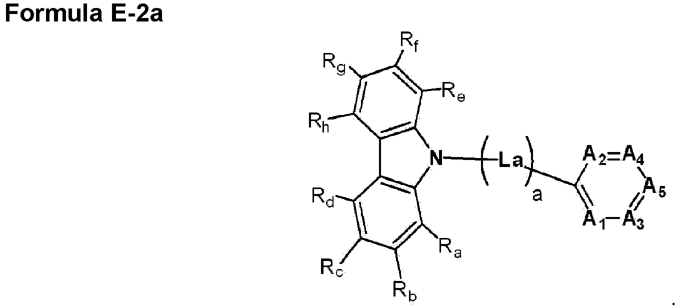

In Formula E-2a, "a" may be an integer of 0 to 10, Lₐ may be a direct linkage, a substituted or unsubstituted arylene group having 6 to 30 ring-forming carbon atoms, or a substituted or unsubstituted heteroarylene group having 2 to 30 ring-forming carbon atoms. In some embodiments, when a is an integer of 2 or more, a plurality of Lₐ's may each independently be a substituted or unsubstituted arylene group having 6 to 30 ring-forming carbon atoms, or a substituted or unsubstituted heteroarylene group having 2 to 30 ring-forming carbon atoms.

In some embodiments, in Formula E-2a, A₁ to A₅ may each independently be N or CRᵢ. Rₐ to Rᵢ may each independently be a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and/or may be bonded to an adjacent group to form a ring. Rₐ to Rᵢ may be bonded to an adjacent group to form a hydrocarbon ring or a heterocycle containing N, O, S, *etc.* as a ring-forming atom.

In some embodiments, in Formula E-2a, two or three selected from among A₁ to A₅ may be N, and the rest may be CRᵢ.

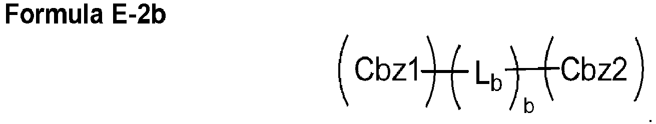

In Formula E-2b, Cbz1 and Cbz2 may each independently be an unsubstituted carbazole group, or a carbazole group substituted with an aryl group having 6 to 30 ring-forming carbon atoms. L_{b} may be a direct linkage, a substituted or unsubstituted arylene group having 6 to 30 ring-forming carbon atoms, or a substituted or unsubstituted heteroarylene group having 2 to 30 ring-forming carbon atoms. In some embodiments, b may be an integer of 0 to 10, and when b is an integer of 2 or more, a plurality of Lb's may each independently be a substituted or unsubstituted arylene group having 6 to 30 ring-forming carbon atoms, or a substituted or unsubstituted heteroarylene group having 2 to 30 ring-forming carbon atoms.

The compound represented by Formula E-2a or Formula E-2b may be represented by any one selected from among the compounds of Compound Group E-2. However, the compounds listed in Compound Group E-2 are examples, and the compound represented by Formula E-2a or Formula E-2b is not limited to those represented in Compound Group E-2.

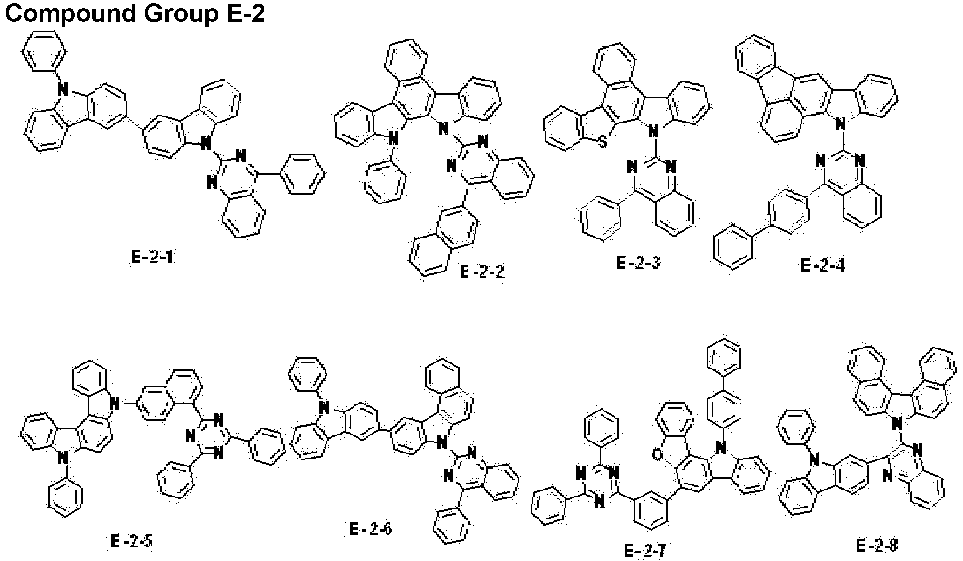

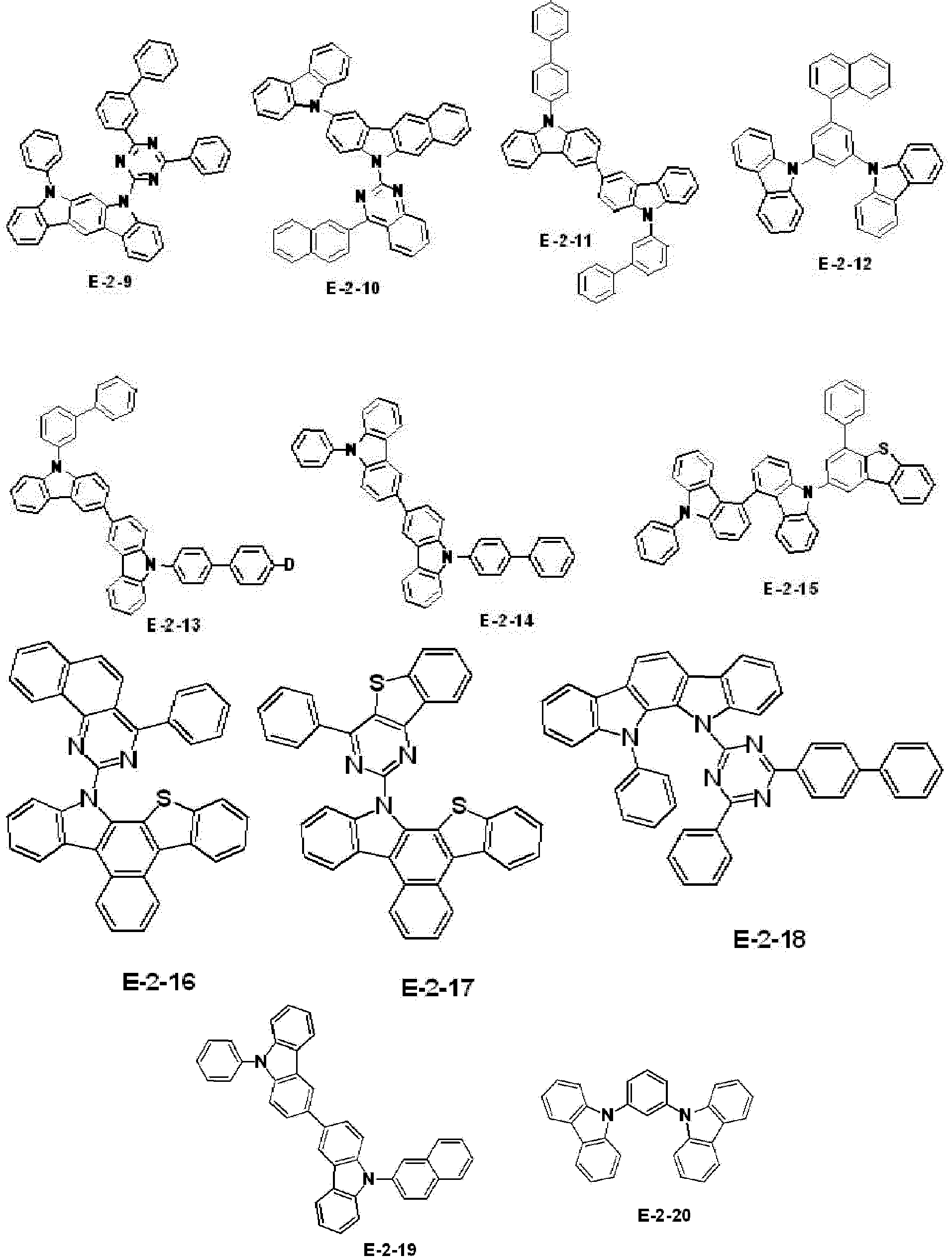

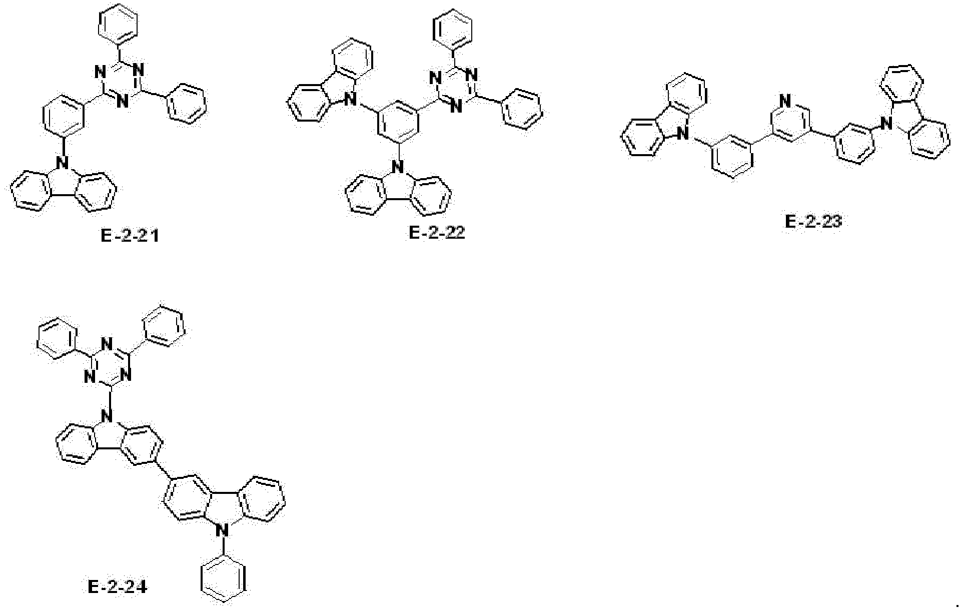

The emission layer EML may further include a material suitable in the art as a host material. For example, the emission layer EML may include, as a host material, at least one of bis(4-(9H-carbazol-9-yl)phenyl)diphenylsilane (BCPDS), (4-(1-(4-(diphenylamino)phenyl)cyclohexyl)phenyl)diphenyl-phosphine oxide (POPCPA), bis[2-(diphenylphosphino)phenyl]ether oxide (DPEPO), 4,4'-bis(N-carbazolyl)-1,1'-biphenyl (CBP), 1,3-bis(carbazol-9-yl)benzene (mCP), 2,8-bis(diphenylphosphoryl)dibenzo[b,d]furan (PPF), 4,4',4"-tris(carbazol-9-yl)-triphenylamine (TCTA), or 1,3,5-tris(1-phenyl-1H-benzo[d]imidazole-2-yl)benzene (TPBi). However, the embodiment of the present disclosure is not limited thereto, for example, tris(8-hydroxyquinolino)aluminum (Alq₃), 9,10-di(naphthalene-2-yl)anthracene (ADN), 2-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane (DPSiO₃), octaphenylcyclotetra siloxane (DPSiO₄), *etc.* may be utilized as a host material.

The emission layer EML may include a compound represented by Formula M-a or Formula M-b. The compound represented by Formula M-a or Formula M-b may be utilized as a phosphorescent dopant material.

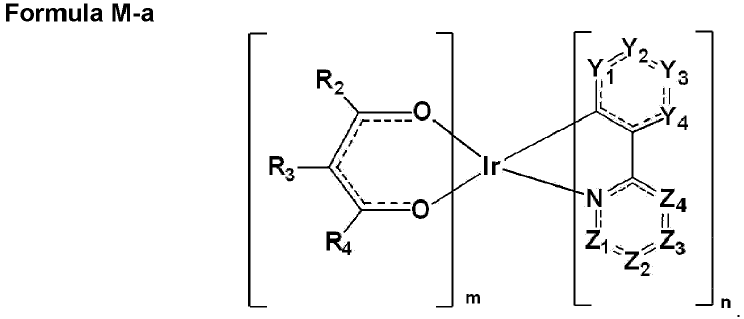

In Formula M-a, Y₁ to Y₄ and Z₁ to Z₄ may each independently be CR₁ or N, R₁ to R₄ may each independently be a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and/or may be bonded to an adjacent group to form a ring. In Formula M-a, m is 0 or 1, and n is 2 or 3. In Formula M-a, when m is 0, n is 3, and when m is 1, n is 2.

The compound represented by Formula M-a may be utilized as a phosphorescent dopant.

The compound represented by Formula M-a may be represented by any one selected from among Compound M-a1 to Compound M-a25. However, Compounds M-a1 to M-a25 are examples, and the compound represented by Formula M-a is not limited to those represented by Compounds M-a1 to M-a25.

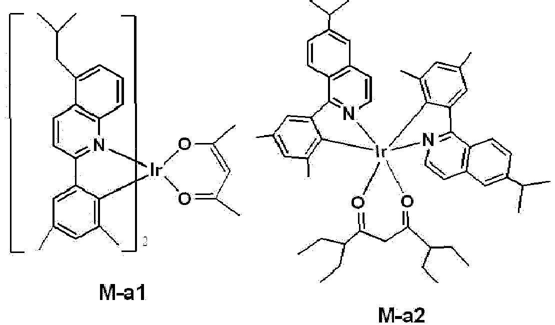

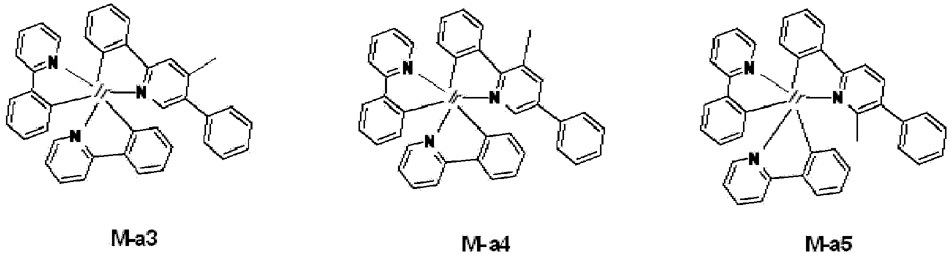

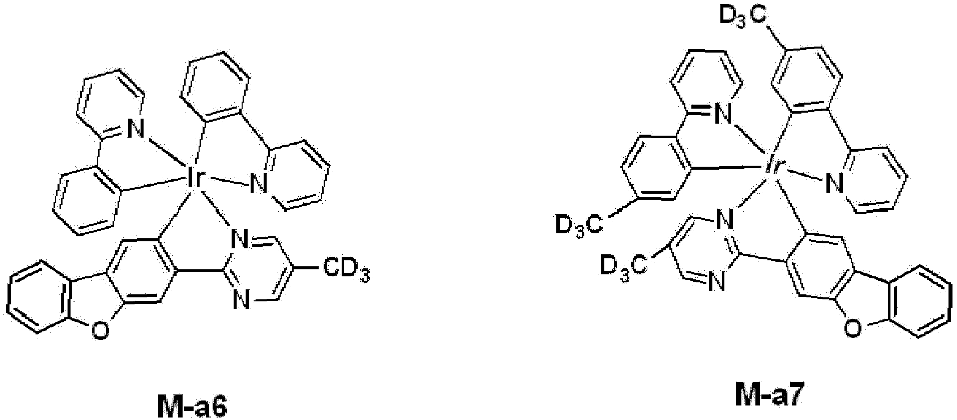

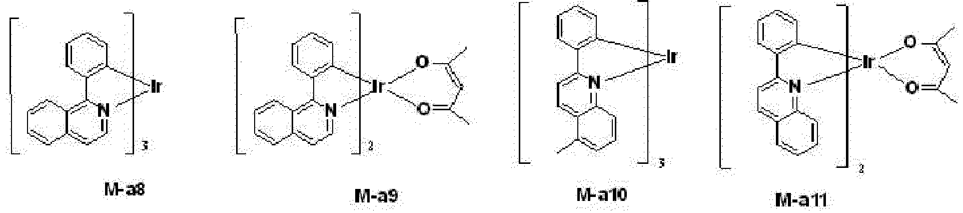

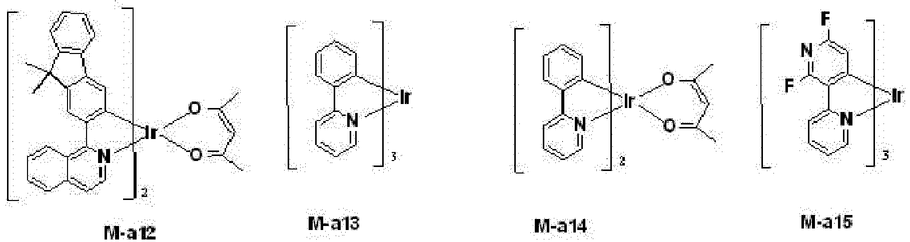

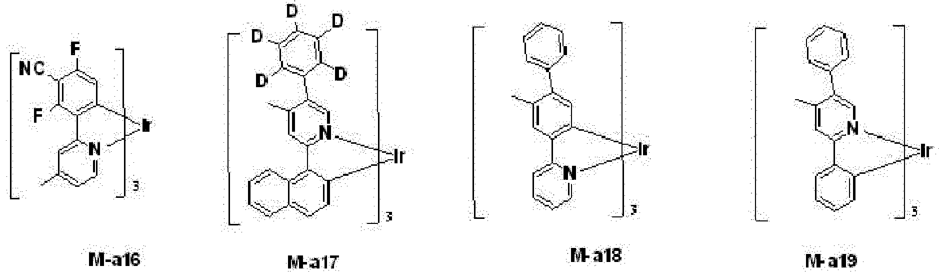

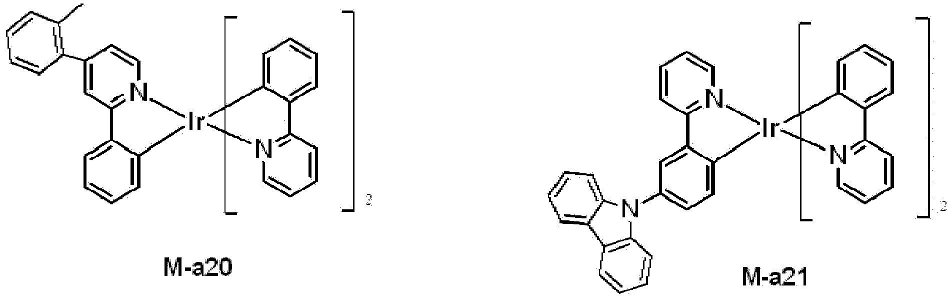

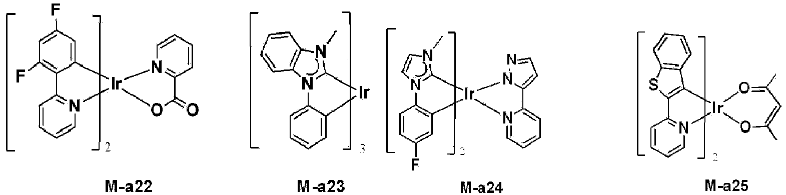

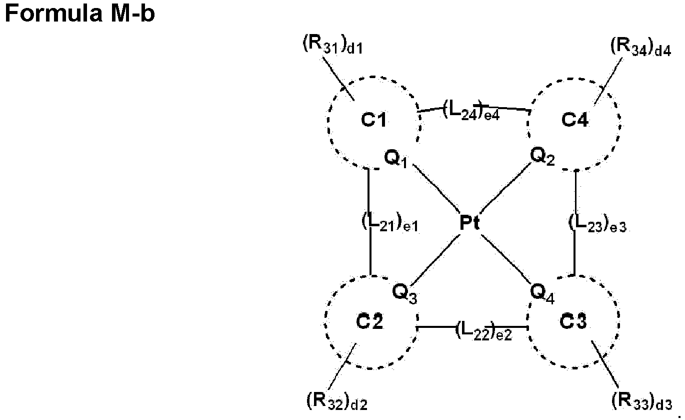

In Formula M-b, O₁ to Q₄ may each independently be C or N, and C1 to C4 may each independently be a substituted or unsubstituted hydrocarbon ring having 5 to 30 ring-forming carbon atoms, or a substituted or unsubstituted heterocycle having 2 to 30 ring-forming carbon atoms. L₂₁ to L₂₄ may each independently be a direct linkage, 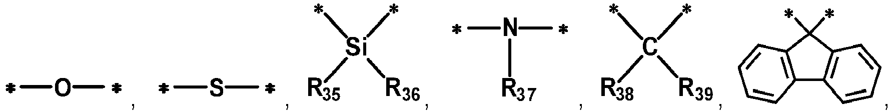 a substituted or unsubstituted divalent alkyl group having 1 to 20 carbon atoms, a substituted or unsubstituted arylene group having 6 to 30 ring-forming carbon atoms, or a substituted or unsubstituted heteroarylene group having 2 to 30 ring-forming carbon atoms, and e1 to e4 may each independently be 0 or 1. R₃₁ to R₃₉ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or may be bonded to an adjacent group to form a ring, and d1 to d4 may each independently be an integer of 0 to 4.

The compound represented by Formula M-b may be utilized as a blue phosphorescent dopant or a green phosphorescent dopant.

The compound represented by Formula M-b may be represented by any one selected from among the compounds. However, the compounds are examples, and the compound represented by Formula M-b is not limited to those represented by the compounds.

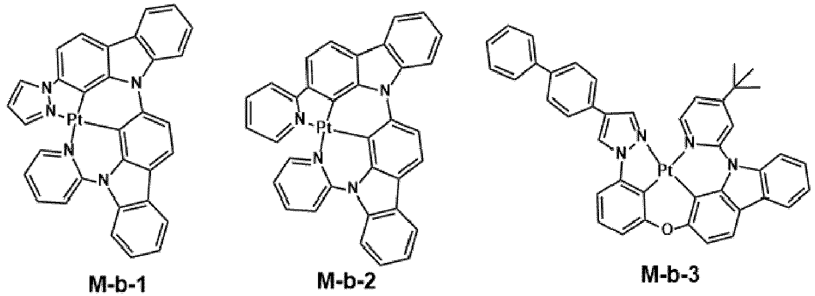

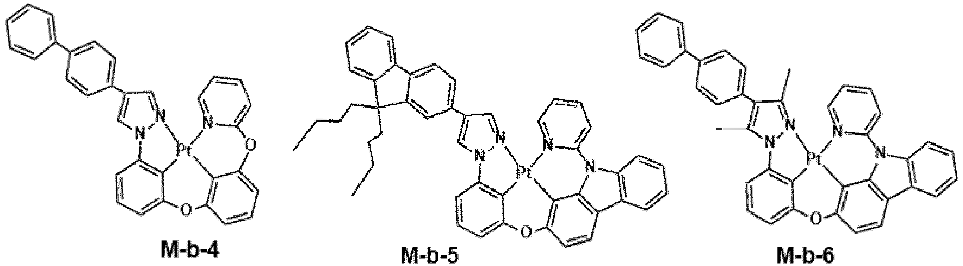

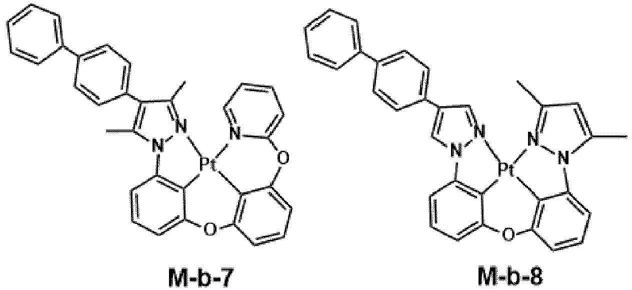

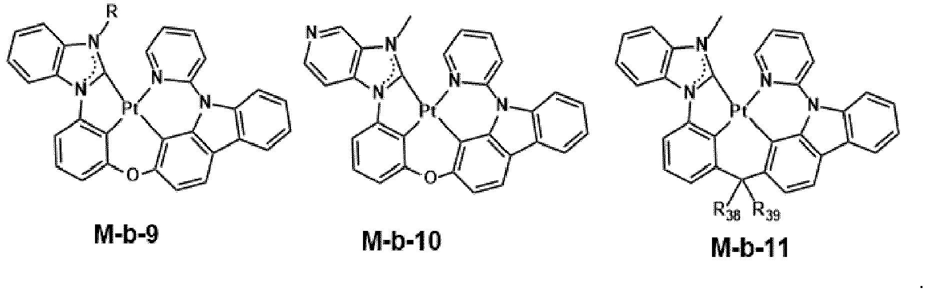

In the compounds above, R, R₃₈, and R₃₉ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In some embodiments, the emission layer EML may include a plurality of phosphorescent dopant materials, and each of the plurality of phosphorescent dopant materials may include the same core metal atom or different core metal atom. In one or more embodiments, the emission layer EML may include two phosphorescent dopant materials, and one phosphorescent dopant material may be represented by Formula M-a, and the other phosphorescent dopant material may be represented by Formula M-b. In some embodiments, the emission layer EML may include two phosphorescent dopant materials, and both the phosphorescent dopant materials may be represented by Formula M-a. In some embodiments, the emission layer EML may include two phosphorescent dopant materials, and both the phosphorescent dopant materials may be represented by Formula M-b.

The emission layer EML may include a compound represented by any one selected from among Formula F-a to Formula F-c. The compound represented by Formula F-a or Formula F-c may be utilized as a fluorescence dopant material.

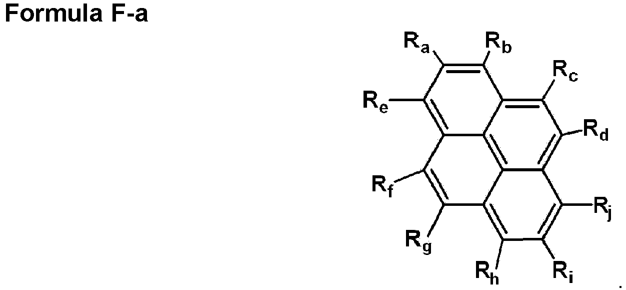

In Formula F-a, two selected from among Rₐ to Rⱼ may each independently be substituted with ^{∗-}NAr₁Ar₂. The others, which are not substituted with ^{∗-}NAr₁Ar₂, among Rₐ to Rⱼ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. In ^{∗-}NAr₁Ar₂, Ar₁ and Ar₂ may each independently be a substituted or unsubstituted aryl group having 6 to 30 ring-forming carbon atoms, or a substituted or unsubstituted heteroaryl group having 2 to 30 ring-forming carbon atoms. For example, at least one of Ar₁ or Ar₂ may be a heteroaryl group containing O or S as a ring-forming atom.

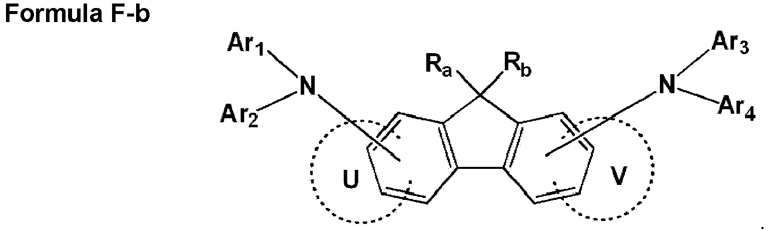

In Formula F-b, Rₐ and R_{b} may each independently be a hydrogen atom, a deuterium atom, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and/or may be bonded to an adjacent group to form a ring. Ar₁ to Ar₄ may each independently be a substituted or unsubstituted aryl group having 6 to 30 ring-forming carbon atoms, or a substituted or unsubstituted heteroaryl group having 2 to 30 ring-forming carbon atoms.

In Formula F-b, U and V may each independently be a substituted or unsubstituted hydrocarbon ring having 5 to 30 ring-forming carbon atoms, or a substituted or unsubstituted heterocycle having 2 to 30 ring-forming carbon atoms.

In Formula F-b, the number of rings represented by U and V may each independently be 0 or 1. For example, in Formula F-b, it means that when the number of U or V is 1, one ring constitutes a fused ring at a portion indicated by U or V, and when the number of U or V is 0, a ring indicated by U or V is not provided. For example, when the number of U is 0 and the number of V is 1, or when the number of U is 1 and the number of V is 0, the fused ring having a fluorene core in Formula F-b may be a cyclic compound having four rings. In some embodiments, when each number of U and V is 0, the fused ring in Formula F-b may be a cyclic compound having three rings. In some embodiments, when each number of U and V is 1, the fused ring having a fluorene core in Formula F-b may be a cyclic compound having five rings.

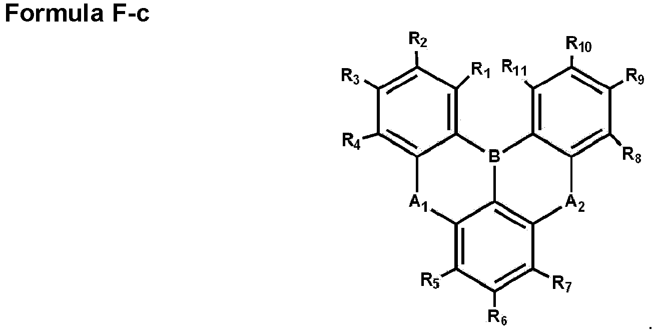

In Formula F-c, A₁ and A₂ may each independently be O, S, Se, or NRₘ, and Rₘ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. R₁ to R₁₁ may each independently be a hydrogen atom, a deuterium atom, a halogen atom, a cyano group, a substituted or unsubstituted amine group, a substituted or unsubstituted boryl group, a substituted or unsubstituted oxy group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or may be bonded to an adjacent group to form a ring.

In Formula F-c, A₁ and A₂ may each independently be bonded to substituents of an adjacent ring to form a condensed ring. For example, when A₁ and A₂ are each independently NRₘ, A₁ may be bonded to R₄ or R₅ to form a ring. In some embodiments, A₂ may be bonded to R₇ or R₈ to form a ring.

In one or more embodiments, the emission layer EML may include, as a suitable dopant material, a styryl derivative (e.g., 1,4-bis[2-(3-N-ethylcarbazoryl)vinyl]benzene (BCzVB), 4-(di-p-tolylamino)-4'-[(di-p-tolylamino)styryl]stilbene (DPAVB), and/or N-(4-((E)-2-(6-((E)-4-(diphenylamino)styryl)naphthalen-2-yl)vinyl)phenyl)-N-phenylbenz enamine (N-BDAVBi)), 4,4'-bis[2-(4-(N,N-diphenylamino)phenyl)vinyl]biphenyl (DPAVBi), perylene and/or a derivative thereof (e.g., 2,5,8,11-tetra-t-butylperylene (TBP)), pyrene and/or a derivative thereof (e.g., 1,1-dipyrene, 1,4-dipyrenylbenzene, 1,4-bis(N,N-dipheny/lamino)pyrene), *etc.*

The emission layer EML may include a suitable phosphorescent dopant material. For example, a metal complex containing iridium (Ir), platinum (Pt), osmium (Os), aurum (Au), titanium (Ti), zirconium (Zr), hafnium (Hf), europium (Eu), terbium (Tb), and/or thulium (Tm) may be utilized as a phosphorescent dopant. For example, iridium(III) bis(4,6-difluorophenylpyridinato-N,C2') (Flrpic), bis(2,4-difluorophenylpyridinato)-tetrakis(1-pyrazolyl)borate iridium(III) (Fir6), and/or platinum octaethyl porphyrin (PtOEP) may be utilized as a phosphorescent dopant. However, the embodiment of the present disclosure is not limited thereto.

The emission layer EML may include a quantum dot material. The core of the quantum dot may be selected from a Group II-VI compound, a Group III-VI compound, a Group I-III-IV compound, a Group III-V compound, a Group III-II-V compound, a Group IV-VI compound, a Group IV element, a Group IV compound, or a combination thereof.

The Group II-VI compound may be selected from the group consisting of a binary compound selected from the group consisting of CdSe, CdTe, CdS, ZnS, ZnSe, ZnTe, ZnO, HgS, HgSe, HgTe, MgSe, MgS, and a mixture thereof; a ternary compound selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and a mixture thereof; and a quaternary compound selected from the group consisting of HgZnTeS, CdZnSeS, CdZnSeTe, CdZnSTe, CdHgSeS, CdHgSeTe, CdHgSTe, HgZnSeS, HgZnSeTe, HgZnSTe, and a mixture thereof.

The Group III-VI compound may include a binary compound such as In₂S₃ and/or In₂Se₃, a ternary compound such as InGaS₃ and/or InGaSe₃, or any combination thereof.

The Group I-III-VI compound may be selected from a ternary compound selected from the group consisting of AgInS, AgInS₂, CuInS, CuInS₂, AgGaS₂, CuGaS₂ CuGaO₂, AgGaO₂, AgAlO_{2,} and a mixture thereof; or a quaternary compound such as AgInGaS₂ and/or CuInGaS₂.

The Group III-V compound may be selected from the group consisting of a binary compound selected from the group consisting of GaN, GaP, GaAs, GaSb, AIN, AIP, AlAs, AlSb, InN, InP, InAs, InSb, and a mixture thereof; a ternary compound selected from the group consisting of GaNP, GaNAs, GaNSb, GaPAs, GaPSb, AINP, AINAs, AINSb, AlPAs, AlPSb, InGaP, InAlP, InNP, InNAs, InNSb, InPAs, InPSb, and a mixture thereof; and a quaternary compound selected from the group consisting of GaAINP, GaAINAs, GaAINSb, GaAlPAs, GaAlPSb, GaInNP, GaInNAs, GaInNSb, GaInPAs, GaInPSb, InAINP, InAINAs, InAINSb, InAIPAs, InAIPSb, and a mixture thereof. In some embodiments, the Group III-V compound may further include a Group II metal. For example, InZnP, *etc.* may be selected as a Group III-II-V compound.

The Group IV-VI compound may be selected from the group consisting of a binary compound selected from the group consisting of SnS, SnSe, SnTe, PbS, PbSe, PbTe, and a mixture thereof; a ternary compound selected from the group consisting of SnSeS, SnSeTe, SnSTe, PbSeS, PbSeTe, PbSTe, SnPbS, SnPbSe, SnPbTe, and a mixture thereof; and a quaternary compound selected from the group consisting of SnPbSSe, SnPbSeTe, SnPbSTe, and a mixture thereof. The Group IV element may be selected from the group consisting of Si, Ge, and a mixture thereof. The Group IV compound may be a binary compound selected from the group consisting of SiC, SiGe, and a mixture thereof.

In this case, a binary compound, a ternary compound, and/or a quaternary compound may be present in a particle with a substantially uniform concentration distribution, or may be present in the same particle with a partially different concentration distribution. In some embodiments, a core/shell structure in which one quantum dot surrounds another quantum dot may also be possible. The core/shell structure may have a concentration gradient in which the concentration of elements present in the shell decreases toward the core.

In some embodiments, the quantum dot may have the above-described core/shell structure including a core containing nanocrystals and a shell around (e.g., surrounding) the core. The shell of the quantum dot may serve as a protection layer to prevent or reduce the chemical deformation of the core so as to maintain semiconductor properties, and/or a charging layer to impart electrophoresis properties to the quantum dot. The shell may be a single layer or a multilayer. An example of the shell of the quantum dot may include a metal oxide, a non-metal oxide, a semiconductor compound, or a combination thereof.

For example, the metal oxide or the non-metal oxide may each independently be a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄, and/or NiO; or a ternary compound such as MgAl₂O₄, CoFe₂O₄, NiFe₂O₄, and/or CoMn₂O₄, but the embodiment of the present disclosure is not limited thereto.

In one or more embodiments, the semiconductor compound may be, for example, CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AIP, AlSb, *etc*., but the embodiment of the present disclosure is not limited thereto.

The quantum dot may have a full width of half maximum (FWHM) of a light emission wavelength spectrum of about 45 nm or less, for example, about 40 nm or less, and for example, about 30 nm or less, and color purity and/or color reproducibility may be improved in any of the above ranges. In some embodiments, light emitted through such a quantum dot is emitted in all directions, and thus a wide viewing angle may be improved.

The form of the quantum dot is not particularly limited as long as it is a suitable form that may be utilized in the art, and for example, the quantum dot in the form of spherical, pyramidal, multi-arm, and/or cubic nanoparticles, nanotubes, nanowires, nanofibers, nanoplate particles, *etc.* may be utilized.

The quantum dot may control the color of emitted light according to the particle size thereof, and accordingly, the quantum dot may have one or more suitable emission colors such as blue, red, and/or green.

In each light emitting device ED of embodiments illustrated in FIGS. 3 to 7, the electron transport region ETR is provided on the emission layer EML. The electron transport region ETR may include at least one of the hole blocking layer HBL, the electron transport layer ETL, or the electron injection layer EIL, but the embodiment of the present disclosure is not limited thereto.

The electron transport region ETR may have a single layer formed of a single material, a single layer formed of a plurality of different materials, or a multilayer structure including a plurality of layers formed of a plurality of different materials.

For example, the electron transport region ETR may have a single layer structure of the electron injection layer EIL or the electron transport layer ETL, and may have a single layer structure formed of an electron injection material and an electron transport material. In some embodiments, the electron transport region ETR may have a single layer structure formed of a plurality of different materials, or may have a structure in which an electron transport layer ETL/electron injection layer EIL, a hole blocking layer HBL/electron transport layer ETL/electron injection layer EIL are stacked in order from the emission layer EML, but the embodiment of the present disclosure is not limited thereto. The electron transport region ETR may have a thickness, for example, from about 100 nm (1,000 Å) to about 150 nm (1,500 Å).

The electron transport region ETR may be formed by utilizing one or more suitable methods such as a vacuum deposition method, a spin coating method, a cast method, a Langmuir-Blodgett (LB) method, an inkjet printing method, a laser printing method, and/or a laser induced thermal imaging (LITI) method.

The electron transport region ETR may include a compound represented by Formula ET-1:

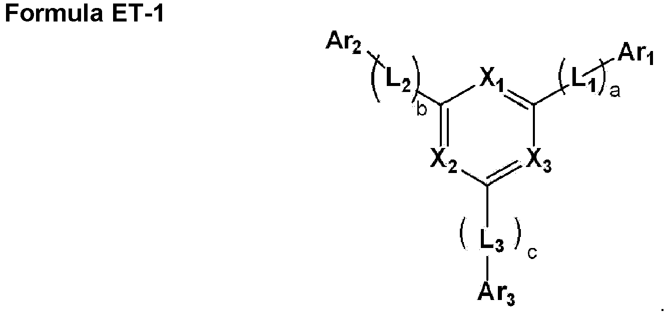

In Formula ET-1, at least one selected from among X₁ to X₃ is N, and the rest are CRₐ. Rₐ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. Ar₁ to Ar₃ may each independently be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula ET-1, a to c may each independently be an integer of 0 to 10. In Formula ET-1, L₁ to L₃ may each independently be a direct linkage, a substituted or unsubstituted arylene group having 6 to 30 ring-forming carbon atoms, or a substituted or unsubstituted heteroarylene group having 2 to 30 ring-forming carbon atoms. In some embodiments, when a to c are an integer of 2 or more, L₁ to L₃ may each independently be a substituted or unsubstituted arylene group having 6 to 30 ring-forming carbon atoms, or a substituted or unsubstituted heteroarylene group having 2 to 30 ring-forming carbon atoms.

The electron transport region ETR may include an anthracene-based compound. However, the embodiment of the present disclosure is not limited thereto, and the electron transport region ETR may include, for example, tris(8-hydroxyquinolinato)aluminum (Alq₃), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazol-1-yl)phenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,08)-(1,1'-biphenyl-4-olato)aluminum (BAlq), berylliumbis(benzoquinolin-10-olate (Bebq₂), 9,10-di(naphthalene-2-yl)anthracene (ADN), 1,3-Bis[3,5-di(pyridin-3-yl)phenyl]benzene (BmPyPhB), or a mixture thereof.

The electron transport region ETR may include at least one selected from among Compound ET1 to Compound ET36:

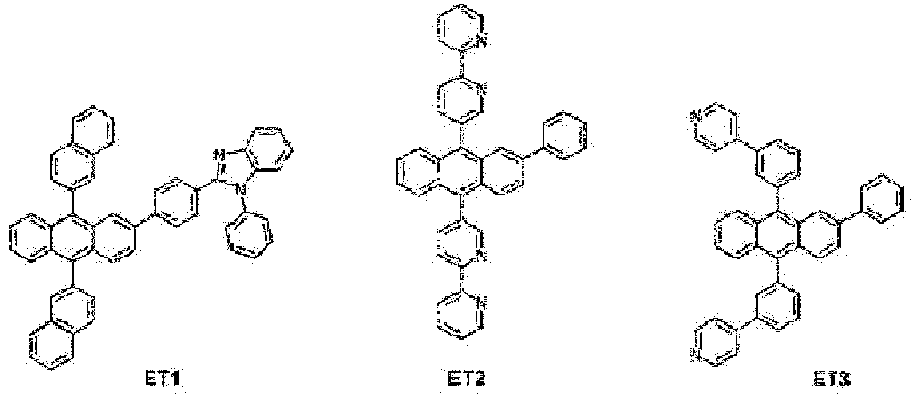

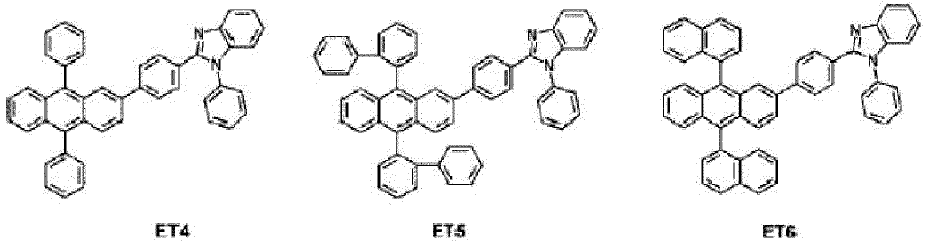

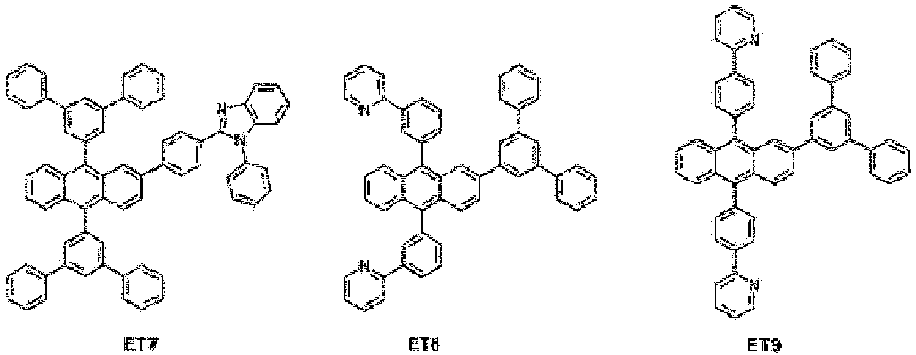

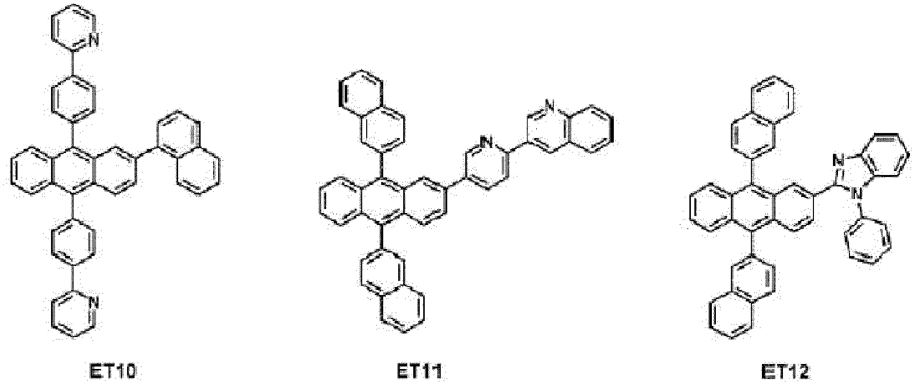

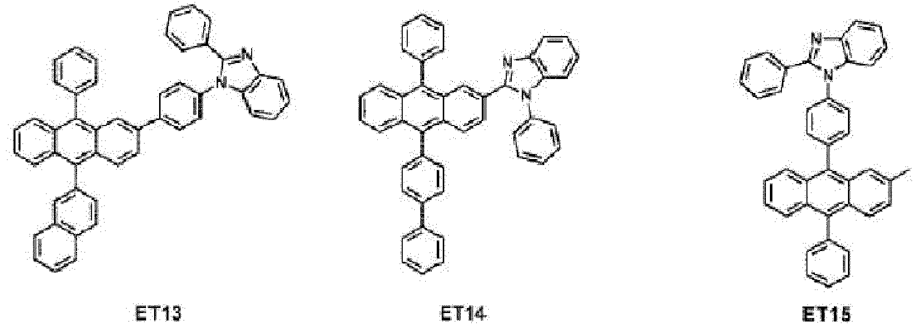

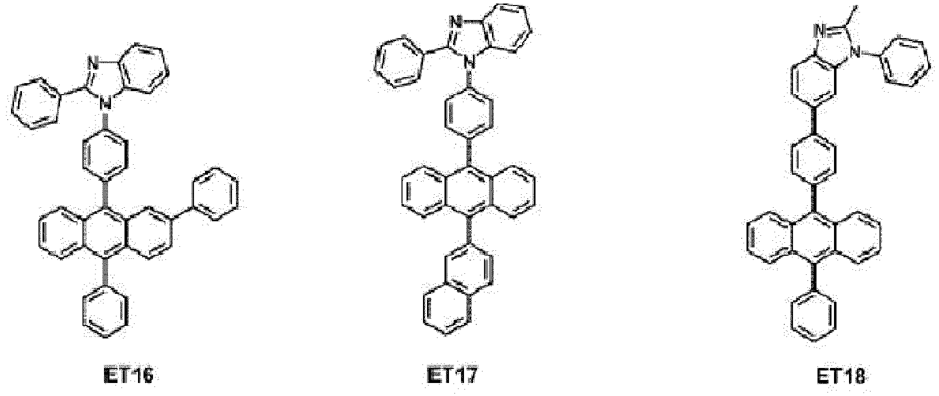

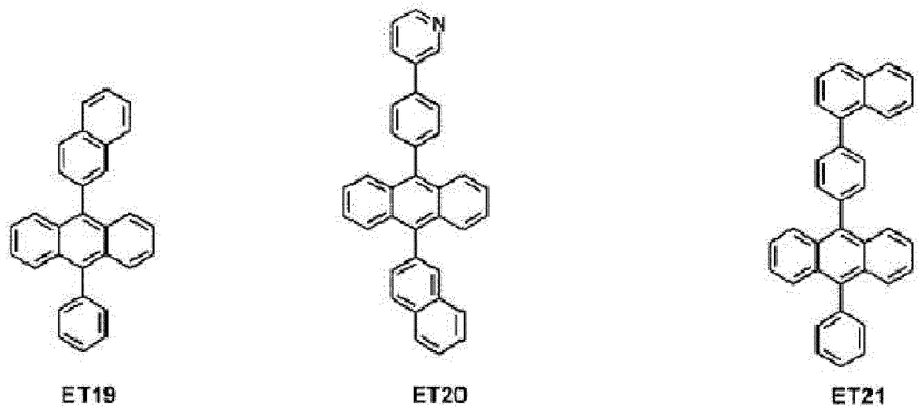

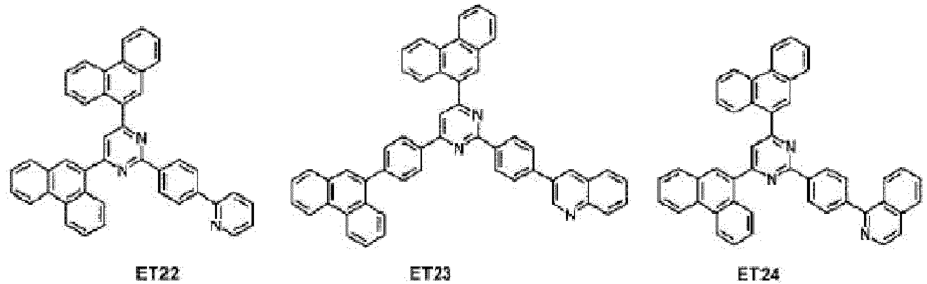

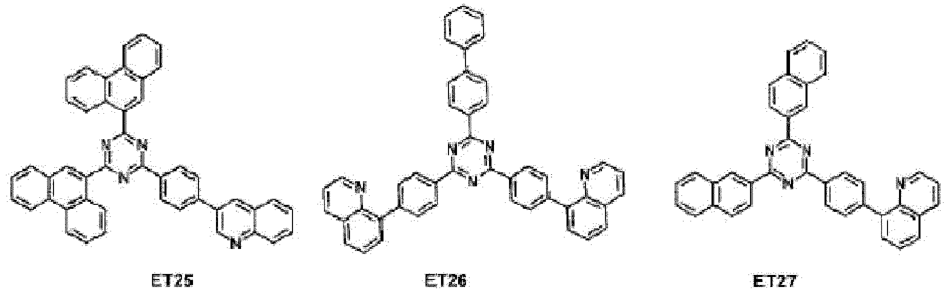

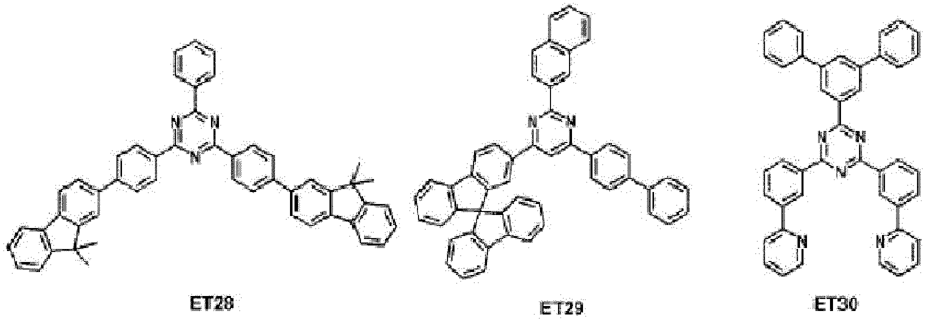

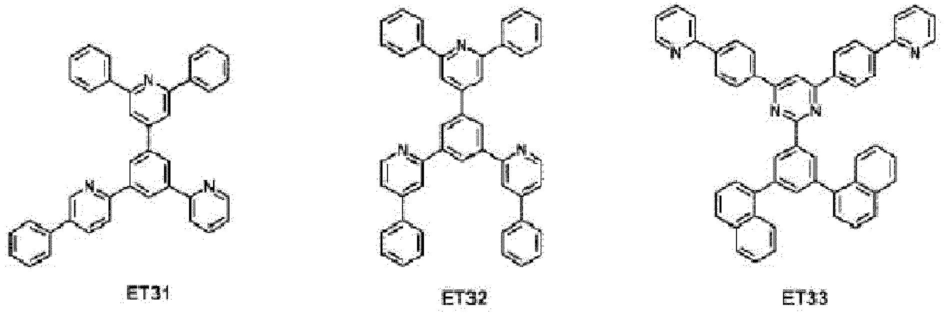

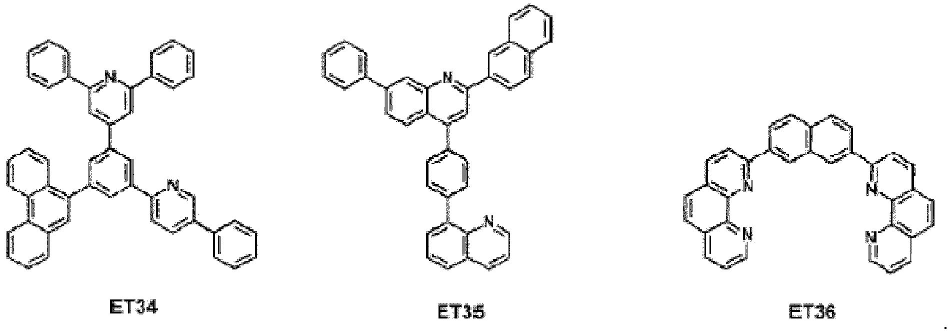

In some embodiments, the electron transport regions ETR may include a metal halide such as LiF, NaCl, CsF, RbCI, RbI, Cul, and/or KI, a lanthanide metal such as Yb, and/or a co-deposited material of the metal halide and the lanthanide metal. For example, the electron transport region ETR may include KI:Yb, RbI:Yb, LiF:Yb, *etc.* as a co-deposited material. In some embodiments, the electron transport region ETR may be formed utilizing a metal oxide such as Li₂O and/or BaO, and/or 8-hydroxyl-lithium quinolate (Liq), *etc*., but the embodiment of the present disclosure is not limited thereto. The electron transport region ETR may also be formed of a mixture material of an electron transport material and an insulating organometallic salt. The organometallic salt may be a material having an energy band gap of about 4 eV or more. For example, the organometallic salt may include, for example, a metal acetate, a metal benzoate, a metal acetoacetate, a metal acetylacetonate, and/or a metal stearate.

The electron transport region ETR may further include at least one of 2,9-dimethyl-4,7-diphenyl-1,10-phenanthroline (BCP), diphenyl(4-(triphenylsilyl)phenyl)phosphine oxide (TSP01), and/or 4,7-diphenyl-1,10-phenanthroline (Bphen), in addition to the above-described materials, but the embodiment of the present disclosure is not limited thereto.

The electron transport region ETR may include the above-described compounds of the hole transport region in at least one of the electron injection layer EIL, the electron transport layer ETL, or the hole blocking layer HBL.

When the electron transport region ETR includes the electron transport layer ETL, the electron transport layer ETL may have a thickness of about 10 nm (100 Å) to about 100 nm (1,000 Å), for example, about 15 nm (150 Å) to about 50 nm (500 Å). When the thickness of the electron transport layer ETL satisfies any of the aforementioned ranges, satisfactory or suitable electron transport characteristics may be obtained without a substantial increase in a driving voltage. When the electron transport region ETR includes the electron injection layer EIL, the electron injection layer EIL may have a thickness of about 0.1 nm (1 Å) to about 10 nm (100 Å), for example, about 0.3 nm (3 Å) to about 9 nm (90 Å). When the thickness of the electron injection layer EIL satisfies any of the above-described ranges, satisfactory or suitable electron injection characteristics may be obtained without a substantial increase in driving voltage.

The second electrode EL2 is provided on the electron transport region ETR. The second electrode EL2 may be a common electrode. The second electrode EL2 may be a cathode or an anode, but the embodiment of the present disclosure is not limited thereto. For example, when the first electrode EL1 is an anode, the second electrode EL2 may be a cathode, and when the first electrode EL1 is a cathode, the second electrode EL2 may be an anode. The second electrode may include at least one selected from among Ag, Mg, Cu, Al, Pt, Pd, Au, Ni, Nd, Ir, Cr, Li, Ca, LiF, Mo, Ti, W, In, Sn, and/or Zn, a compound of two or more thereof, a mixture of two or more thereof, and/or an oxide thereof.

The second electrode EL2 may be a transmissive electrode, a transflective electrode, or a reflective electrode. When the second electrode EL2 is the transmissive electrode, the second electrode EL2 may be formed of a transparent metal oxide, for example, indium tin oxide (ITO), indium zinc oxide (IZO), zinc oxide (ZnO), indium tin zinc oxide (ITZO), *etc.*

When the second electrode EL2 is the transflective electrode or the reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, or a compound or mixture thereof (e.g., AgMg, AgYb, and/or MgAg). In some embodiments, the second electrode EL2 may have a multilayer structure including a reflective film or a transflective film formed of any of the above-described materials, and a transparent conductive film formed of ITO, IZO, ZnO, ITZO, *etc.* For example, the second electrode EL2 may include the above-described metal materials, combinations of at least two metal materials of the above-described metal materials, oxides of the above-described metal materials, and/or the like.

In some embodiments, the second electrode EL2 may be connected with an auxiliary electrode. When the second electrode EL2 is connected with the auxiliary electrode, the resistance of the second electrode EL2 may be decreased.

In some embodiments, a capping layer CPL may further be disposed on the second electrode EL2 of the light emitting device ED of one or more embodiments. The capping layer CPL may include a multilayer or a single layer.

In one or more embodiments, the capping layer CPL may be an organic layer or an inorganic layer. For example, when the capping layer CPL contains an inorganic material, the inorganic material may include an alkaline metal compound (for example, LiF), an alkaline earth metal compound (for example, MgF₂), SiON, SiNₓ, SiOy, *etc.*

For example, when the capping layer CPL contains an organic material, the organic material may include α-NPD, NPB, TPD, m-MTDATA, Alq₃, CuPc, N4,N4,N4',N4'-tetra(biphenyl-4-yl)biphenyl-4,4'-diamine (TPD15), 4,4',4"-tris(carbazol sol-9-yl)triphenylamine (TCTA), *etc*., or may include an epoxy resin, or an acrylate such as a methacrylate. However, the embodiment of the present disclosure is not limited thereto, and the capping layer CPL may include at least one selected from among Compounds P1 to P5:

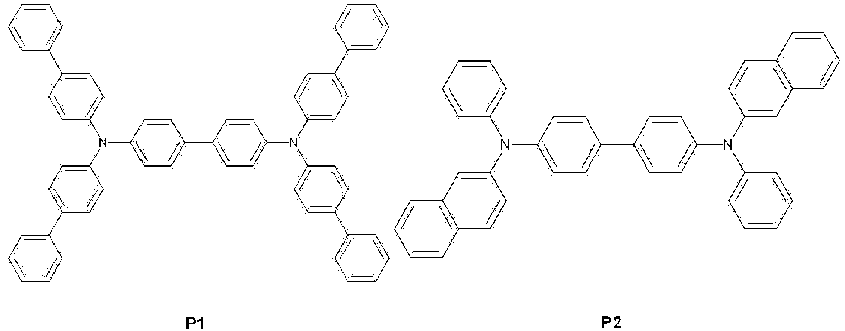

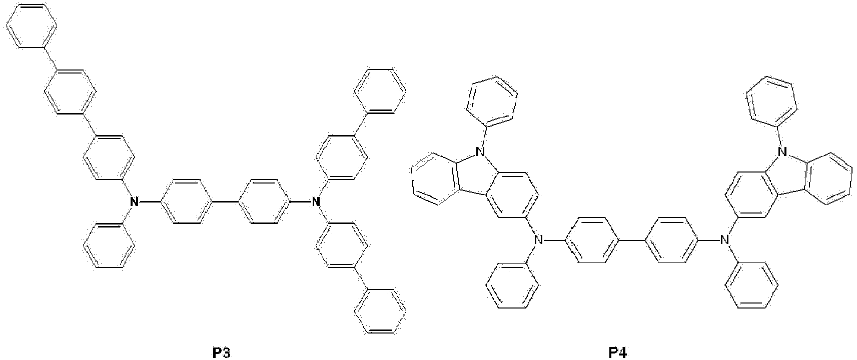

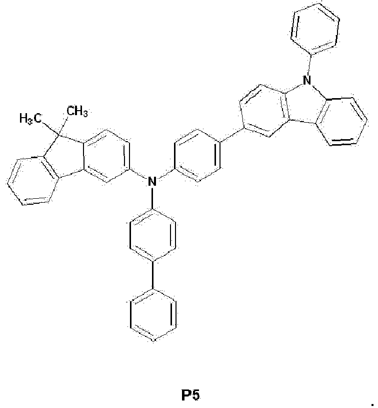

In some embodiments, the refractive index of the capping layer CPL may be about 1.6 or more. For example, the refractive index of the capping layer CPL may be about 1.6 or more with respect to light in a wavelength range of about 550 nm to about 660 nm.

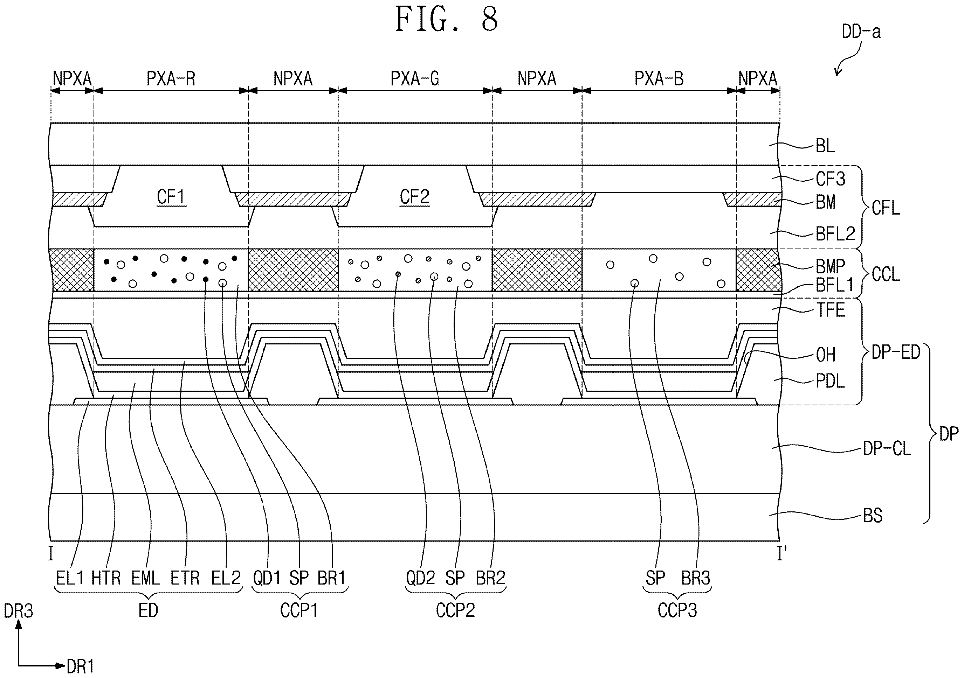

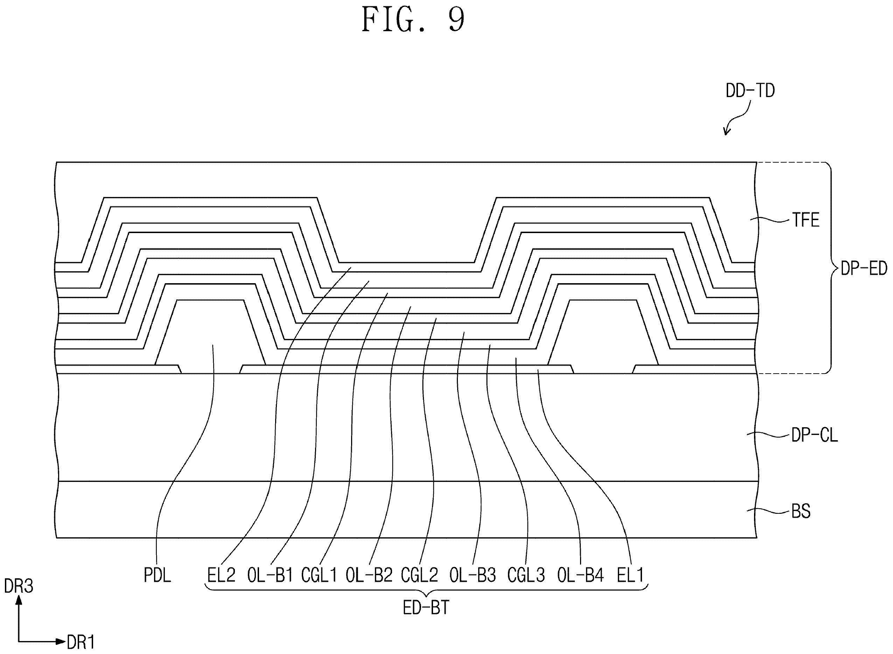

Each of FIGS. 8 and 9 is a cross-sectional view of a display apparatus according to one or more embodiments of the present disclosure. Hereinafter, in describing the display apparatuses of embodiments with reference to FIGS. 8 and 9, the duplicated features which have been described in FIGS. 1 to 7 are not described again, but their differences will be mainly described.

Referring to FIG. 8, the display apparatus DD according to one or more embodiments may include a display panel DP including a display device layer DP-ED, a light control layer CCL disposed on the display panel DP, and a color filter layer CFL.

In one or more embodiments illustrated in FIG. 8, the display panel DP may include a base layer BS, a circuit layer DP-CL provided on the base layer BS, and the display device layer DP-ED, and the display device layer DP-ED may include a light emitting device ED.

The light emitting device ED may include a first electrode EL1, a hole transport region HTR disposed on the first electrode EL1, an emission layer EML disposed on the hole transport region HTR, an electron transport region ETR disposed on the emission layer EML, and a second electrode EL2 disposed on the electron transport region ETR. In some embodiments, the structures of the light emitting devices of FIGS. 3 to 7 as described above may be equally applied to the structure of the light emitting device ED illustrated in FIG. 8.

Referring to FIG. 8, the emission layer EML may be disposed in an opening OH defined in a pixel defining film PDL. For example, the emission layer EML which is divided by the pixel defining film PDL and provided corresponding to each light emitting regions PXA-R, PXA-G, and PXA-B may be to emit light in substantially the same wavelength range. In the display apparatus DD of one or more embodiments, the emission layer EML may be to emit blue light. In some embodiments, the emission layer EML may be provided as a common layer in the entire light emitting regions PXA-R, PXA-G, and PXA-B.

The light control layer CCL may be disposed on the display panel DP. The light control layer CCL may include a light conversion body. The light conversion body may be a quantum dot, a phosphor, and/or the like. The light conversion body may be to emit provided (e.g., incident) light by converting the wavelength thereof. For example, the light control layer CCL may be a layer containing the quantum dot and/or a layer containing the phosphor.

The light control layer CCL may include a plurality of light control parts CCP1, CCP2 and CCP3. The light control parts CCP1, CCP2, and CCP3 may be spaced apart from each other.

Referring to FIG. 8, divided patterns BMP may be disposed between the light control parts CCP1, CCP2 and CCP3 which are spaced apart from each other, but the embodiment of the present disclosure is not limited thereto. FIG. 8 illustrates that the divided patterns BMP do not overlap the light control parts CCP1, CCP2 and CCP3, but at least a portion of the edges of the light control parts CCP1, CCP2 and CCP3 may overlap the divided patterns BMP.

The light control layer CCL may include a first light control part CCP1 containing a first quantum dot QD1 which converts (e.g., is configured to convert) first color light provided from the light emitting device ED into second color light, a second light control part CCP2 containing a second quantum dot QD2 which converts (e.g., is configured to convert) the first color light into third color light, and a third light control part CCP3 which transmits (e.g., is configured to transmit) the first color light.

In one or more embodiments, the first light control part CCP1 may provide red light that is the second color light, and the second light control part CCP2 may provide green light that is the third color light. The third light control part CCP3 may provide blue light by transmitting the blue light that is the first color light provided from the light emitting device ED. For example, the first quantum dot QD1 may be a red quantum dot, and the second quantum dot QD2 may be a green quantum dot. The same descriptions as those provided above may be applied with respect to the quantum dots QD1 and QD2.

In some embodiments, the light control layer CCL may further include a scatterer SP. The first light control part CCP1 may include the first quantum dot QD1 and the scatterer SP, the second light control part CCP2 may include the second quantum dot QD2 and the scatterer SP, and the third light control part CCP3 may not include (e.g., may exclude) any quantum dot but may include the scatterer SP.

The scatterer SP may be inorganic particles. For example, the scatterer SP may include at least one of TiO₂, ZnO, Al₂O₃, SiO₂, or hollow sphere silica. The scatterer SP may include any one selected from among TiO₂, ZnO, Al₂O₃, SiO₂, and hollow sphere silica, or may be a mixture of at least two materials selected from among TiO₂, ZnO, Al₂O₃, SiO₂, and hollow sphere silica.

The first light control part CCP1, the second light control part CCP2, and the third light control part CCP3 may respectively include base resins BR1, BR2, and BR3 in which the quantum dots QD1 and QD2 and the scatterer SP are dispersed. In one or more embodiments, the first light control part CCP1 may include the first quantum dot QD1 and the scatterer SP dispersed in a first base resin BR1, the second light control part CCP2 may include the second quantum dot QD2 and the scatterer SP dispersed in a second base resin BR2, and the third light control part CCP3 may include the scatterer SP dispersed in a third base resin BR3. The base resins BR1, BR2, and BR3 are media in which the quantum dots QD1 and QD2 and the scatterer SP are dispersed, and may be formed of one or more suitable resin compositions, which may be generally referred to as a binder. For example, the base resins BR1, BR2, and BR3 may be acrylic-based resins, urethane-based resins, silicone-based resins, epoxy-based resins, *etc.* The base resins BR1, BR2, and BR3 may be transparent resins. In one or more embodiments, the first base resin BR1, the second base resin BR2, and the third base resin BR3 may be the same as or different from each other.

The light control layer CCL may include a barrier layer BFL1. The barrier layer BFL1 may serve to prevent or reduce the penetration of moisture and/or oxygen (hereinafter, referred to as 'moisture/oxygen'). The barrier layer BFL1 may be disposed on the light control parts CCP1, CCP2, and CCP3 to block or reduce the light control parts CCP1, CCP2 and CCP3 from being exposed to moisture/oxygen. In some embodiments, the barrier layer BFL1 may cover the light control parts CCP1, CCP2, and CCP3. In some embodiments, the barrier layer BFL2 may be provided between the color filter layer CFL and the light control parts CCP1, CCP2, and CCP3.

The barrier layers BFL1 and BFL2 may include at least one inorganic layer. For example, the barrier layers BFL1 and BFL2 may include an inorganic material. For example, the barrier layers BFL1 and BFL2 may include a silicon nitride, an aluminum nitride, a zirconium nitride, a titanium nitride, a hafnium nitride, a tantalum nitride, a silicon oxide, an aluminum oxide, a titanium oxide, a tin oxide, a cerium oxide, a silicon oxynitride, a metal thin film which secures (e.g., facilitates, allows) a transmittance, *etc.* In some embodiments, the barrier layers BFL1 and BFL2 may further include an organic film. The barrier layers BFL1 and BFL2 may be formed of a single layer or a plurality of layers.

In the display apparatus DD of one or more embodiments, the color filter layer CFL may be disposed on the light control layer CCL. For example, the color filter layer CFL may be directly disposed on the light control layer CCL. In this case, the barrier layer BFL2 may not be provided.

The color filter layer CFL may include a light shielding part BM and color filters CF1, CF2, and CF3. The color filter layer CFL may include a first filter CF1 configured to transmit the second color light, a second filter CF2 configured to transmit the third color light, and a third filter CF3 configured to transmit the first color light. For example, the first filter CF1 may be a red filter, the second filter CF2 may be a green filter, and the third filter CF3 may be a blue filter. The filters CF1, CF2, and CF3 each may include a polymeric photosensitive resin and a pigment or dye. The first filter CF1 may include a red pigment or dye, the second filter CF2 may include a green pigment or dye, and the third filter CF3 may include a blue pigment or dye. However, the embodiment of the present disclosure is not limited thereto, and the third filter CF3 may not include (e.g., may exclude) a pigment or dye. The third filter CF3 may include a polymeric photosensitive resin and may not include (e.g., may exclude) a pigment or dye. The third filter CF3 may be transparent. The third filter CF3 may be formed of a transparent photosensitive resin.

Furthermore, in one or more embodiments, the first filter CF1 and the second filter CF2 may be a yellow filter. The first filter CF1 and the second filter CF2 may not be separated but may be provided as one filter.

The light shielding part BM may be a black matrix. The light shielding part BM may include an organic light shielding material or an inorganic light shielding material containing a black pigment or dye. The light shielding part BM may prevent or reduce light leakage, and may separate boundaries between the adjacent filters CF1, CF2, and CF3. In one or more embodiments, the light shielding part BM may be formed of a blue filter.

The first to third filters CF1, CF2, and CF3 may be disposed corresponding to the red light emitting region PXA-R, the green light emitting region PXA-G, and the blue light emitting region PXA-B, respectively.

A base substrate BL may be disposed on the color filter layer CFL. The base substrate BL may be a member which provides a base surface in which the color filter layer CFL, the light control layer CCL, and/or the like are disposed. The base substrate BL may be a glass substrate, a metal substrate, a plastic substrate, *etc.* However, the embodiment of the present disclosure is not limited thereto, and the base substrate BL may be an inorganic layer, an organic layer, or a composite material layer. In some embodiments, the base substrate BL may not be provided.

FIG. 9 is a cross-sectional view illustrating a portion of a display apparatus according to one or more embodiments. In the display apparatus DD-TD of one or more embodiments, the light emitting device ED-BT may include a plurality of light emitting structures OL-B1, OL-B2, OL-B3, and OL-B4. The light emitting device ED-BT may include a first electrode EL1 and a second electrode EL2 which face each other, and the plurality of light emitting structures OL-B1, OL-B1, OL-B2, and OL-B3 sequentially stacked in the thickness direction between the first electrode EL1 and the second electrode EL2. The light emitting structures OL-B1, OL-B2, OL-B3, and OL-B4 each may include an emission layer EML (see FIG. 8) and a hole transport region HTR and an electron transport region ETR disposed with the emission layer EML (see FIG. 8) located therebetween.

For example, the light emitting device ED-BT included in the display apparatus DD-TD of one or more embodiments may be a light emitting device having a tandem structure and including a plurality of emission layers.

In one or more embodiments illustrated in FIG. 9, all light beams respectively emitted from the light emitting structures OL-B1, OL-B2, OL-B3, and OL-B4 may be blue light. However, the embodiment of the present disclosure is not limited thereto, and the light beams respectively emitted from the light emitting structures OL-B1, OL-B2, OL-B3, and OL-B4 may have wavelength ranges different from each other. For example, the light emitting device ED-BT including the plurality of light emitting structures OL-B1, OL-B2, OL-B3, and OL-B4 which emit light beams having wavelength ranges different from each other may be to emit white light.

Charge generation layers CGL1, CGL2 and CGL3 may be respectively disposed between two neighboring light emitting structures OL-B1, OL-B2, OL-B3, and OL-B4. The charge generation layers CGL1, CGL2 and CGL3 may include a p-type charge generation layer and/or an n-type charge generation layer.

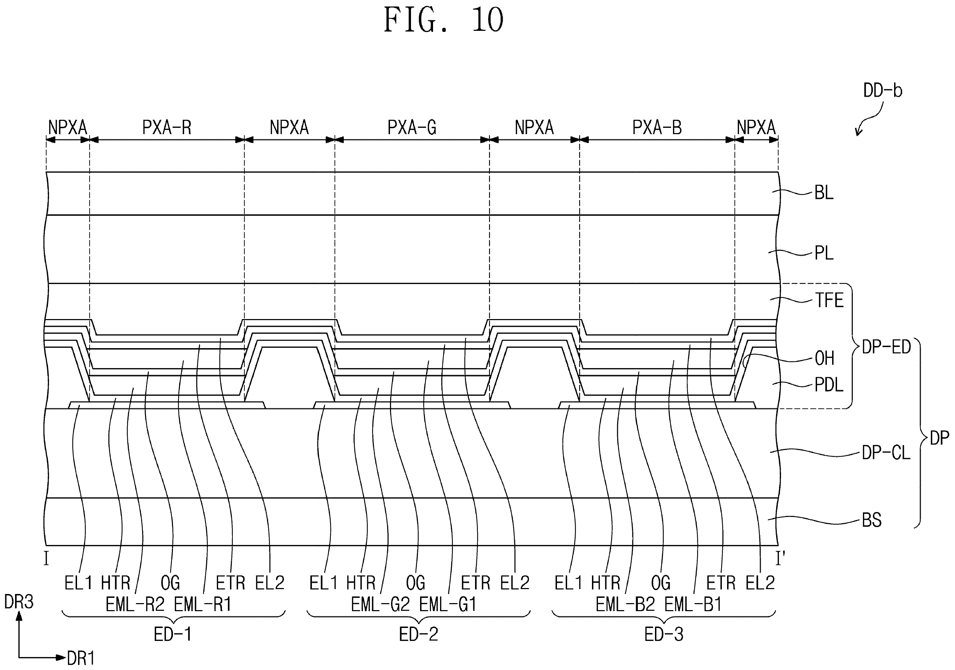

Referring to FIG. 10, the display apparatus DD-b according to one or more embodiments may include light emitting devices ED-1, ED-2, and ED-3 in which two emission layers are stacked. Compared with the display apparatus DD of the embodiment(s) illustrated in FIG. 2, the embodiment(s) illustrated in FIG. 10 has a difference in that the first to third light emitting devices ED-1, ED-2, and ED-3 each include two emission layers stacked in the thickness direction. In each of the first to third light emitting devices ED-1, ED-2, and ED-3, the two emission layers may be to emit light in substantially the same wavelength region.

The first light emitting device ED-1 may include a first red emission layer EML-R1 and a second red emission layer EML-R2. The second light emitting device ED-2 may include a first green emission layer EML-G1 and a second green emission layer EML-G2. In some embodiments, the third light emitting device ED-3 may include a first blue emission layer EML-B1 and a second blue emission layer EML-B2. An emission auxiliary part OG may be disposed between the first red emission layer EML-R1 and the second red emission layer EML-R2, between the first green emission layer EML-G1 and the second green emission layer EML-G2, and between the first blue emission layer EML-B1 and the second blue emission layer EML-B2.

The emission auxiliary part OG may include a single layer or a multilayer. The emission auxiliary part OG may include a charge generation layer. For example, the emission auxiliary part OG may include an electron transport region, a charge generation layer, and a hole transport region that are sequentially stacked. The emission auxiliary part OG may be provided as a common layer in the whole of the first to third light emitting devices ED-1, ED-2, and ED-3. However, the embodiment of the present disclosure is not limited thereto, and the emission auxiliary part OG may be provided by being patterned within the openings OH defined in the pixel defining film PDL.

The first red emission layer EML-R1, the first green emission layer EML-G1, and the first blue emission layer EML-B1 may be disposed between the hole transport region HTR and the emission auxiliary part OG. The second red emission layer EML-R2, the second green emission layer EML-G2, and the second blue emission layer EML-B2 may be disposed between the emission auxiliary part OG and the electron transport region ETR.

For example, the first light emitting device ED-1 may include the first electrode EL1, the hole transport region HTR, the second red emission layer EML-R2, the emission auxiliary part OG, the first red emission layer EML-R1, the electron transport region ETR, and the second electrode EL2 that are sequentially stacked. The second light emitting device ED-2 may include the first electrode EL1, the hole transport region HTR, the second green emission layer EML-G2, the emission auxiliary part OG, the first green emission layer EML-G1, the electron transport region ETR, and the second electrode EL2 that are sequentially stacked. The third light emitting device ED-3 may include the first electrode EL1, the hole transport region HTR, the second blue emission layer EML-B2, the emission auxiliary part OG, the first blue emission layer EML-B1, the electron transport region ETR, and the second electrode EL2 that are sequentially stacked.

In some embodiments, an optical auxiliary layer PL may be disposed on the display device layer DP-ED. The optical auxiliary layer PL may include a polarizing layer. The optical auxiliary layer PL may be disposed on the display panel DP and control reflected light in the display panel DP due to external light. In one or more embodiments, the optical auxiliary layer PL in the display apparatus according to one or more embodiments may not be provided.

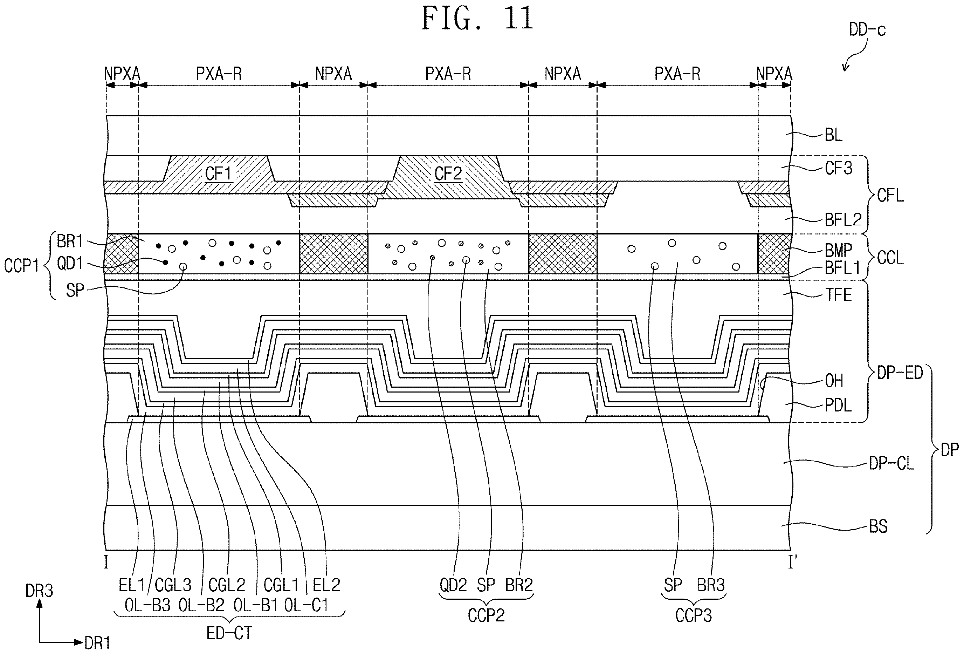

Unlike FIGS. 8 and 10, FIG. 11 illustrates that a display apparatus DD-c includes four light emitting structures OL-B1, OL-B2, OL-B3, and OL-C1. A light emitting device ED-CT may include a first electrode EL1 and a second electrode EL2 which face each other, and first to fourth light emitting structures OL-B1, OL-B2, OL-B3, and OL-C1 that are sequentially stacked in the thickness direction between the first electrode EL1 and the second electrode EL2. Charge generation layers CGL1, CGL2, and CGL3 may be disposed between neighboring ones of the first to fourth light emitting structures OL-B1, OL-B2, OL-B3, and OL-C1. Among the four light emitting structures, the first to third light emitting structures OL-B1, OL-B2, and OL-B3 may be to emit blue light, and the fourth light emitting structure OL-C1 may be to emit green light. However, the embodiment of the present disclosure is not limited thereto, and the first to fourth light emitting structures OL-B1, OL-B2, OL-B3, and OL-C1 may be to emit light beams in different wavelength regions.

Hereinafter, with reference to Examples and Comparative Examples, a compound according to one or more embodiments of the present disclosure and a light emitting device of one or more embodiments will be described in more detail. Examples described below are only illustrations to assist the understanding of the present disclosure, and the scope of the present disclosure is not limited thereto.

### Examples

### 1. Synthesis of Amine Compound

First, a synthetic method of an amine compound according to the present embodiments will be described in more detail by illustrating the synthetic methods of Compounds 4, 14, 18, 20, 24, 35, 63, 68, 77, 91, 165, and 172. Also, in the following descriptions, the synthetic method of the amine compound is provided as an example, but the synthetic method according to one or more embodiments of the present disclosure is not limited to the Examples.

### (1) Synthesis of Compound 4

Amine compound 4 according to one or more embodiments may be synthesized by, for example, the reaction below.

### Synthesis of Intermediate 4-1

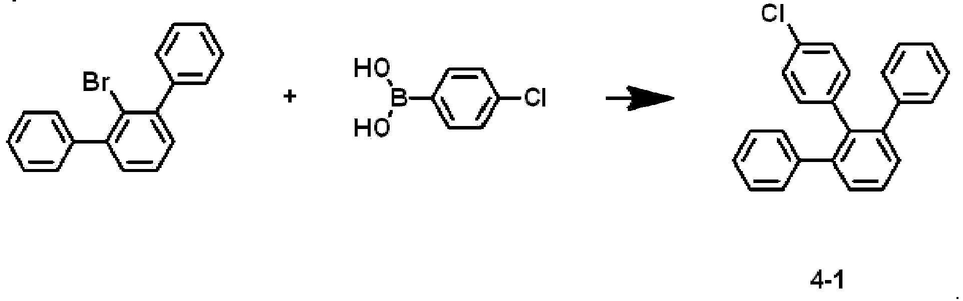

(4-chlorophenyl)boronic acid (1.56 g, 10.0 mmol), 2'-bromo-1,1':3',1"-terphenyl (3.09 g, 10.0 mmol), Pd(PPh₃)₄ (0.58 g, 0.5 mmol), and K₂CO₃ (4.14 g, 30.0 mmol) were dissolved in a mixed solution (60 mL) of THF/H₂O (2/1), and then the resulting mixture was stirred at about 80 °C for about 16 hours. The reaction solution was cooled to room temperature and then extracted three times with water (60 mL) and diethyl ether (60 mL). An extracted organic layer was dried over MgSO₄, and residues obtained by evaporating the solvent were separated and purified by silica gel column chromatography to obtain Intermediate 4-1 (2.39 g, yield 70%). The resulting compound was identified through LC-MS, and the results are as follows: C₂₄H₁₇Cl: M⁺340.8

### Synthesis of Intermediate 4-2

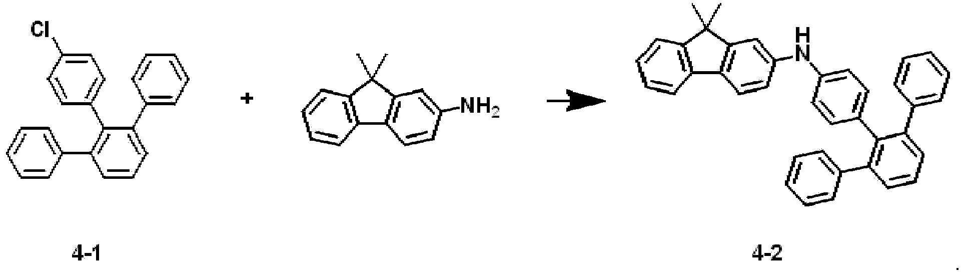

Intermediate 4-1 (3.41 g, 10.0 mmol), 9,9-dimethyl-9H-fluoren-2-amine (3.14 g, 15 mmol), tris(dibenzylideneacetone)dipalladium(0) (Pd₂dba₃) (0.46 g, 0.5 mmol), P(t-Bu)₃ (0.24 g, 1 mmol), and sodium tert-butoxide (2.88 g, 30 mmol) were dissolved in o-xylene (60 mL) and then stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Intermediate 4-2 (3.60 g, yield 70%). The resulting compound was identified through LC-MS, and the results are as follows: C₃₉H₃₁N: M+513.6

### Synthesis of Compound 4

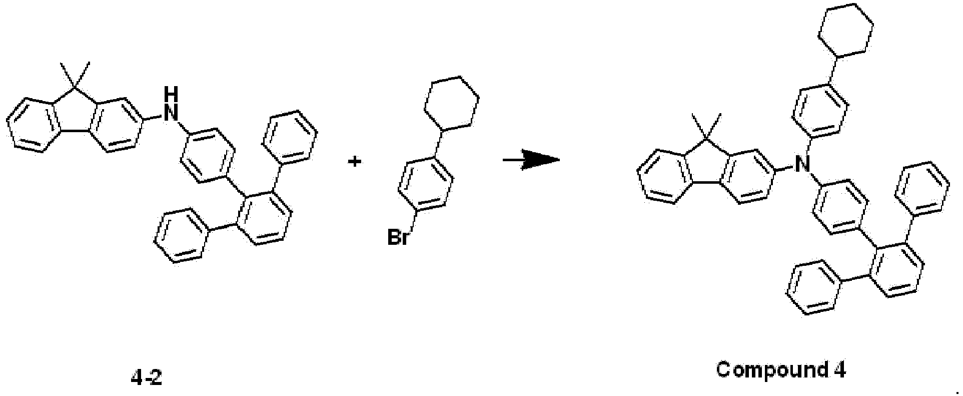

Intermediate 4-2 (5.14 g, 10.0 mmol), 1-bromo-4-cyclohexylbenzene (2.39 g, 10 mmol), Pd₂dba₃ (0.46 g, 0.5 mmol), P(t-Bu)₃ (0.24 g, 1 mmol), and sodium tert-butoxide (2.88 g, 30 mmol) were dissolved in toluene (60 mL) and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Compound 4 (4.36 g, yield 65%). The resulting compound was identified through MS/FAB and 1H NMR, and the results are listed in Table 1 below.

### (2) Synthesis of Compound 14

### Synthesis of Intermediate 14-1

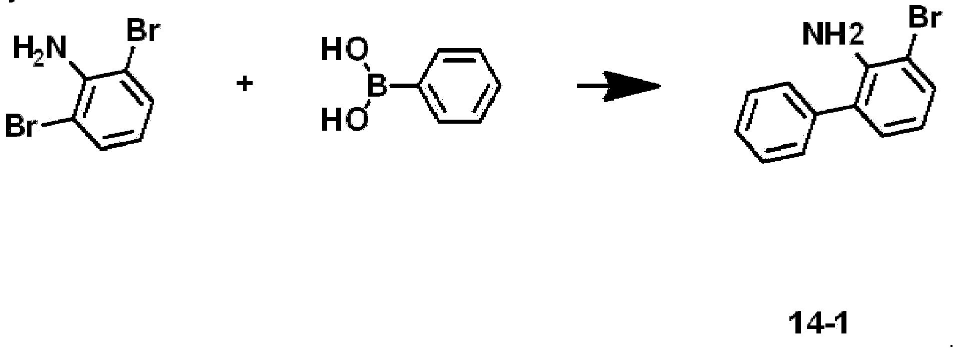

Phenylboronic acid (1.22 g, 10.0 mmol), 2,6-dibromoaniline (3.01 g, 12.0 mmol), Pd(PPh₃)₄ (0.58 g, 0.5 mmol), and K₂CO₃ (4.14 g, 30.0 mmol) were dissolved in a mixed solution (60 mL) of THF/H₂O (2/1), and then the resulting mixture was stirred at about 80 °C for about 16 hours. The reaction solution was cooled to room temperature and then extracted three times with water (60 mL) and diethyl ether (60 mL). An extracted organic layer was dried over MgSO₄, and residues obtained by evaporating the solvent were separated and purified by silica gel column chromatography to obtain Intermediate 14-1 (1.24 g, yield 50%). The resulting compound was identified through LC-MS, and the results are as follows: C₁₂H₁₀BrN: M⁺248.1

### Synthesis of Intermediate 14-2

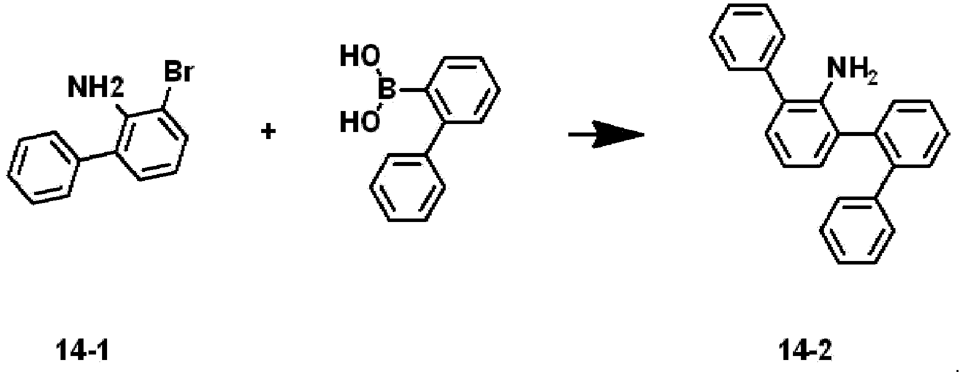

2-biphenylboronic acid (1.98 g, 10.0 mmol), Intermediate 14-1 (2.48 g, 10.0 mmol), Pd(PPh₃)₄ (0.58 g, 0.5 mmol), and K₂CO₃ (4.14 g, 30.0 mmol) were dissolved in a mixed solution (60 mL) of THF/H₂O (2/1), and then the resulting mixture was stirred at about 80 °C for about 16 hours. The reaction solution was cooled to room temperature and then extracted three times with water (60 mL) and diethyl ether (60 mL). An extracted organic layer was dried over MgSO₄, and residues obtained by evaporating the solvent were separated and purified by silica gel column chromatography to obtain Intermediate 14-2 (2.09 g, yield 65%). The resulting compound was identified through LC-MS, and the results are as follows: C₂₄H₁₉N: M⁺321.4

### Synthesis of Intermediate 14-3

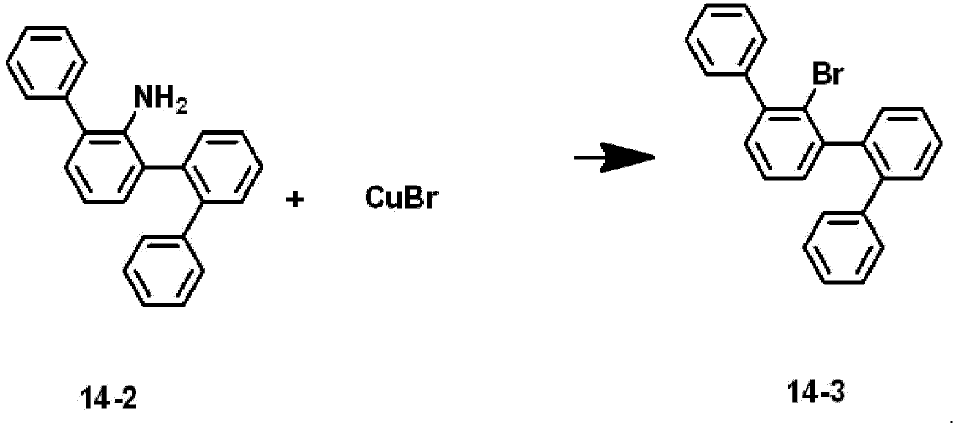

Intermediate 14-2 (3.21 g, 10 mmol) and CuBr (4.29 g, 30 mmol) were dissolved in 48% hydrobromic acid aqueous solution (10 mL), and then 2.07 g of NaNO₂ (in H₂O) was slowly added thereto at about 0 °C. The resulting mixture was stirred at room temperature for about 5 hours, and then Na₂S₂O₃ (3 g) was dissolved in water and added thereto, and the resulting mixture was washed three times with DCM (30 mL). The washed DCM layer was dried over MgSO₄, and dried under reduced pressure to obtain a product, and then the product was separated and purified by silica gel column chromatography to obtain Intermediate 14-3 (2.70 g, yield 70%). The resulting compound was identified through LC-MS, and the results are as follows: C₂₄H₁₇Br: M+385.3

### Synthesis of Intermediate 14-4

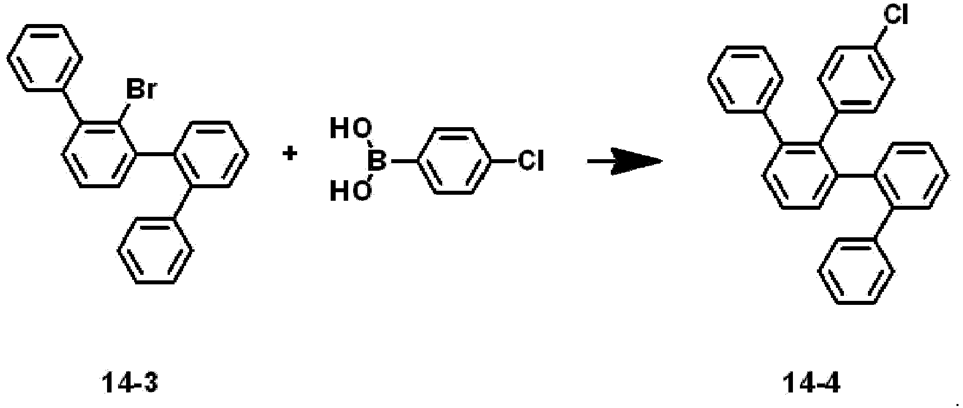

(4-chlorophenyl)boronic acid (1.56 g, 10.0 mmol), Intermediate 14-3 (3.85 g, 10.0 mmol), Pd(PPh₃)₄ (0.58 g, 0.5 mmol), and K₂CO₃ (4.14 g, 30.0 mmol) were dissolved in a mixed solution (60 mL) of THF/H₂O (2/1), and then the resulting mixture was stirred at about 80 °C for about 16 hours. The reaction solution was cooled to room temperature and then extracted three times with water (60 mL) and diethyl ether (60 mL). An extracted organic layer was dried over MgSO₄, and residues obtained by evaporating the solvent were separated and purified by silica gel column chromatography to obtain Intermediate 14-4 (2.92 g, yield 70%). The resulting compound was identified through LC-MS, and the results are as follows: C₃₀H₂₁Cl: M⁺416.9

### Synthesis of Intermediate 14-5

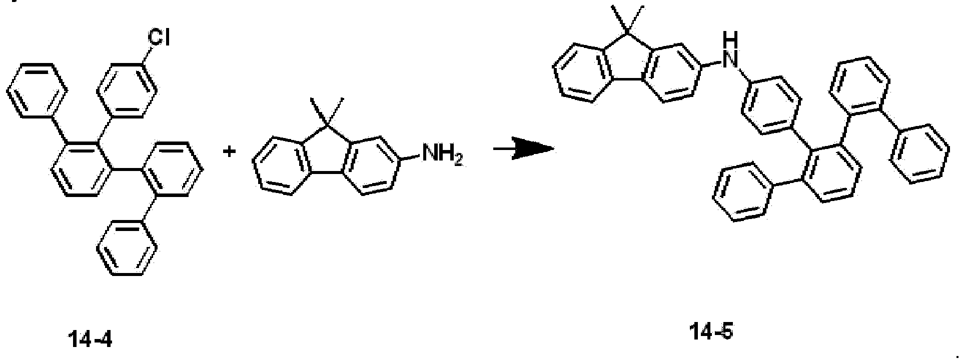

Intermediate 14-4 (4.17g, 10.0 mmol), 9,9-dimethyl-9H-fluoren-2-amine (3.14 g, 15 mmol), Pd₂dba₃ (0.46 g, 0.5 mmol), P(t-Bu)₃ (0.24 g, 1 mmol), and sodium tert-butoxide (2.88 g, 30 mmol) were dissolved in o-xylene (60 mL) and then stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Intermediate 14-5 (4.13 g, yield 70%). The resulting compound was identified through LC-MS, and the results are as follows: C₄₅H₃₅N: M+589.7

### Synthesis of Compound 14

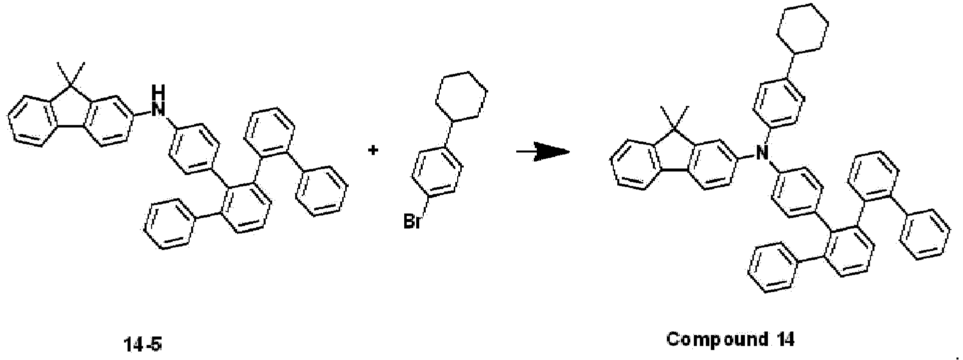

Intermediate 14-5 (5.90 g, 10.0 mmol), 1-bromo-4-cyclohexylbenzene (2.39 g, 10 mmol), Pd₂dba₃ (0.46 g, 0.5 mmol), P(t-Bu)₃ (0.24 g, 1 mmol), and sodium tert-butoxide (2.88 g, 30 mmol) were dissolved in toluene (60 mL), and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Compound 14 (4.86 g, yield 65%). The resulting compound was identified through MS/FAB and 1H NMR, and the results are listed in Table 1.

### (3) Synthesis of Compound 18

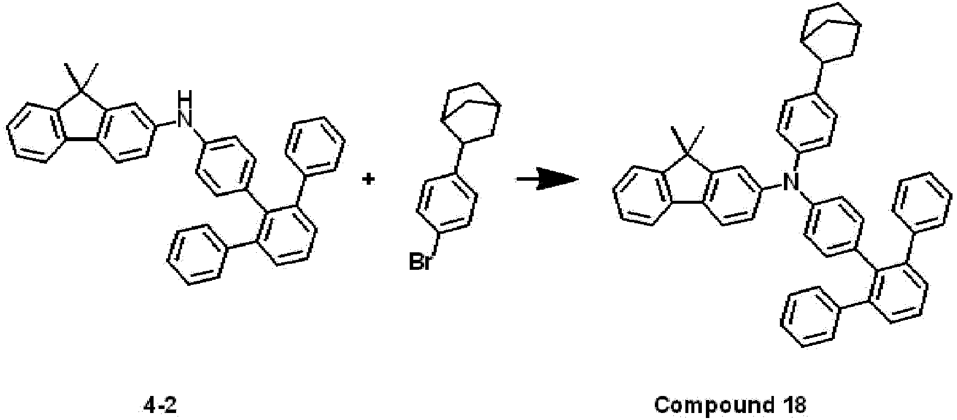

### Synthesis of Compound 18

Compound 18 was synthesized in substantially the same manner as the synthesis of Compound 4 except for using 2-(4-bromophenyl)bicyclo[2.2.1]heptane instead of 1-bromo-4-cyclohexylbenzene. The resulting compound was identified through MS/FAB and 1H NMR, and the results are listed in Table 1.

### (4) Synthesis of Compound 20

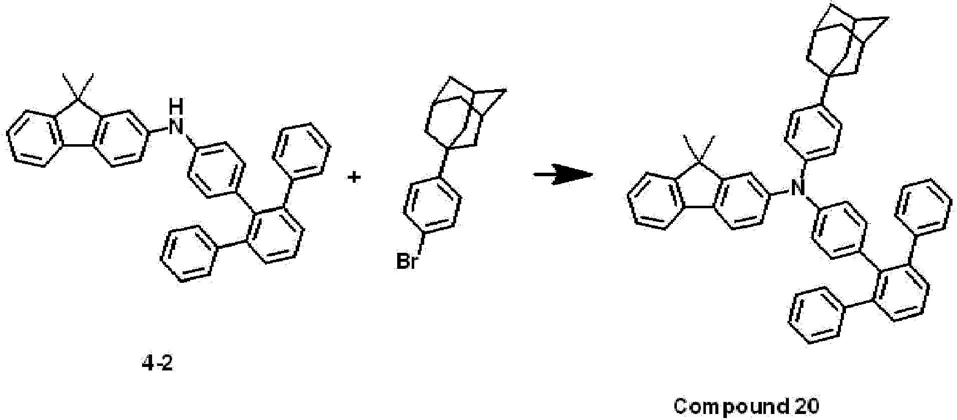

### (Synthesis of Compound 20)

Compound 20 was synthesized in substantially the same manner as the synthesis of Compound 4 except for using (3r,5r,7r)-1-(4-bromophenyl)adamantane instead of 1-bromo-4-cyclohexylbenzene. The resulting compound was identified through MS/FAB and 1H NMR, and the results are listed in Table 1.

### (5) Synthesis of Compound 24

### Synthesis of Intermediate 24-1

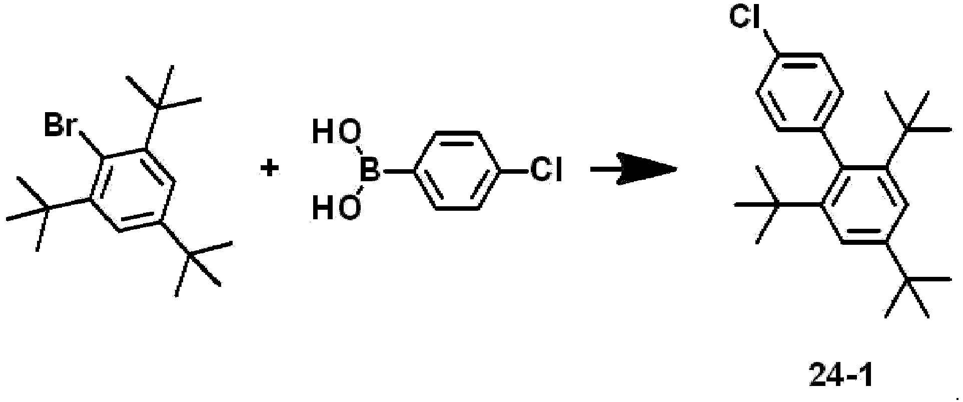

(4-chlorophenyl)boronic acid (1.56 g, 10.0 mmol), 2-bromo-1,3,5-tri-tert-butylbenzene (3.25 g, 10.0 mmol), Pd(PPh₃)₄ (0.58 g, 0.5 mmol), and K₂CO₃ (4.14 g, 30.0 mmol) were dissolved in a mixed solution (60 mL) of THF/H₂O (2/1), and then the resulting mixture was stirred at about 80 °C for about 16 hours. The reaction solution was cooled to room temperature and then extracted three times with water (60 mL) and diethyl ether (60 mL). An extracted organic layer was dried over MgSO₄, and residues obtained by evaporating the solvent were separated and purified by silica gel column chromatography to obtain Intermediate 24-1 (2.50 g, yield 70%). The resulting compound was identified through LC-MS, and the results are as follows: C₂₄H₃₃Cl: M⁺356.9

### Synthesis of Intermediate 24-2

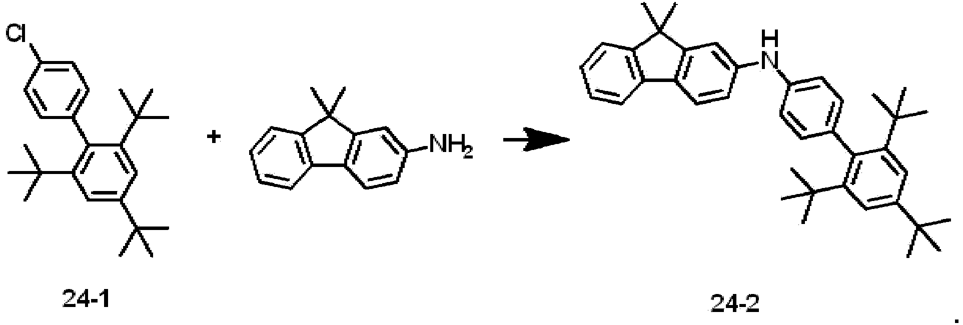

Intermediate 24-1 (3.57g, 10.0 mmol), 9,9-dimethyl-9H-fluoren-2-amine (3.14 g, 15 mmol), Pd₂dba₃ (0.46 g, 0.5 mmol), P(t-Bu)₃ (0.24 g, 1 mmol), and sodium tert-butoxide (2.88 g, 30 mmol) were dissolved in o-xylene (60 mL) and then stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Intermediate 24-2 (3.71 g, yield 70%). The resulting compound was identified through LC-MS, and the results are as follows: C₃₉H₄₇N: M+529.8

### Synthesis of Compound 24

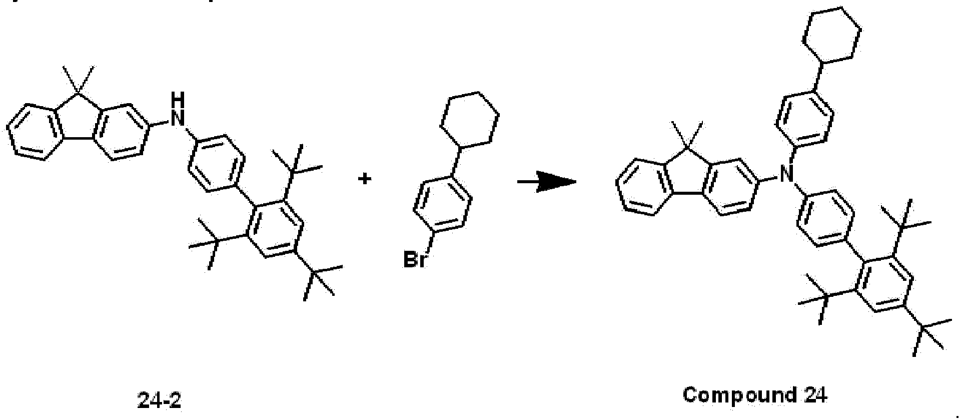

Intermediate 24-2 (5.30g, 10.0 mmol), 1-bromo-4-cyclohexylbenzene (2.39 g, 10 mmol), Pd₂dba₃ (0.46 g, 0.5 mmol), P(t-Bu)₃ (0.24 g, 1 mmol), and sodium tert-butoxide (2.88 g, 30 mmol) were dissolved in toluene (60 mL), and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Compound 24 (4.47 g, yield 65%). The resulting compound was identified through MS/FAB and 1H NMR, and the results are listed in Table 1.

### (6) Synthesis of Compound 35

### Synthesis of Intermediate 35-1

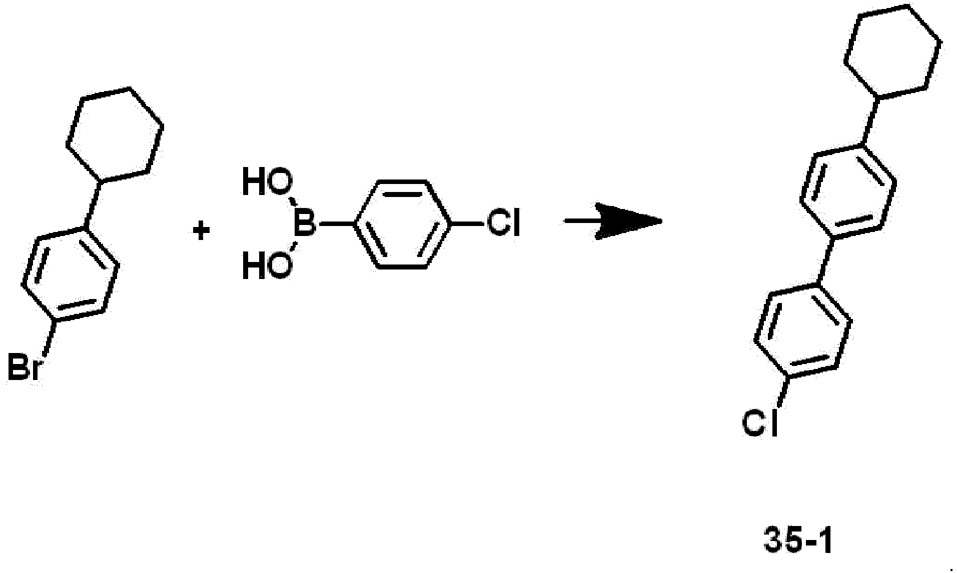

(4-chlorophenyl)boronic acid (1.56 g, 10.0 mmol), 1-bromo-4-cyclohexylbenzene (2.39 g, 10.0 mmol), Pd(PPh₃)₄ (0.58 g, 0.5 mmol), and K₂CO₃ (4.14 g, 30.0 mmol) were dissolved in a mixed solution (60 mL) of THF/H₂O (2/1), and then the resulting mixture was stirred at about 80 °C for about 16 hours. The reaction solution was cooled to room temperature and then extracted three times with water (60 mL) and diethyl ether (60 mL). An extracted organic layer was dried over MgSO₄, and residues obtained by evaporating the solvent were separated and purified by silica gel column chromatography to obtain Intermediate 35-1 (1.90 g, yield 70%). The resulting compound was identified through LC-MS, and the results are as follows: C₁₈H₁₉Cl: M⁺270.8

### Synthesis of Compound 35

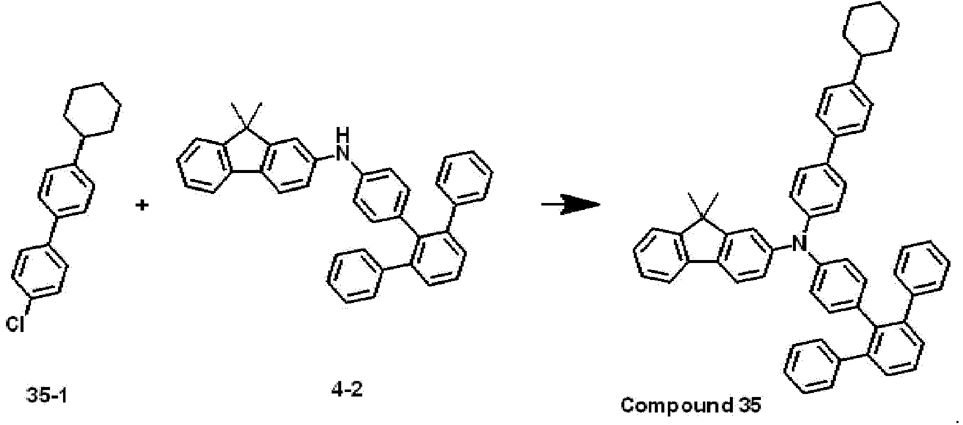

Intermediate 35-1 (2.71 g, 10.0 mmol) Intermediate 4-2 (5.14 g, 10 mmol), Pd₂dba₃ (0.46 g, 0.5 mmol), P(t-Bu)₃ (0.24 g, 1 mmol), and sodium tert-butoxide (2.88 g, 30 mmol) were dissolved in toluene (60 mL), and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Compound 35 (4.86 g, yield 65%). The resulting compound was identified through MS/FAB and 1H NMR, and the results are listed in Table 1.

### (7) Synthesis of Compound 63

### Synthesis of Intermediate 63-1

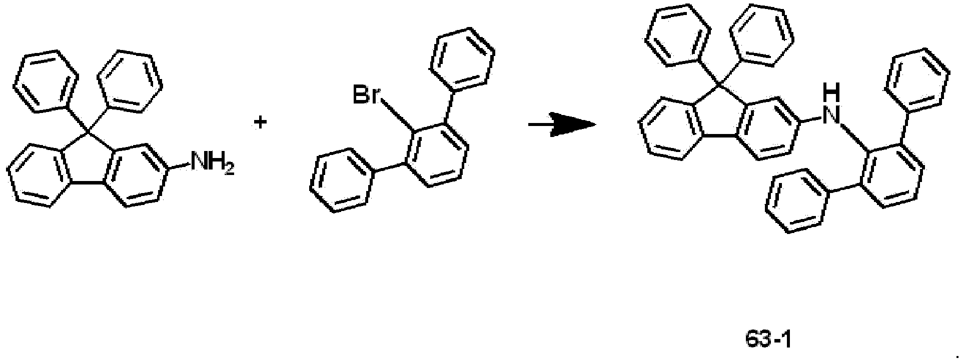

9,9-diphenyl-9H-fluoren-2-amine (5.00 g, 15.0 mmol), 2'-bromo-1,1':3',1"-terphenyl (3.09 g, 10 mmol), Pd₂dba₃ (0.46 g, 0.5 mmol), P(t-Bu)₃ (0.24 g, 1 mmol), and sodium tert-butoxide (2.88 g, 30 mmol) were dissolved in o-xylene (60 mL), and then the resulting mixture was stirred at about 80 °C for about 18 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Intermediate 63-1 (2.81 g, yield 50%). The resulting compound was identified through LC-MS, and the results are as follows: C₄₃H₃₁N: M+561.7

### Synthesis of Compound 63

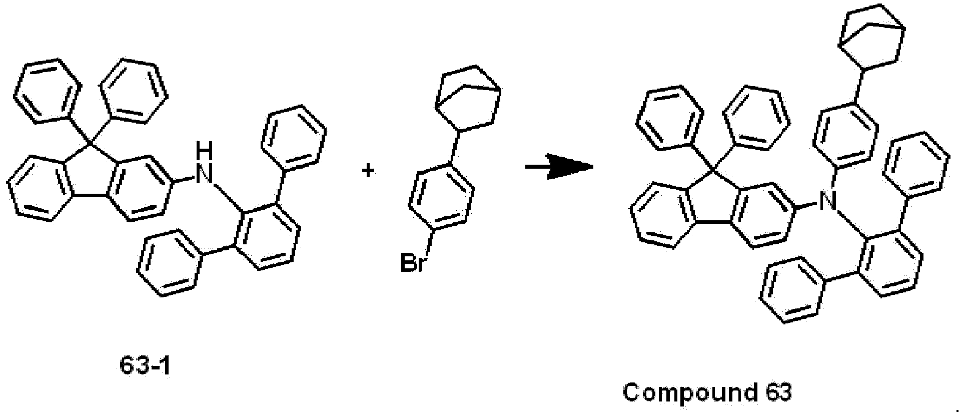

Intermediate 63-1 (5.62 g, 10.0 mmol), 2-(4-bromophenyl)bicyclo[2.2.1]heptane (2.51 g, 10 mmol), Pd₂dba₃ (0.46 g, 0.5 mmol), P(t-Bu)₃ (0.24 g, 1 mmol), and sodium tert-butoxide (2.88 g, 30 mmol) were dissolved in toluene (60 mL), and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Compound 63 (3.30 g, yield 45%). The resulting compound was identified through MS/FAB and 1H NMR, and the results are listed in Table 1.

### (8) Synthesis of Compound 68

### Synthesis of Intermediate 68-1

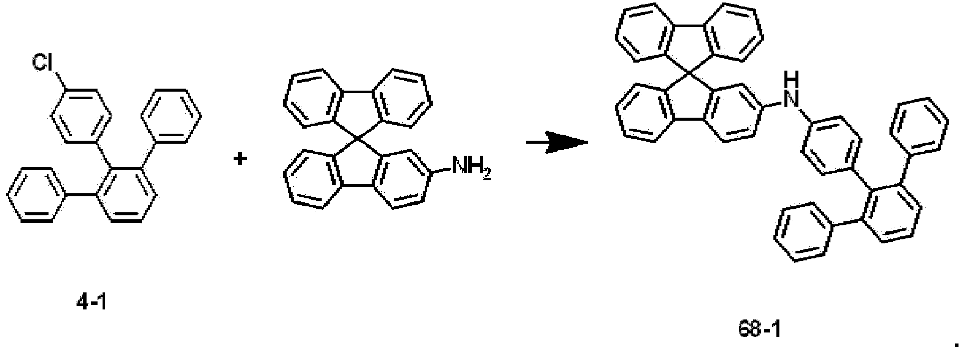

Intermediate 68-1 was synthesized in substantially the same manner as the synthesis of Intermediate 4-2 except for using 9,9'-spirobi[fluoren]-2-amine instead of 9,9-dimethyl-9H-fluoren-2-amine. The resulting compound was identified through LC-MS, and the results are as follows: C₄₉H₃₃N: M+635.8

### Synthesis of Compound 68

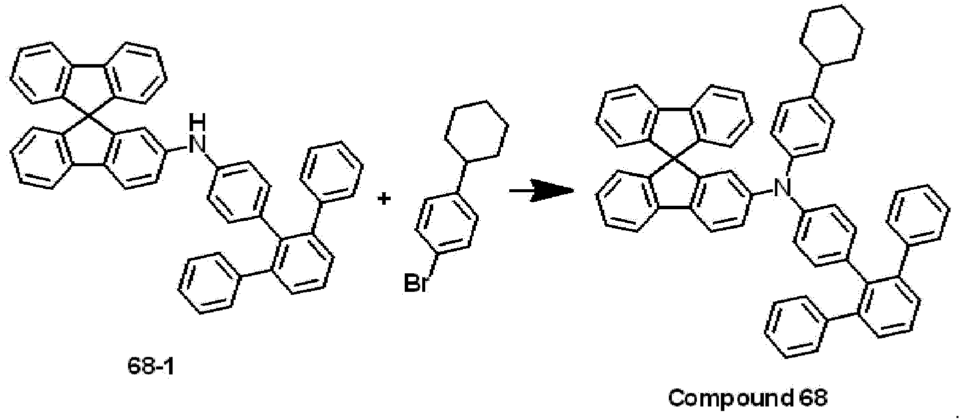

Compound 68 was synthesized in substantially the same manner as the synthesis of Compound 4 except for using Intermediate 68-1 instead of Intermediate 4-2. The resulting compound was identified through MS/FAB and 1H NMR, and the results are listed in Table 1.

### (9) Synthesis of Compound 77

### Synthesis of Intermediate 77-1

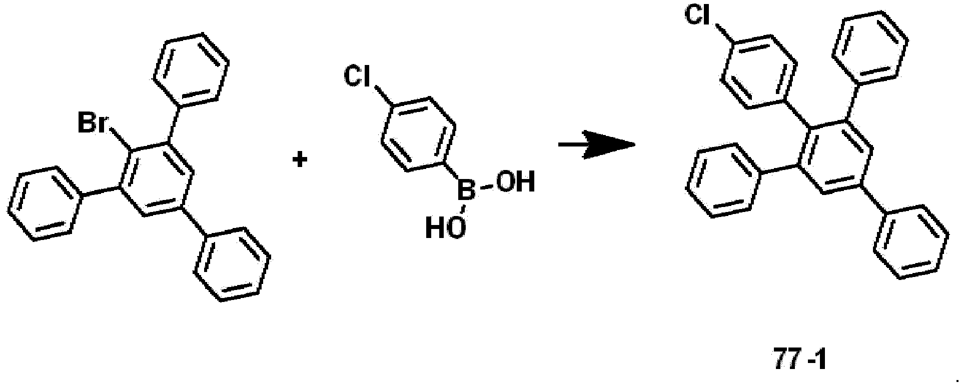

(4-chlorophenyl)boronic acid (1.56 g, 10.0 mmol), 2'-bromo-5'-phenyl-1,1':3',1"-terphenyl (3.85 g, 10.0 mmol), Pd(PPh₃)₄ (0.58 g, 0.5 mmol), and K₂CO₃ (4.14 g, 30.0 mmol) were dissolved in a mixed solution (60 mL) of THF/H₂O (2/1), and then the resulting mixture was stirred at about 80 °C for about 16 hours. The reaction solution was cooled to room temperature and then extracted three times with water (60 mL) and diethyl ether (60 mL). An extracted organic layer was dried over MgSO₄, and residues obtained by evaporating the solvent were separated and purified by silica gel column chromatography to obtain Intermediate 77-1 (2.92 g, yield 70%). The resulting compound was identified through LC-MS, and the results are as follows: C₃₀H₂₁Cl: M⁺416.9

### Synthesis of Intermediate 77-2

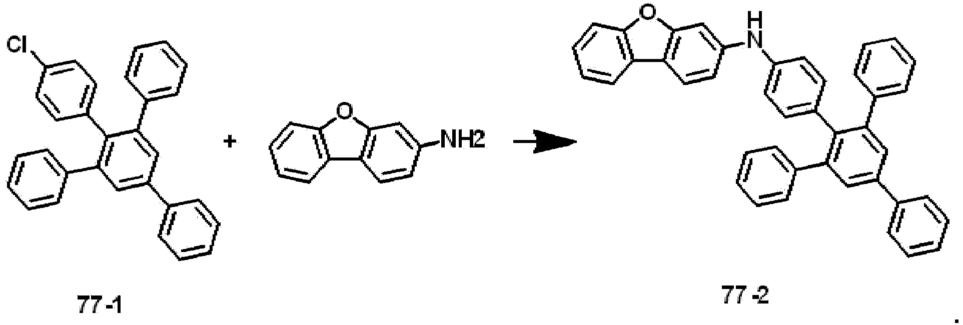

Intermediate 77-1 (3.41 g, 10.0 mmol), dibenzo[b,d]furan-3-amine (2.75 g, 15 mmol), Pd₂dba₃ (0.46 g, 0.5 mmol), P(t-Bu)₃ (0.24 g, 1 mmol), and sodium tert-butoxide (2.88 g, 30 mmol) were dissolved in o-xylene (60 mL), and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Intermediate 77-2 (3.95 g, yield 70%). The resulting compound was identified through LC-MS, and the results are as follows: C₄₂H₂₉NO: M+563.7

### Synthesis of Compound 77

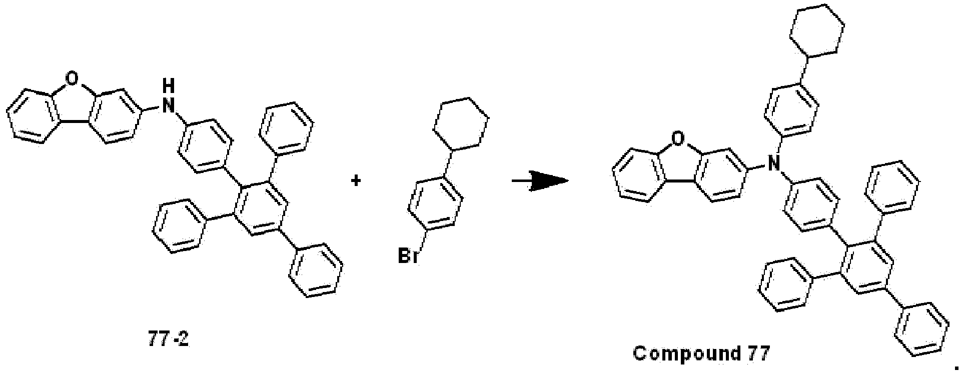

Intermediate 77-2 (5.64 g, 10.0 mmol), 1-bromo-4-cyclohexylbenzene (2.39 g, 10 mmol), Pd₂dba₃ (0.46 g, 0.5 mmol), P(t-Bu)₃ (0.24 g, 1 mmol), and sodium tert-butoxide (2.88 g, 30 mmol) were dissolved in toluene (60 mL), and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Compound 77 (4.69 g, yield 65%). The resulting compound was identified through MS/FAB and 1H NMR, and the results are listed in Table 1.

### (10) Synthesis of Compound 91

### Synthesis of Intermediate 91-1

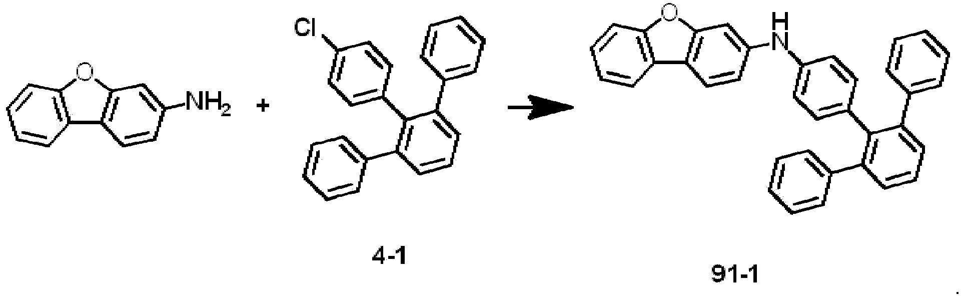

Dibenzo[b,d]furan-3-amine (2.75 g, 15.0 mmol), Intermediate 4-1 (3.41 g, 10.0 mmol), Pd₂dba₃ (0.46 g, 0.5 mmol), P(t-Bu)₃ (0.24 g, 1 mmol), and sodium tert-butoxide (2.88 g, 30 mmol) were dissolved in o-xylene (60 mL), and then the resulting mixture was stirred at about 80 °C for about 18 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Intermediate 91-1 (2.44g, yield 50 %). The resulting compound was identified through LC-MS, and the results are as follows: C₂₄H₁₇N: M+340.8

### Synthesis of Compound 91

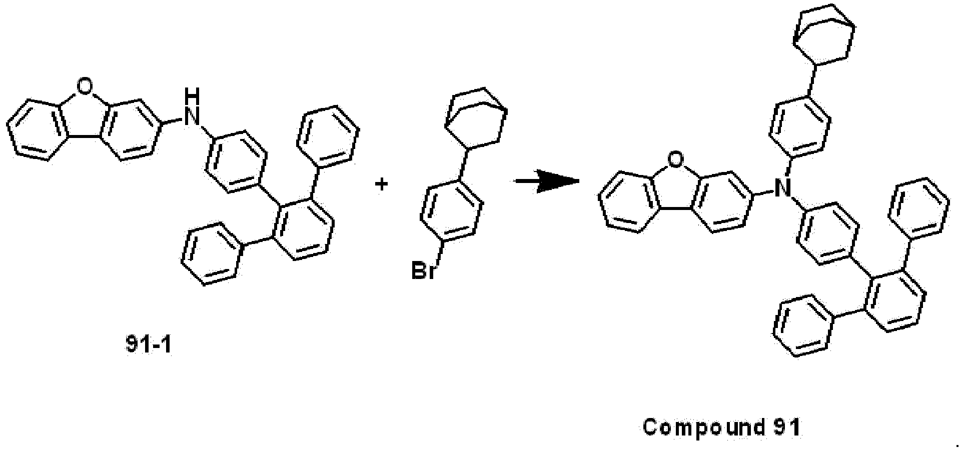

Intermediate 91-1 (4.88 g, 10.0 mmol), 2-(4-bromophenyl)bicyclo[2.2.2]octane (2.65 g, 10 mmol), Pd₂dba₃ (0.46 g, 0.5 mmol), P(t-Bu)₃ (0.24 g, 1 mmol), and sodium tert-butoxide (2.88 g, 30 mmol) were dissolved in toluene (60 mL), and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Compound 91 (3.02 g, yield 45%). The resulting compound was identified through MS/FAB and 1H NMR, and the results are listed in Table 1.

### (11) Synthesis of Compound 165

### Synthesis of Intermediate 165-1

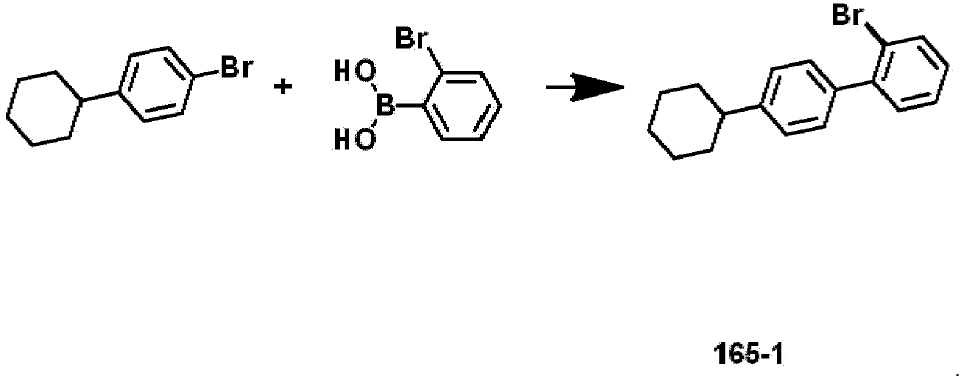

(2-bromophenyl)boronic acid (2.01 g, 10.0 mmol), 1-bromo-4-cyclohexylbenzene (2.39 g, 10.0 mmol), Pd(PPh₃)₄ (0.58 g, 0.5 mmol), and K₂CO₃ (4.14 g, 30.0 mmol) were dissolved in a mixed solution (60 mL) of THF/H₂O (2/1), and then the resulting mixture was stirred at about 80 °C for about 16 hours. The reaction solution was cooled to room temperature and then extracted three times with water (60 mL) and diethyl ether (60 mL). An extracted organic layer was dried over MgSO₄, and residues obtained by evaporating the solvent were separated and purified by silica gel column chromatography to obtain Intermediate 165-1 (1.89 g, yield 60%). The resulting compound was identified through LC-MS, and the results are as follows: C₁₈H₁₉Br: M⁺315.2

### Synthesis of Intermediate 165-2

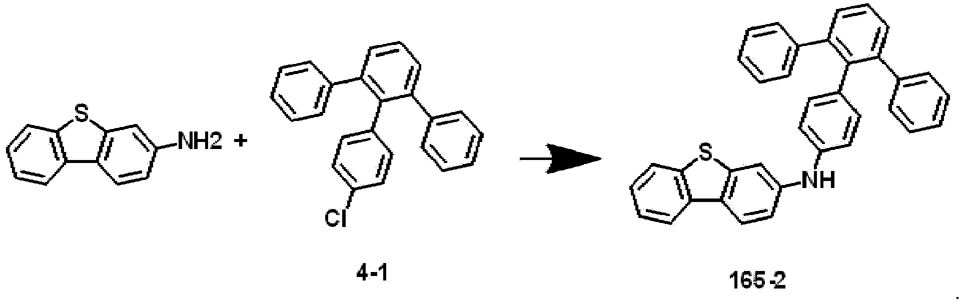

Intermediate 4-1 (3.41 g, 10.0 mmol), dibenzo[b,d]thiophen-3-amine (2.99 g, 15 mmol), Pd₂dba₃ (0.46 g, 0.5 mmol), P(t-Bu)₃ (0.24 g, 1 mmol), and sodium tert-butoxide (2.88 g, 30 mmol) were dissolved in o-xylene (60 mL), and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Intermediate 165-2 (3.53 g, yield 70%). The resulting compound was identified through LC-MS, and the results are as follows: C₃₆H₂₅NS: M+503.6

### Synthesis of Compound 165

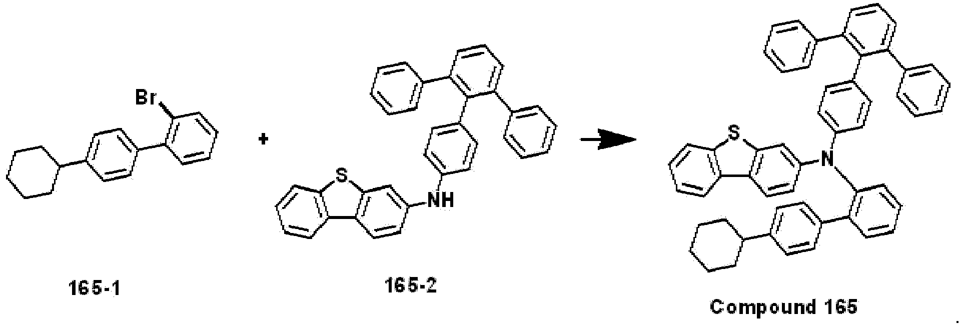

Intermediate 165-1 (3.15 g, 10.0 mmol) Intermediate 165-2 (5.04 g, 10 mmol), Pd₂dba₃ (0.46 g, 0.5 mmol), P(t-Bu)₃ (0.24 g, 1 mmol), and sodium tert-butoxide (2.88 g, 30 mmol) were dissolved in toluene (60 mL), and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Compound 165 (4.80 g, yield 65 %). The resulting compound was identified through MS/FAB and 1H NMR, and the results are listed in Table 1.

### (12) Synthesis of Compound 172

### Synthesis of Intermediate 172-1

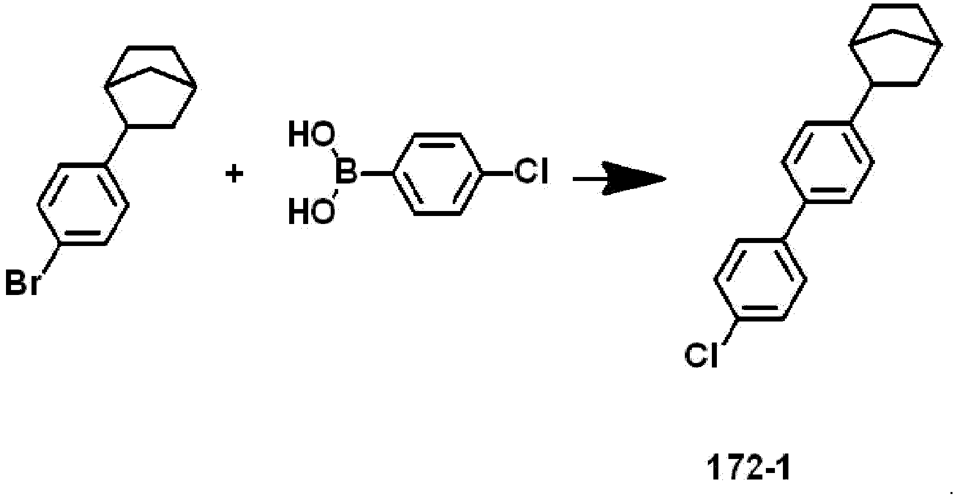

(4-chlorophenyl)boronic acid (1.56 g, 10.0 mmol), 2-(4-bromophenyl)bicyclo[2.2.1]heptane (2.51 g, 10.0 mmol), Pd(PPh₃)₄ (0.58 g, 0.5 mmol), and K₂CO₃ (4.14 g, 30.0 mmol) were dissolved in a mixed solution (60 mL) of THF/H₂O (2/1), and then the resulting mixture was stirred at about 80 °C for about 16 hours. The reaction solution was cooled to room temperature and then extracted three times with water (60 mL) and diethyl ether (60 mL). An extracted organic layer was dried over MgSO₄, and residues obtained by evaporating the solvent were separated and purified by silica gel column chromatography to obtain Intermediate 172-1 (1.98 g, yield 70%). The resulting compound was identified through LC-MS, and the results are as follows: C₁₉H₁₉Cl: M⁺282.8

### Synthesis of Compound 172

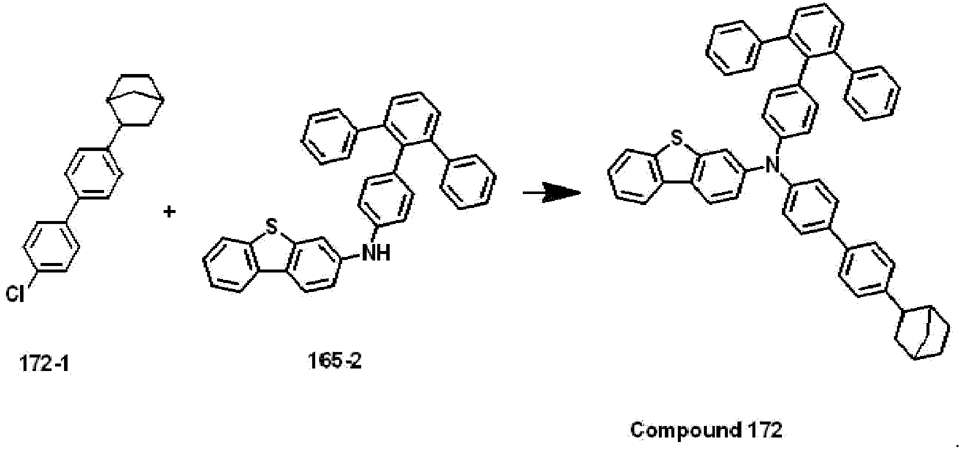

Intermediate 172-1 (2.83 g, 10.0 mmol) Intermediate 165-2 (5.04 g, 10 mmol), Pd₂dba₃ (0.46 g, 0.5 mmol), P(t-Bu)₃ (0.24 g, 1 mmol), and sodium tert-butoxide (2.88 g, 30 mmol) were dissolved in toluene (60 mL), and then the resulting mixture was stirred at about 80 °C for about 3 hours. The reaction solution was cooled to room temperature, 40 mL of water was added thereto, and the mixture was extracted three times with 50 mL of ethyl ether. An extracted organic layer was dried over MgSO₄, and residues obtained by evaporating the solvent were separated and purified by silica gel column chromatography to obtain Compound 172 (4.88 g, yield 65%). The resulting compound was identified through MS/FAB and 1H NMR, and the results are listed in Table 1.

### 2. Evaluation of Amine Compound Physical Properties

The results of MS/FAB and ¹H NMR of Example Compounds are listed in Table 1:

**Table 1**

| Compound | ¹H NMR (CDCl₃, 400 MHz) | MS/FAB | |
|---|---|---|---|
| | | found | calc. |
| Compound 4 | 8.06(d, 2H), 7.90-7.79(m, 7H), 7.55-7.28(m, 14H), 7.18-7.16(m, 3H), 7.06(d, 2H), 2.72(m, 1H), 1.86-1.43(m, 16H) | 671.85 | 671.93 |
| Compound 14 | 8.06-8.05(m, 2H), 7.96-7.79(m, 9H), 7.60-7.28(m, 16H), 7.18-7.16(m, 3H), 7.06(d, 2H), 2.72(m, 1H), 1.86-1.43(m, 16H) | 747.92 | 748.03 |
| Compound 18 | 8.06(d, 2H), 7.90-7.79(m, 7H), 7.55-7.28(m, 14H), 7.18-7.16(m, 3H), 7.06(d, 2H), 2.62(m, 1H), 2.13-1.88 (m, 5H), 1.69(s, 6H), 1.58-1.31 (m, 5H) | 683.56 | 683.94 |
| Compound 20 | 8.06(d, 2H), 7.90-7.79(m, 7H), 7.55-7.28(m, 14H), 7.16-7.10(m, 5H), 2.05-1.76(m, 15H), 1.69(s, 6H) | 723.88 | 724.00 |
| Compound 24 | 7.90-7.86(m, 2H), 7.55-7.54(m, 3H), 7.38-7.28(m, 7H), 7.18-7.16(m, 3H), 7.06(d, 2H), 2.72(m, 1H), 1.86-1.31 (m, 43H) | 687.75 | 688.06 |
| Compound 35 | 8.06(d, 2H), 7.90-7.79(m, 7H), 7.55-7.28(m, 22H), 7.16(d, 1H), 2.72(m, 1H), 1.86-1.43(m, 16H) | 747.95 | 748.03 |
| Compound 63 | 8.20(d, 2H), 7.90-7.86(m, 2H), 7.55(d, 1H), 7.43-7.06(m, 29H), 2.62(m, 1H), 2.13-1.88 (m, 5H), 1.58-1.31 (m, 5H) | 731.56 | 731.98 |
| Compound 68 | 8.20(d, 2H), 7.90-7.79(m, 9H), 7.55-7.18(m, 22H), 7.06-7.05(m, 3H), 2.72(m, 1H), 1.86-1.43(m, 10H) | 793.85 | 794.05 |
| Compound 77 | 8.23(s, 2H), 8.03-7.98(m, 2H), 7.80-7.75(m, 7H), 7.55-7.31 (m, 16H), 7.18(d, 2H), 7.06(d, 2H), 6.91(d, 1H), 2.72(m, 1H), 1.86-1.43(m, 10H) | 721.62 | 721.94 |
| Compound 91 | 8.03-7.98(m, 4H), 7.83-7.79(m, 6H), 7.55-7.31(m, 13H), 7.18(d, 2H), 7.06(d, 2H), 6.91(d, 1H), 2.62(m, 1H), 1.79-1.38 (m, 12H) | 671.36 | 671.88 |
| Compound 165 | 8.45(d, 1H), 8.10-8.01(m, 4H), 7.93(d, 1H), 7.83-7.79(m, 5H), 7.64(s, 1H), 7.56-7.37(m, 19H), 7.14(m, 1H), 2.72(m, 1H), 1.86-1.43(m, 10H) | 737.87 | 738.01 |
| Compound 172 | 8.45(d, 1H), 8.06-8.01(m, 3H), 7.93(d, 1H), 7.83-7.79(m, 5H), 7.64(s, 1H), 7.56-7.37(m, 21H), 2.62(m, 1H), 2.13-1.88 (m, 5H), 1.58-1.31 (m, 5H) | 749.12 | 749.31 |

### 3. Manufacture and Evaluation of Light Emitting Device Including Amine Compound

### Manufacture of Light Emitting Device

The light emitting device of one or more embodiments including the amine compound of one or more embodiments in a hole transport layer was manufactured as follows. Amine Compounds 4, 14, 18, 20, 24, 35, 63, 68, 77, 91, 165, and 172, which are Example Compounds as described above, were utilized as a hole transport layer material to manufacture the light emitting devices of Examples 1 to 12, respectively, and Compound 4 and Comparative Example Compound C2 and Compound 18 and Comparative Example Compound C2 were utilized as hole transport layer materials to manufacture the light emitting devices of Examples 13 and 14, respectively. Comparative Examples 1 to 9 correspond to the light emitting devices manufactured by utilizing Comparative Example Compounds C1 to C9 as a hole transport layer material, respectively, and Comparative Example 10 corresponds to the light emitting device manufactured by utilizing Comparative Example Compounds C2 and C5 as hole transport layer materials.

### Example Compounds

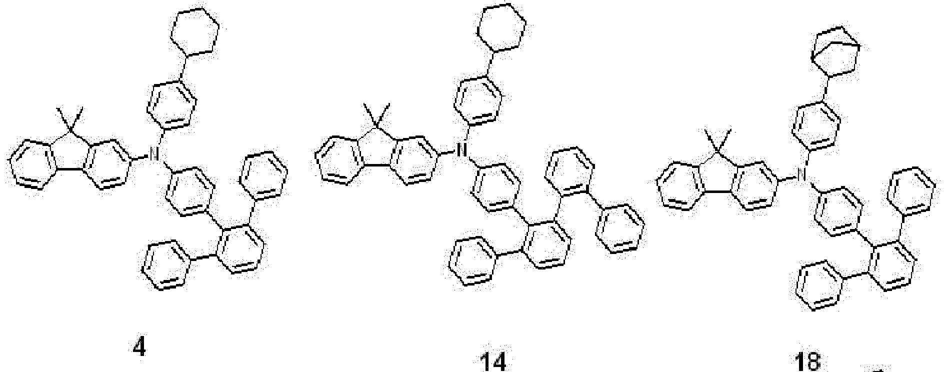

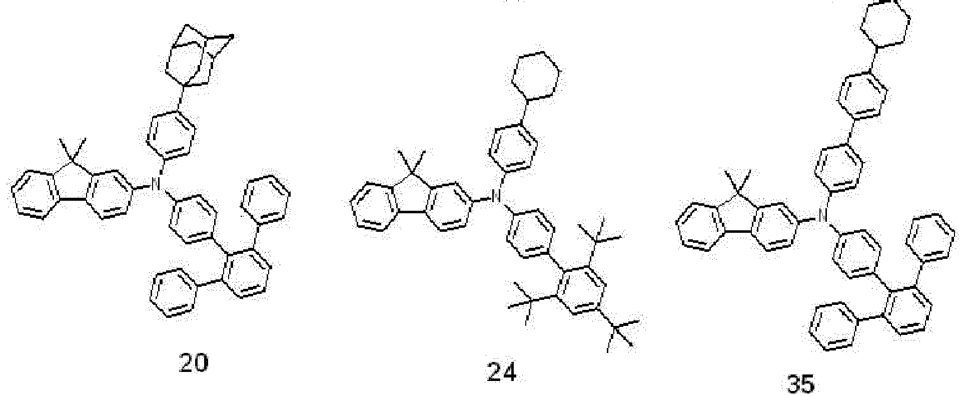

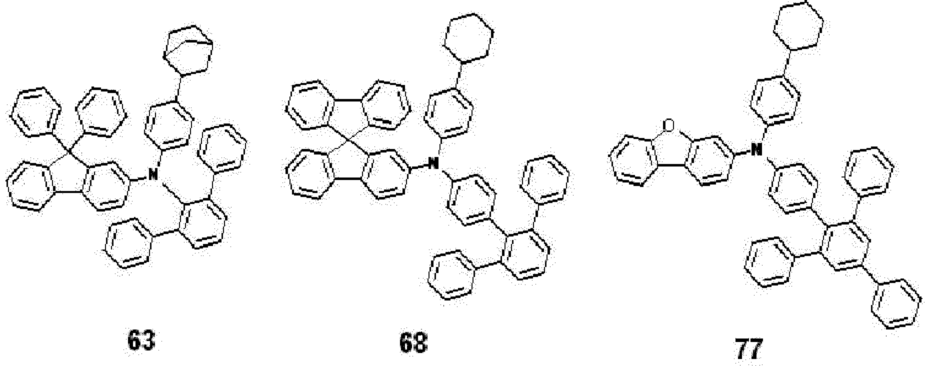

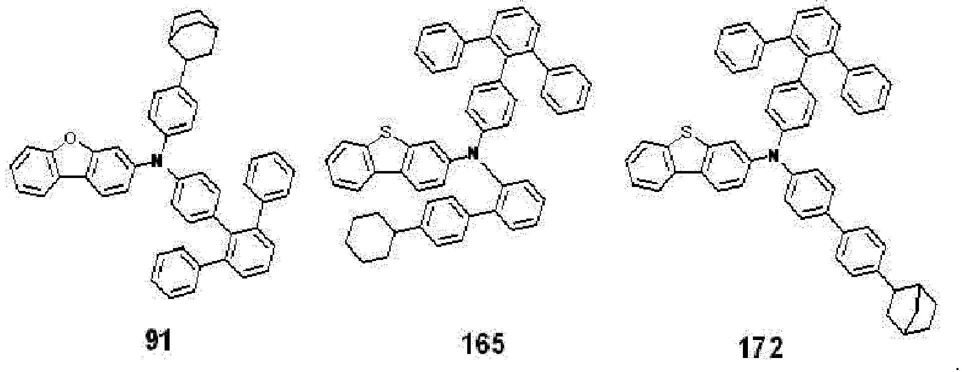

Comparative Example Compounds C1 to C9 were utilized to manufacture devices of Comparative Examples 1 to 10.

### Comparative Example Compounds

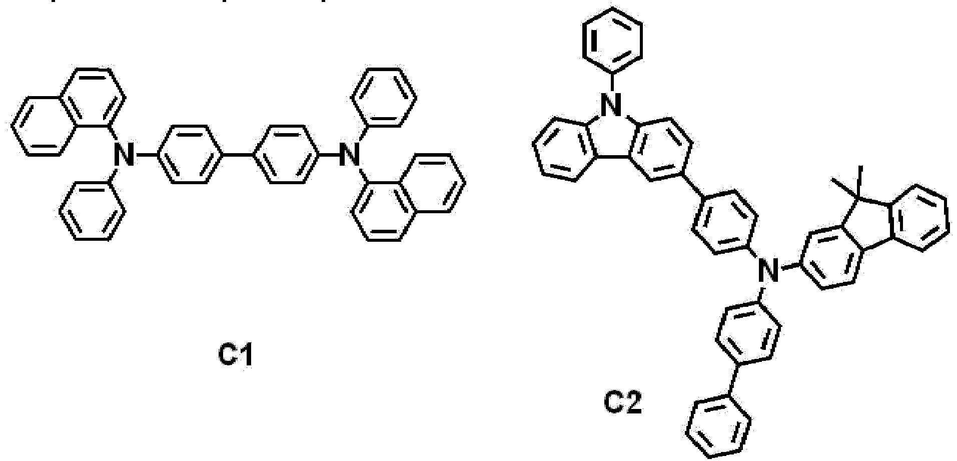

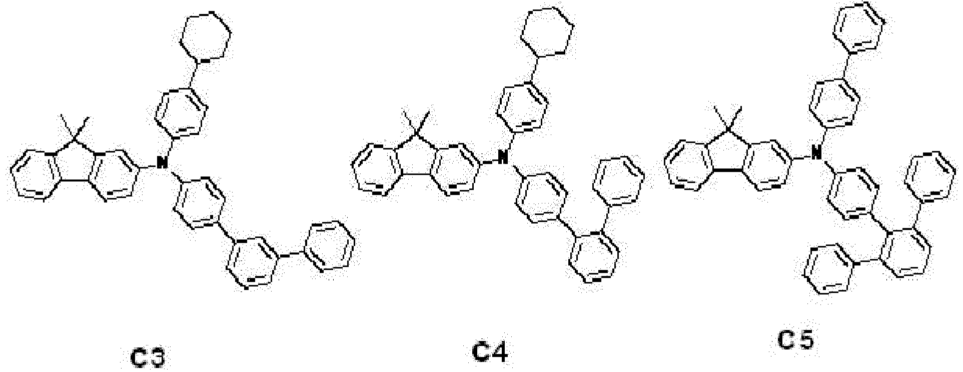

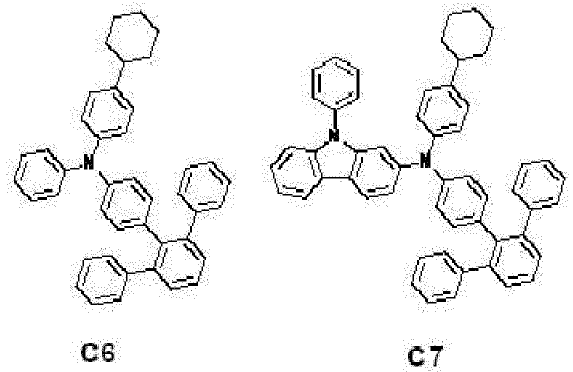

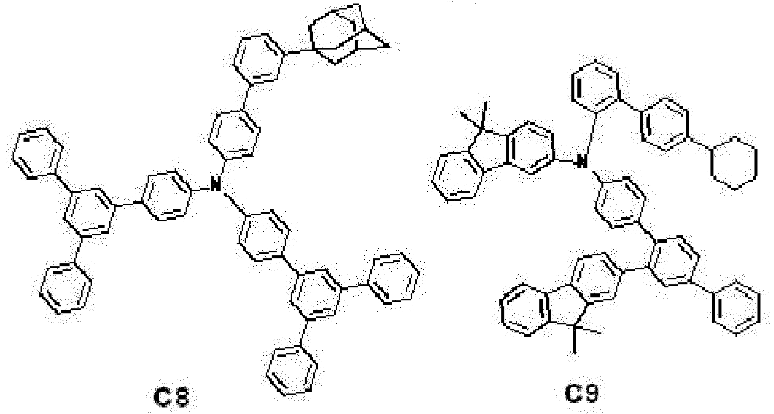

### Example 1

An ITO glass substrate of about 15 Ω/cm² (about 120 nm (1,200 Å)) of Corning Inc. was cut to a size of 50 mm x 50 mm x 0.7 mm, washed with isopropyl alcohol and pure water, and cleansed by ultrasonic waves for about 5 minutes, and then irradiated with ultraviolet rays for about 30 minutes and treated with ozone. Then, 4,4',4"-tris{N,-(2-naphthyl)-N-phenylamino}-triphenylamine (2-TNATA) was deposited in vacuum to form a 60 nm-thick (600 Å-thick) hole injection layer, and Example Compound 4 was deposited in vacuum to form a 30 nm-thick (300 Å-thick) hole transport layer.

On the hole transport layer, 9,10-di(naphthalen-2-yl)anthracene (DNA) and 4,4'-bis[2-(4-(N,N-diphenylamino)phenyl)vinyl]biphenyl (DPAVBi) as blue fluorescent hosts were co-deposited in a ratio of 98:2 to form a 30 nm-thick (300 Å-thick) emission layer.

On the emission layer, a 30 nm-thick (300 Å-thick) electron transport layer was formed with tris(8-hydroxyquinolino)aluminum (Alq₃), and then LiF was deposited to form a 1 nm-thick (10 Å-thick) electron injection layer. On the electron injection layer, a 300 nm-thick (3000 Å-thick) second electrode was formed with aluminum (Al).

Compounds utilized for manufacturing the light emitting devices of Examples and Comparative Examples are disclosed below.

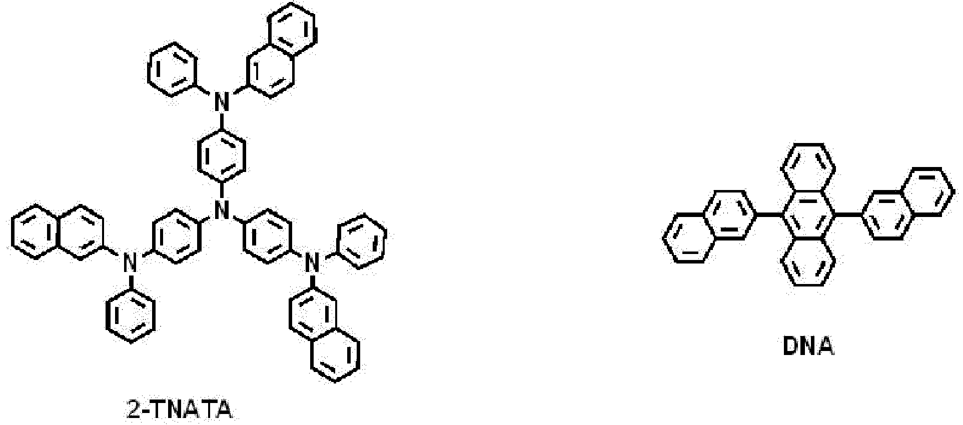

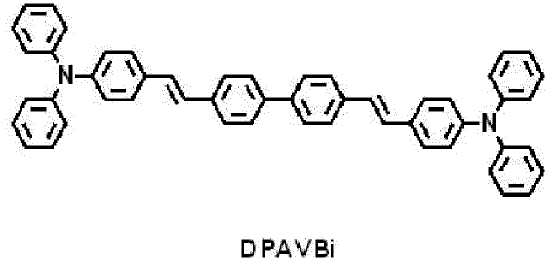

### Example 2

Compared with Example 1, the light emitting device was manufactured in substantially the same manner as Example 1 except for utilizing Example Compound 14 instead of Example Compound 4 when the hole transport layer was formed.

### Example 3

Compared with Example 1, the light emitting device was manufactured in substantially the same manner as Example 1 except for utilizing Example Compound 18 instead of Example Compound 4 when the hole transport layer was formed.

### Example 4

Compared with Example 1, the light emitting device was manufactured in substantially the same manner as Example 1 except for utilizing Example Compound 20 instead of Example Compound 4 when the hole transport layer was formed.

### Example 5

Compared with Example 1, the light emitting device was manufactured in substantially the same manner as Example 1 except for utilizing Example Compound 24 instead of Example Compound 4 when the hole transport layer was formed.

### Example 6

Compared with Example 1, the light emitting device was manufactured in substantially the same manner as Example 1 except for utilizing Example Compound 35 instead of Example Compound 4 when the hole transport layer was formed.

### Example 7

Compared with Example 1, the light emitting device was manufactured in substantially the same manner as Example 1 except for utilizing Example Compound 63 instead of Example Compound 4 when the hole transport layer was formed.

### Example 8

Compared with Example 1, the light emitting device was manufactured in substantially the same manner as Example 1 except for utilizing Example Compound 68 instead of Example Compound 4 when the hole transport layer was formed.

### Example 9

Compared with Example 1, the light emitting device was manufactured in substantially the same manner as Example 1 except for utilizing Example Compound 77 instead of Example Compound 4 when the hole transport layer was formed.

### Example 10

Compared with Example 1, the light emitting device was manufactured in substantially the same manner as Example 1 except for utilizing Example Compound 91 instead of Example Compound 4 when the hole transport layer was formed.

### Example 11

Compared with Example 1, the light emitting device was manufactured in substantially the same manner as Example 1 except for utilizing Example Compound 165 instead of Example Compound 4 when the hole transport layer was formed.

### Example 12

Compared with Example 1, the light emitting device was manufactured in substantially the same manner as Example 1 except for utilizing Example Compound 172 instead of Example Compound 4 when the hole transport layer was formed.

### Example 13

An ITO glass substrate of about 15 Ω/cm² (about 120 nm (1,200 Å)) of Corning Inc. was cut to a size of 50 mm x 50 mm x 0.7 mm, washed with isopropyl alcohol and pure water, and cleansed by ultrasonic waves for about 5 minutes, and then irradiated with ultraviolet rays for about 30 minutes and treated with ozone. Then, 2-TNATA was deposited in vacuum to form a 60 nm-thick (600 Å-thick) hole injection layer.

On the hole injection layer, Example Compound 4 was deposited in vacuum to form a 10 nm-thick (100 Å-thick) first hole transport layer. On the first hole transport layer, Comparative Example Compound C2 was deposited in vacuum to form a 10 nm-thick (100 Å-thick) second hole transport layer, and on the second hole transport layer, Example Compound 13 was deposited in vacuum to form a 10 nm-thick (100 Å-thick) third hole transport layer.

On the hole transport layer, 9,10-di(naphthalen-2-yl)anthracene (DNA) and 4,4'-bis[2-(4-(N,N-diphenylamino)phenyl)vinyl]biphenyl (DPAVBi) as blue fluorescence hosts were co-deposited in a ratio of 98:2 to form a 30 nm-thick (300 Å-thick) emission layer.

On the emission layer, a 30 nm-thick (300 Å-thick) electron transport layer was formed with tris(8-hydroxyquinolino)aluminum (Alq₃), and then LiF was deposited to form a 1 nm-thick (10 Å-thick) electron injection layer. On the electron injection layer, a 300 nm-thick (3,000 Å-thick) second electrode was formed with aluminum (Al).

### Example 14

Compared with Example 13, the light emitting device was manufactured in substantially the same manner as Example 13 except for utilizing Example Compound 18 instead of Example Compound 4 when the first and third hole transport layers were formed.

### Comparative Example 1

Compared with Example 1, the light emitting device was manufactured in substantially the same manner as Example 1 except for utilizing Comparative Example Compound C1 instead of Example Compound 1 when the hole transport layer was formed.

### Comparative Example 2

Compared with Example 1, the light emitting device was manufactured in substantially the same manner as Example 1 except for utilizing Comparative Example Compound C2 instead of Example Compound 1 when the hole transport layer was formed.

### Comparative Example 3

Compared with Example 1, the light emitting device was manufactured in substantially the same manner as Example 1 except for utilizing Comparative Example Compound C3 instead of Example Compound 1 when the hole transport layer was formed.

### Comparative Example 4

Compared with Example 1, the light emitting device was manufactured in substantially the same manner as Example 1 except for utilizing Comparative Example Compound C4 instead of Example Compound 1 when the hole transport layer was formed.

### Comparative Example 5

Compared with Example 1, the light emitting device was manufactured in substantially the same manner as Example 1 except for utilizing Comparative Example Compound C5 instead of Example Compound 1 when the hole transport layer was formed.

### Comparative Example 6

Compared with Example 1, the light emitting device was manufactured in substantially the same manner as Example 1 except for utilizing Comparative Example Compound C6 instead of Example Compound 1 when the hole transport layer was formed.

### Comparative Example 7

Compared with Example 1, the light emitting device was manufactured in substantially the same manner as Example 1 except for utilizing Comparative Example Compound C7 instead of Example Compound 1 when the hole transport layer was formed.

### Comparative Example 8

Compared with Example 1, the light emitting device was manufactured in substantially the same manner as Example 1 except for utilizing Comparative Example Compound C8 instead of Example Compound 1 when the hole transport layer was formed.

### Comparative Example 9

Compared with Example 1, the light emitting device was manufactured in substantially the same manner as Example 1 except for utilizing Comparative Example Compound C9 instead of Example Compound 1 when the hole transport layer was formed.

### Comparative Example 10

Compared with Example 13, the light emitting device was manufactured in substantially the same manner as Example 13 except for utilizing Comparative Example Compound C5 instead of Example Compound 4 when the first and third hole transport layers were formed.

### Evaluation of Light Emitting Device Characteristics

Evaluation results of the light emitting devices of Examples 1 to 14, and Comparative Examples 1 to 10 are listed in Table 2. Brightness, luminous efficiency, and half service life of each of the light emitting devices are listed in Table 2.

In the characteristic evaluation results of Examples and Comparative Examples listed in Table 2, driving voltages and current densities were measured by utilizing V7000 OLED IVL Test System (Polaronix). The luminous efficiency shows an efficiency value measured at a current density of 50 mA/cm². The half service life shows a half service life value measured at a current density of 100 mA/cm².

**Table 2**

| Examples of manufactured devices | Hole transport layer Materials | Driving voltage (V) | Current density (mA/cm²) | Brightness (cd/m²) | Efficiency (cd/A) | Luminous color | Half service life (hr @100 mA/cm²) |
|---|---|---|---|---|---|---|---|
| Example 1 | Compound 4 | 5.05 | 50 | 3105 | 6.21 | Blue | 560 |
| Example 2 | Compound 14 | 4.98 | 50 | 3110 | 6.22 | Blue | 580 |
| Example 3 | Compound 18 | 5.05 | 50 | 3175 | 6.35 | Blue | 600 |
| Example 4 | Compound 20 | 5.02 | 50 | 3175 | 6.35 | Blue | 630 |
| Example 5 | Compound 24 | 4.95 | 50 | 3160 | 6.32 | Blue | 575 |
| Example 6 | Compound 35 | 4.92 | 50 | 3115 | 6.23 | Blue | 520 |
| Example 7 | Compound 63 | 5.03 | 50 | 3130 | 6.26 | Blue | 555 |
| Example 8 | Compound 68 | 5.02 | 50 | 3115 | 6.23 | Blue | 565 |
| Example 9 | Compound 77 | 5.08 | 50 | 3175 | 6.35 | Blue | 600 |
| Example 10 | Compound 91 | 5.05 | 50 | 3060 | 6.12 | Blue | 558 |
| Example 11 | Compound 165 | 5.12 | 50 | 3060 | 6.12 | Blue | 620 |
| Example 12 | Compound 172 | 5.12 | 50 | 3130 | 6.26 | Blue | 590 |
| Example 13 | Compound 4/Comparative Example Compound C2/Compound 4 | 5.18 | 50 | 3260 | 6.52 | Blue | 680 |
| Example 14 | Compound 18/Comparative Example Compound C2/Compound 18 | 5.18 | 50 | 3275 | 6.55 | Blue | 690 |
| Comparative Example 1 | Comparative Example Compound C1 | 7.01 | 50 | 2645 | 5.29 | Blue | 258 |
| Comparative Example 2 | Comparative Example Compound C2 | 5.00 | 50 | 2950 | 5.90 | Blue | 510 |
| Comparative Example 3 | Comparative Example Compound C3 | 5.15 | 50 | 2940 | 5.88 | Blue | 410 |
| Comparative Example 4 | Comparative Example Compound C4 | 5.11 | 50 | 2955 | 5.91 | Blue | 430 |
| Comparative Example 5 | Comparative Example Compound C5 | 5.05 | 50 | 2875 | 5.75 | Blue | 520 |
| Comparative Example 6 | Comparative Example Compound C6 | 5.80 | 50 | 2555 | 5.11 | Blue | 250 |
| Comparative Example 7 | Comparative Example Compound C7 | 5.50 | 50 | 2615 | 5.23 | Blue | 350 |
| Comparative Example 8 | Comparative Example Compound C8 | 5.69 | 50 | 2665 | 5.33 | Blue | 450 |
| Comparative Example 9 | Comparative Example Compound C9 | 5.15 | 50 | 2925 | 5.85 | Blue | 500 |
| Comparative Example 10 | Comparative Example C5/Comparative Example Compound C2/Comparative Example C5 | 5.18 | 50 | 3000 | 6.00 | Blue | 550 |

Referring to the results of Table 2, it may be seen that Examples of the light emitting devices utilizing the amine compound according to one or more embodiments of the present disclosure as a hole transport layer material exhibit low driving voltage, relatively higher brightness, luminous efficiency, and device service life, while emitting the same blue light when compared with Comparative Examples. Moreover, it may be confirmed that Examples 13 and 14 each including the plurality of hole transport layers exhibit improved device characteristics in luminous efficiency and device service life when compared with Comparative Example 10. In some embodiments, for the luminous efficiency characteristic of the light emitting device, it may be confirmed that Examples 13 and 14 each including the plurality of hole transport layers have further improvement in luminous efficiency and device service life when compared with Examples 1 to 12. The Example Compounds include a structure in which a first substituent, a second substituent, and a third substituent are linked to a core nitrogen atom, the first substituent has a fluorenyl moiety, a dibenzofuranyl moiety, or a dibenzothiophenyl moiety, the second substituent has a structure in which an aliphatic cycloalkyl group such as a cyclohexyl group, a bicycloheptanyl group, a bicyclooctanyl group, a bicyclononanyl group, and/or an adamantyl group is linked via an arylene or heteroarylene linker, and the third substituent has a structure in which at least two additional substituents are bonded to a phenyl group, and the additional substituents bonded to the phenyl group are each independently a silyl group, an alkyl group, an aryl group, or a heteroaryl group. The amine compound of one or more embodiments having such a structure has an improvement in hole transport ability while having a low refractive characteristic (e.g., refraction of light may be reduced), and thus the application of the amine compound to the light emitting device may achieve luminous efficiency improvement and long service life of the light emitting device. In one or more embodiments, the light emitting device includes the amine compound as a hole transport layer material of the light emitting device, thereby improving efficiency and a service life of the light emitting device.

Comparative Example Compounds C1 and C2 included in Comparative Examples 1 and 2, respectively, do not include the first substituent, the second substituent, and the third substituent linked to the core nitrogen atom. Accordingly, it may be confirmed that when the Comparative Example Compounds are applied to the light emitting devices, the brightness, luminous efficiency, and half service life are reduced compared with Example Compounds.

Comparative Example Compounds C3, C4, and C9 included in Comparative Examples 3, 4, and 9, respectively, include only one additional substituent at the phenyl group, unlike the third substituent included in the amine compound of the present disclosure. Accordingly, it may be confirmed that when the Comparative Example Compounds are applied to the light emitting devices, the brightness, luminous efficiency, and half service life are reduced compared with Example Compounds.

Comparative Example Compound C5 included in Comparative Examples 5 and 10 does not include an aliphatic cycloalkyl group such as a cyclohexyl group, a bicycloheptanyl group, a bicyclooctanyl group, a bicyclononanyl group, and/or an adamantyl group, unlike the second substituent included in the amine compound of the present disclosure. Accordingly, it may be confirmed that when the Comparative Example Compounds are applied to the light emitting devices, the brightness, luminous efficiency, and half service life are reduced compared with Example Compounds.

Comparative Example Compounds C6 to C8 included in Comparative Examples 6 to 8, respectively, do not include the first substituent included in the amine compound of the present disclosure, that is, the fluorenyl moiety, the dibenzofuranyl moiety, or the dibenzothiophenyl moiety. Accordingly, it may be confirmed that when the Comparative Example Compounds are applied to the light emitting devices, the brightness, luminous efficiency, and half service life are reduced compared with Example Compounds.

The light emitting device of one or more embodiments may exhibit improved device characteristics with high efficiency and a long service life.

The amine compound of one or more embodiments may be included in a hole transport region of the light emitting device to contribute to high efficiency and a long service life of the light emitting device.

Although the present disclosure has been described with reference to embodiments of the present disclosure, it will be understood that the present disclosure should not be limited to these embodiments but one or more suitable changes and modifications can be made by those skilled in the art without departing from the scope of the present claims.

The display device and/or any other relevant devices or components according to embodiments of the present invention described herein may be implemented utilizing any suitable hardware, firmware (e.g. an application-specific integrated circuit), software, or a combination of software, firmware, and hardware.
For example, the various components of the device may be formed on one integrated circuit (IC) chip or on separate IC chips. Further, the various components of the device may be implemented on a flexible printed circuit film, a tape carrier package (TCP), a printed circuit board (PCB), or formed on one substrate. Further, the various components of the device may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer readable media such as, for example, a CD-ROM, flash drive, or the like. Also, a person of skill in the art should recognize that the functionality of various computing devices may be combined or integrated into a single computing device, or the functionality of a particular computing device may be distributed across one or more other computing devices without departing from the scope of the embodiments of the present disclosure.

Accordingly, the technical scope of the present disclosure is not intended to be limited to the contents set forth in the detailed description of the specification, but is intended to be defined by the appended claims.

## Claims

1. An amine compound represented by Formula 1: 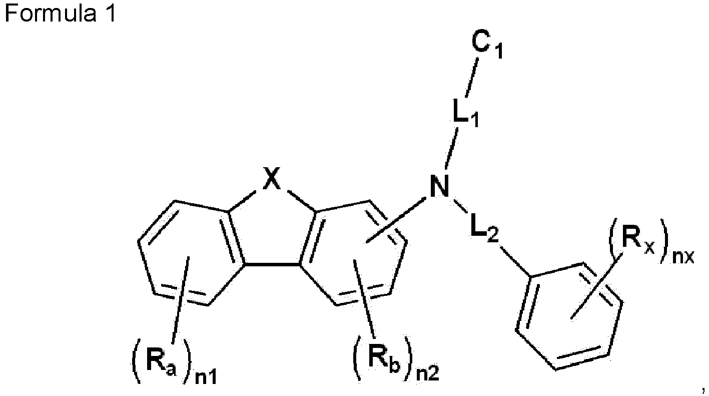
wherein, in Formula 1,
X is CR₁R₂, O, or S,
C₁ is a substituted or unsubstituted cycloalkyl group having 6 to 12 ring-forming carbon atoms,
L₁ and L₂ are each independently a substituted or unsubstituted arylene group having 6 to 30 ring-forming carbon atoms, or a substituted or unsubstituted heteroarylene group having 2 to 30 ring-forming carbon atoms,
R₁, R₂, Rₐ, and R_{b} are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or are bonded to an adjacent group to form a ring,
Rₓ is a substituted or unsubstituted silyl group, a substituted or unsubstituted alkyl group having 1 to 20 carbon atoms, a substituted or unsubstituted aryl group having 6 to 30 ring-forming carbon atoms, or a substituted or unsubstituted heteroaryl group having 6 to 30 ring-forming carbon atoms,
nx is an integer of 2 to 5,
n1 is an integer of 0 to 4, and
n2 is an integer of 0 to 3.

2. The amine compound of claim 1, wherein the amine compound represented by Formula 1 is a monoamine compound.

3. The amine compound of claim 1, wherein the amine compound represented by Formula 1 is represented by Formula 1-1: 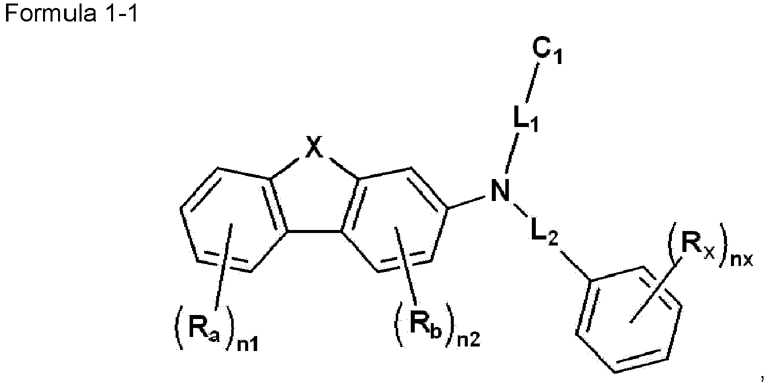 and
wherein, in Formula 1-1,
X, C₁, L₁, L₂, R₁, R₂, Rₐ, R_{b}, Rₓ, nx, n1, and n2 are the same as defined in Formula 1.

4. The amine compound of claim 1, wherein the amine compound represented by Formula 1 is represented by any one selected from among Formula 2-1 to Formula 2-3: 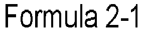 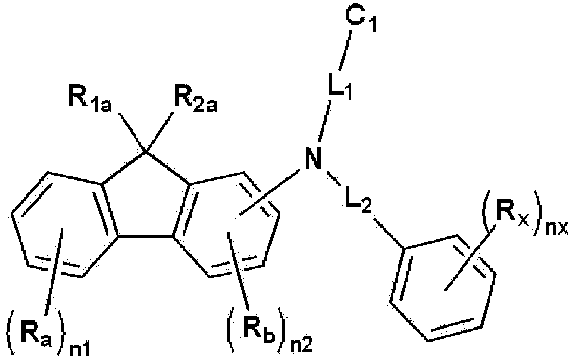 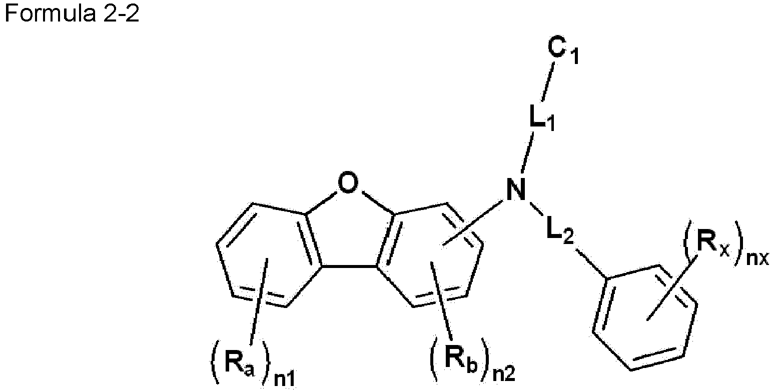 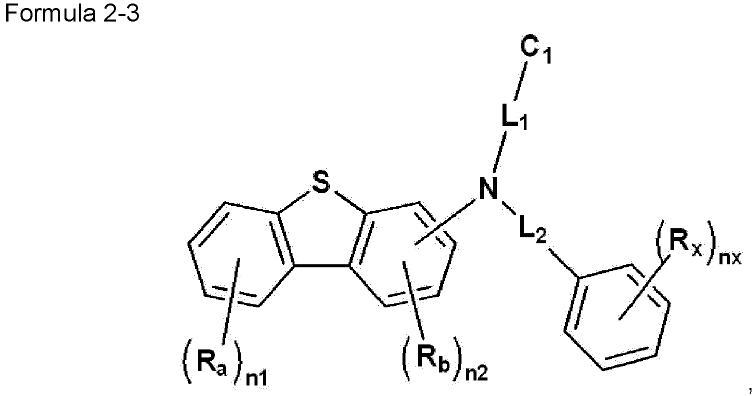 and
wherein, in Formula 2-1 to Formula 2-3,
R₁ₐ and R₂ₐ are each independently a substituted or unsubstituted alkyl group having 1 to 20 carbon atoms, or a substituted or unsubstituted aryl group having 6 to 30 ring-forming carbon atoms, and/or are bonded to an adjacent group to form a ring, and
C₁, L₁, L₂, R₁, R₂, Rₐ, R_{b}, Rₓ, nx, n1, and n2 are the same as defined in Formula 1.

5. The amine compound of claim 1, wherein the amine compound represented by Formula 1 is represented by Formula 3: 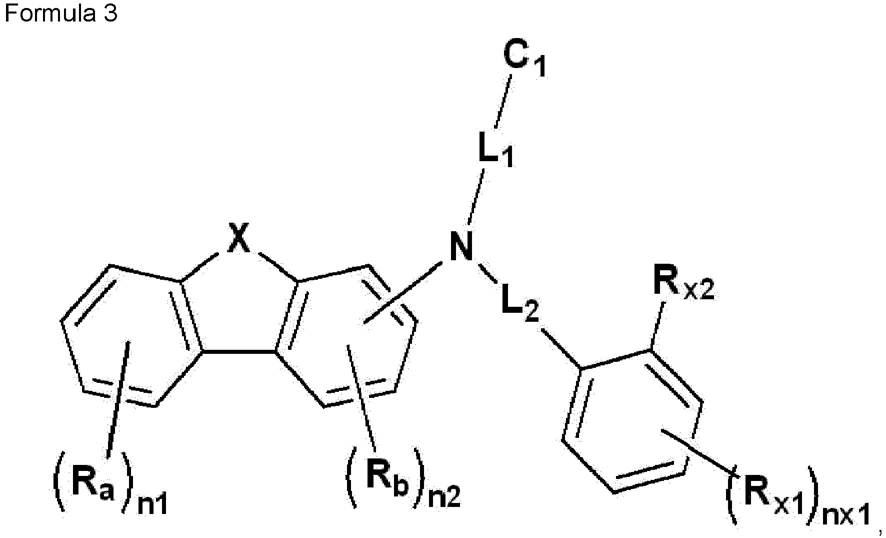 and
wherein, in Formula 3,
Rₓ₁ and Rₓ₂ are each independently a substituted or unsubstituted silyl group, a substituted or unsubstituted alkyl group of 1 to 20 carbon atoms, a substituted or unsubstituted aryl group of 6 to 30 ring-forming carbon atoms, or a substituted or unsubstituted heteroaryl group of 6 to 30 ring-forming carbon atoms,
nx1 is an integer of 1 to 4, and
X, C₁, L₁, L₂, R₁, R₂, Rₐ, R_{b}, n1, and n2 are the same as defined in Formula 1.

6. The amine compound of at least one of claims 1 to 5, wherein C₁ is a substituted or unsubstituted cyclohexyl group, a substituted or unsubstituted bicycloheptanyl group, a substituted or unsubstituted bicyclooctanyl group, a substituted or unsubstituted bicyclononanyl group, or a substituted or unsubstituted adamantyl group.

7. The amine compound of at least one of claims 1 to 6, wherein Rₓ is a substituted or unsubstituted trimethylsilyl group, a substituted or unsubstituted isopropyl group, a substituted or unsubstituted t-butyl group, a substituted or unsubstituted cyclohexyl group, a substituted or unsubstituted bicycloheptanyl group, a substituted or unsubstituted adamantyl group, a substituted or unsubstituted phenyl group, a substituted or unsubstituted biphenyl group, or a substituted or unsubstituted naphthyl group.

8. The amine compound of at least one of claims 1 to 7, wherein L₁ and L₂ are each independently represented by any one selected from among Formula 4-1 to Formula 4-10: 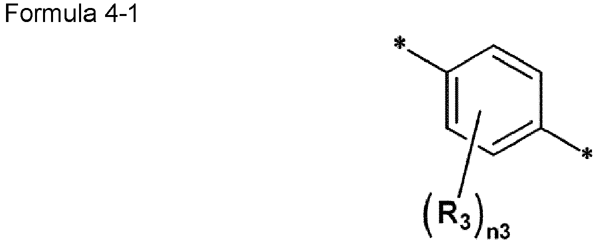 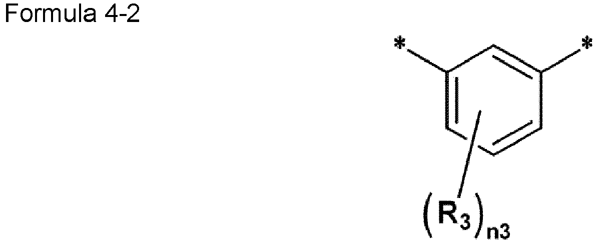 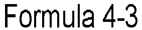 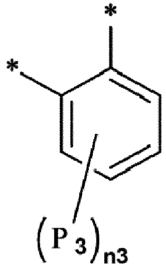 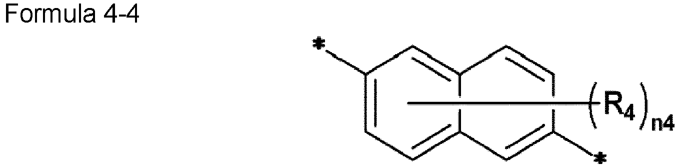 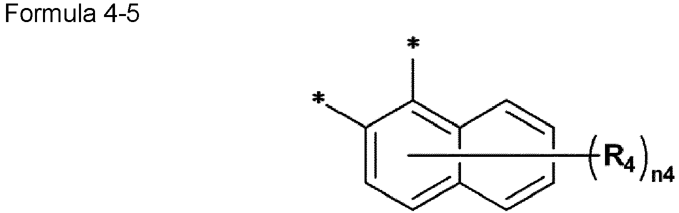 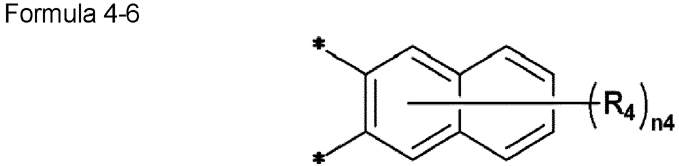 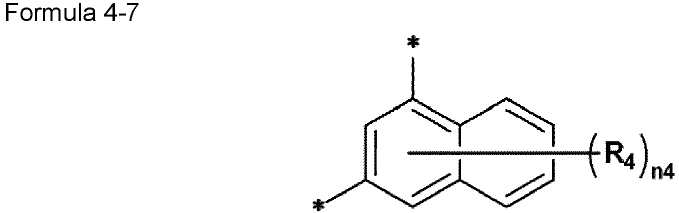 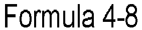 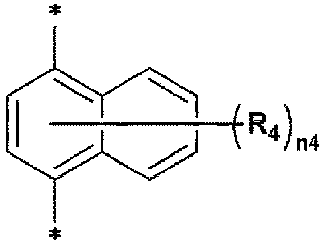 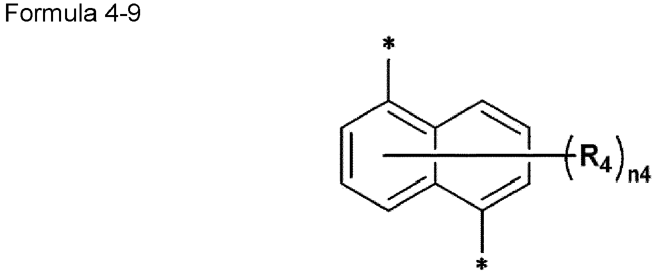 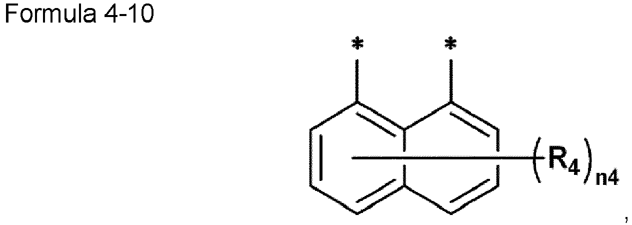 and
wherein, in Formula 4-1 to Formula 4-10,
R₃ and R₄ are each independently a hydrogen atom, a deuterium atom, a halogen atom, a substituted or unsubstituted alkyl group having 1 to 20 carbon group, a substituted or unsubstituted aryl group having 6 to 30 ring-forming carbon atoms, or a substituted or unsubstituted heteroaryl group having 2 to 30 ring-forming carbon atoms,
n3 is an integer of 0 to 4, and
n4 is an integer of 0 to 6.

9. The amine compound of claim 1, 6, 7 or 8, wherein the amine compound represented by Formula 1 is at least one selected from among compounds represented by Compound Group 1:

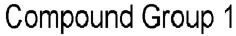

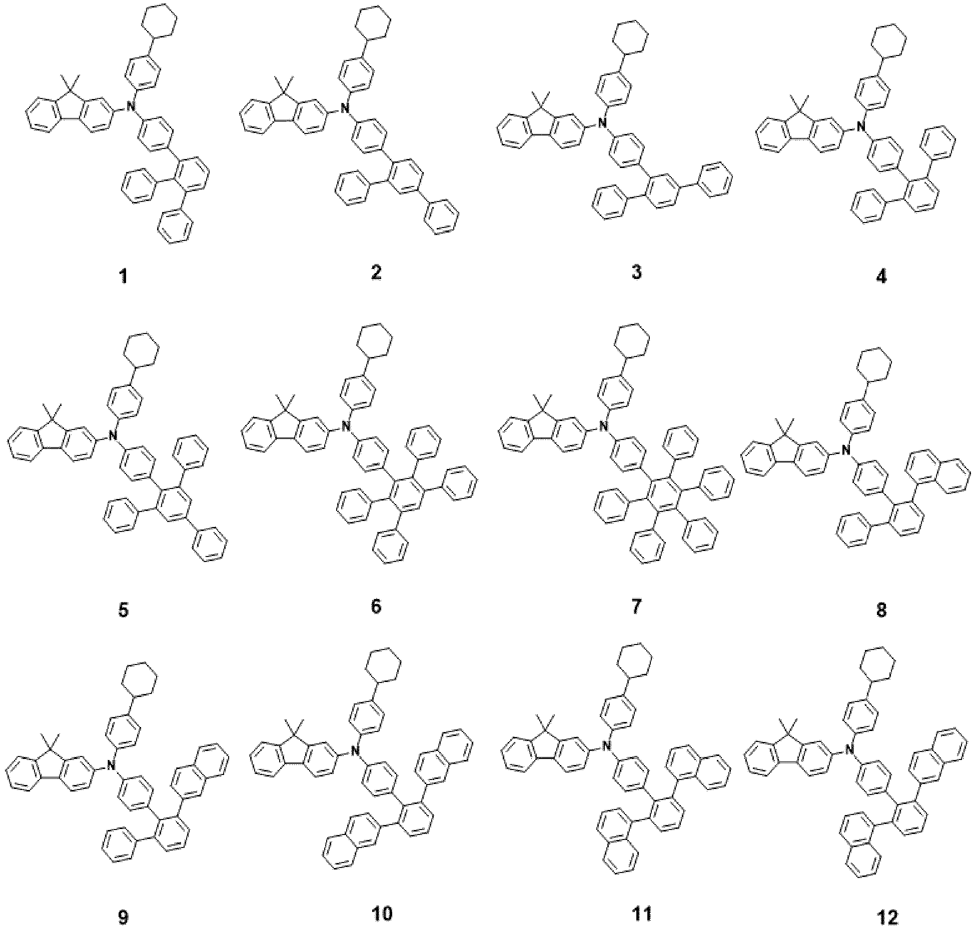

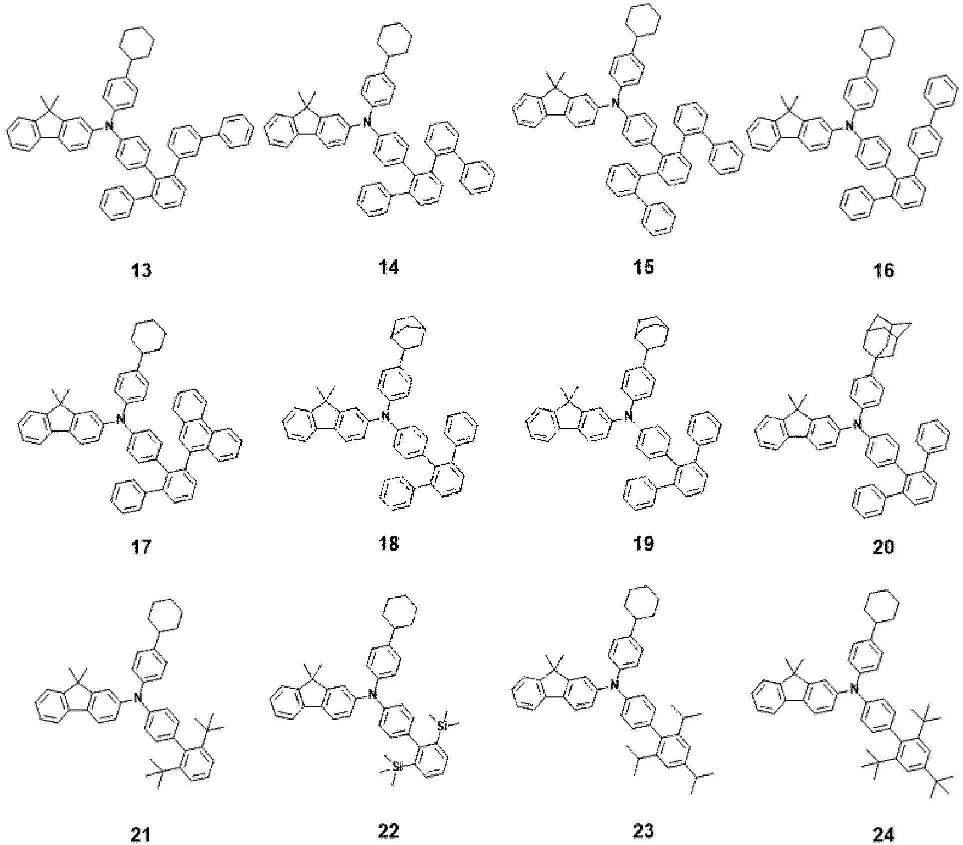

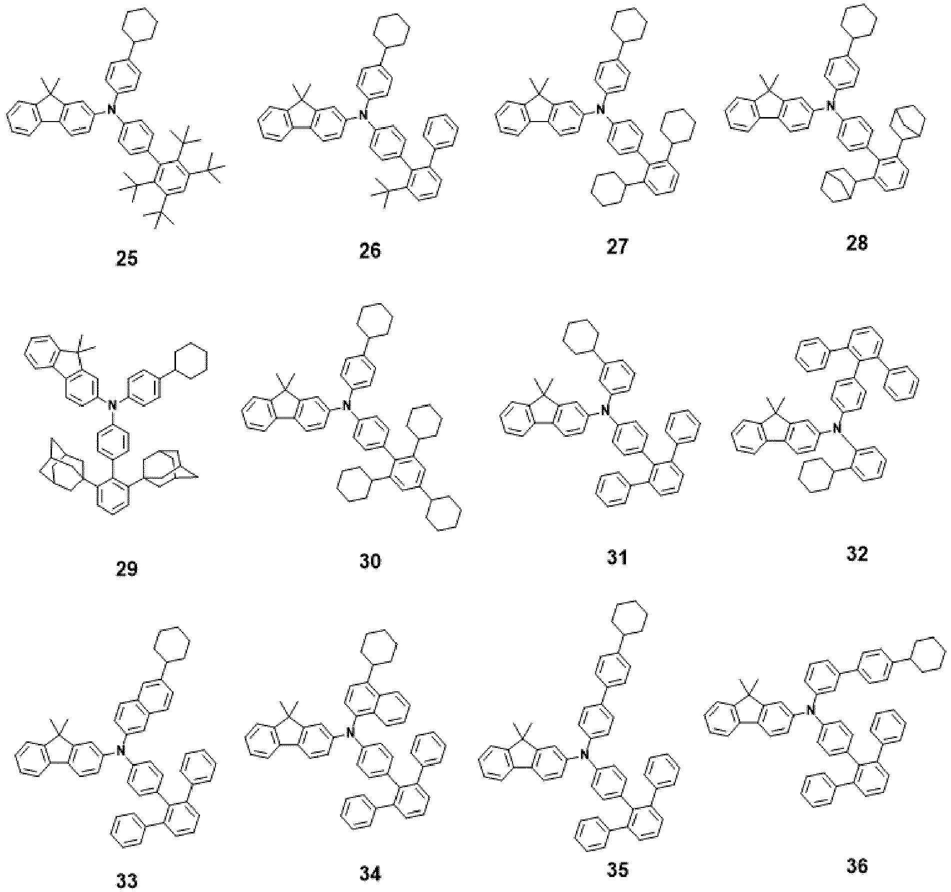

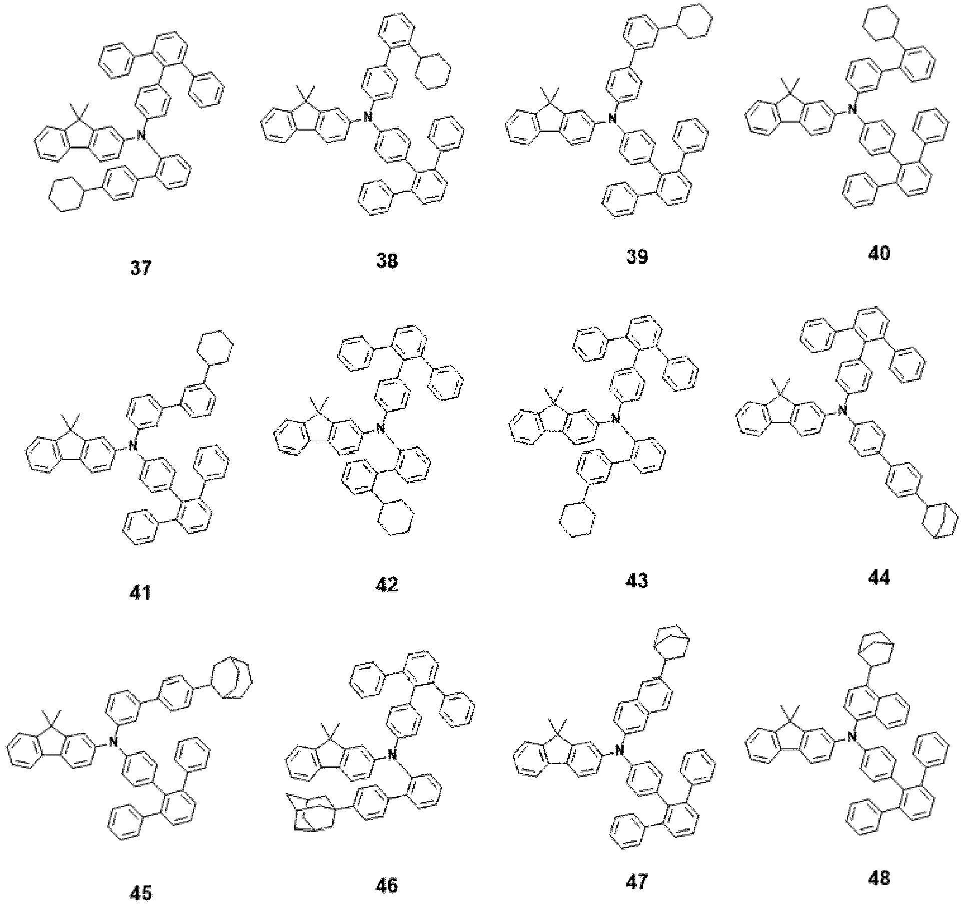

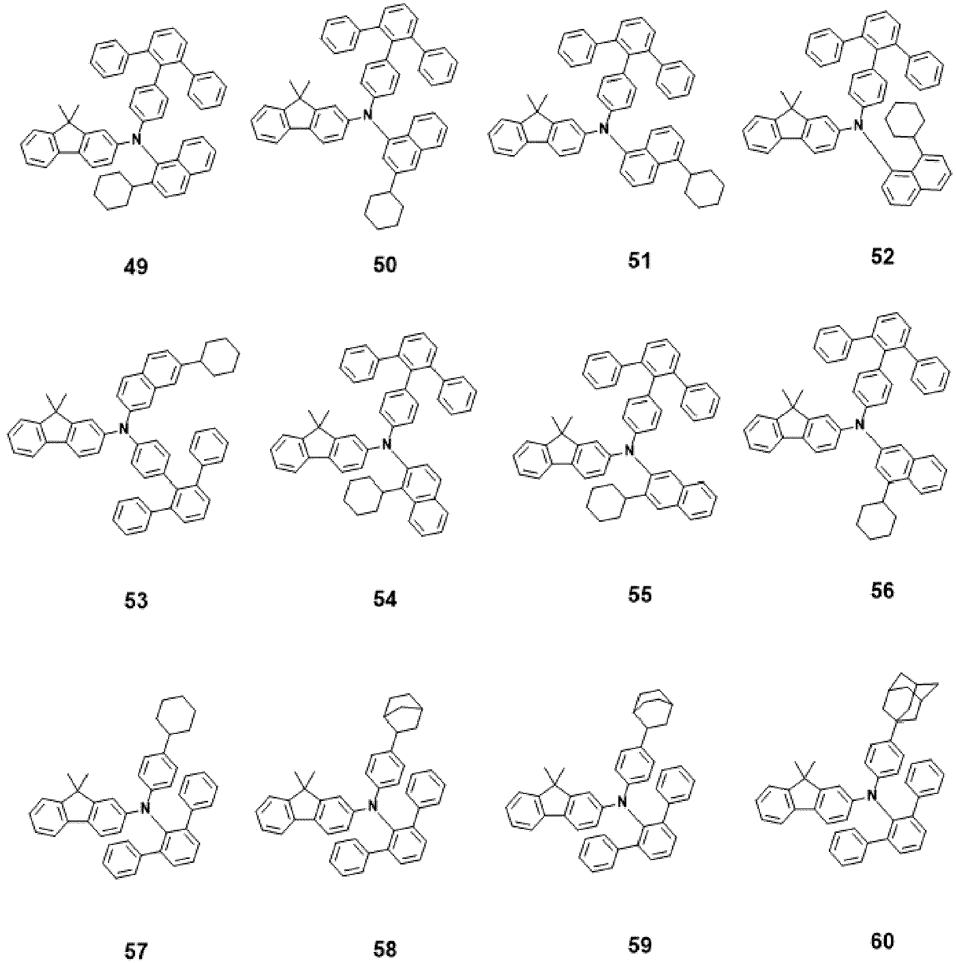

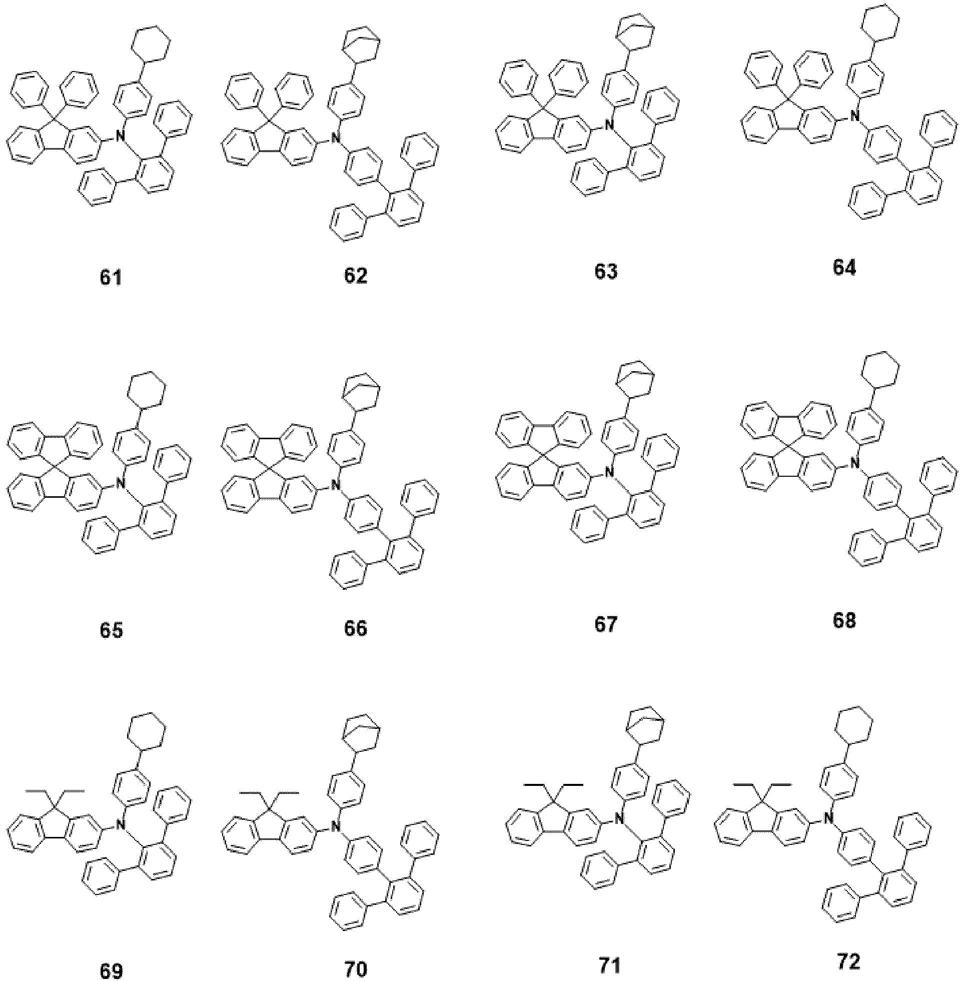

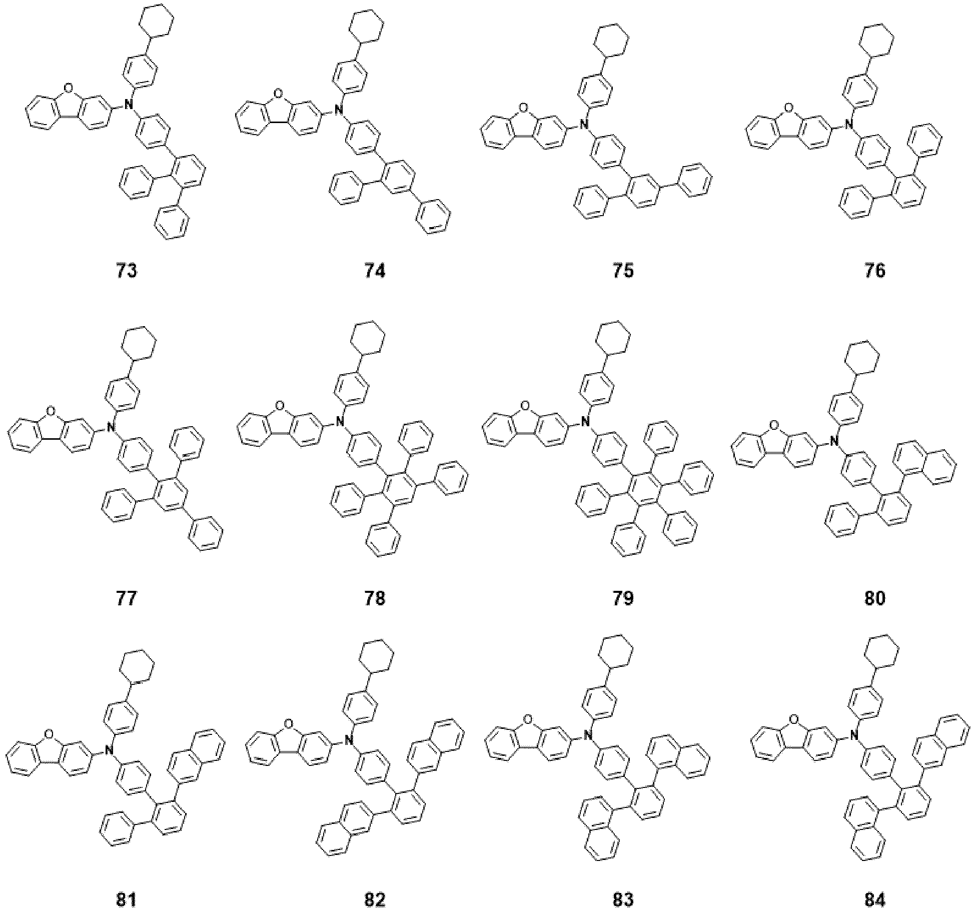

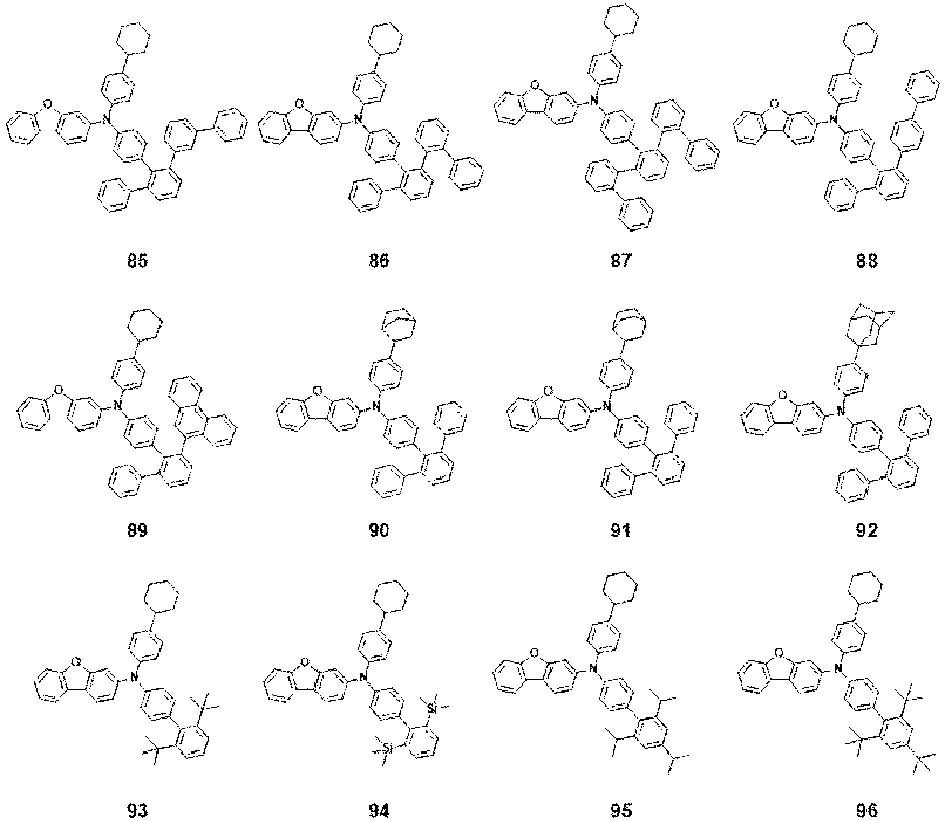

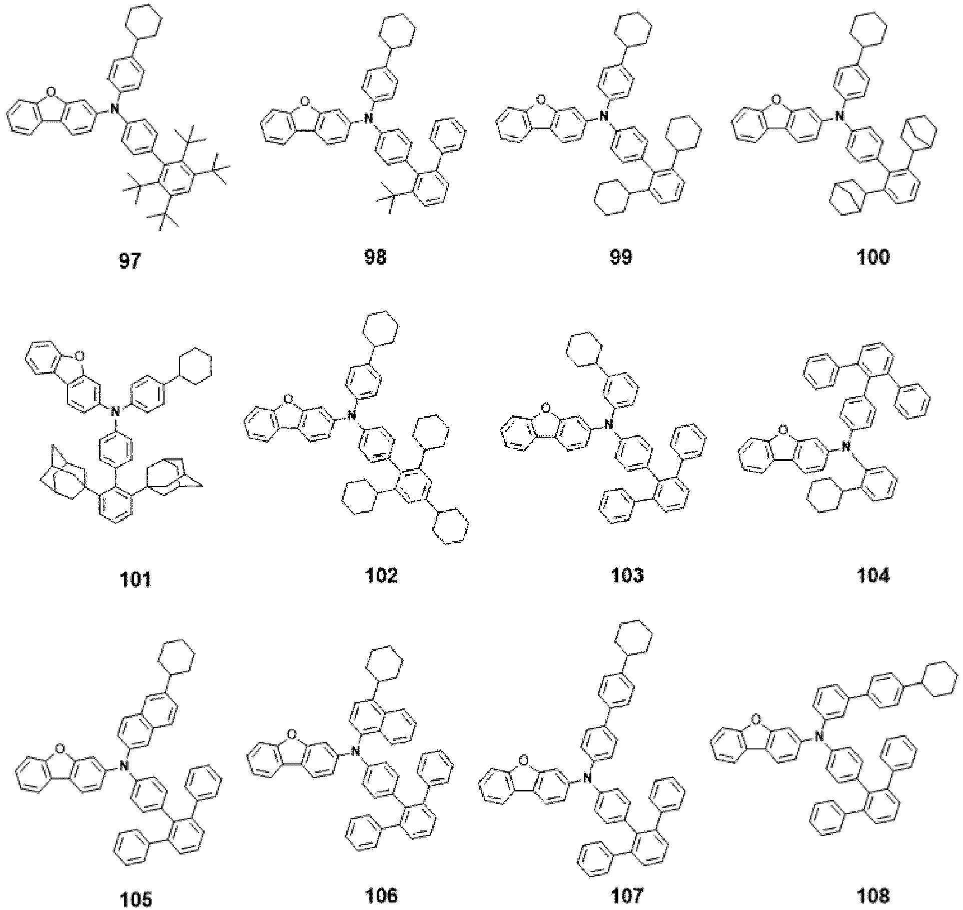

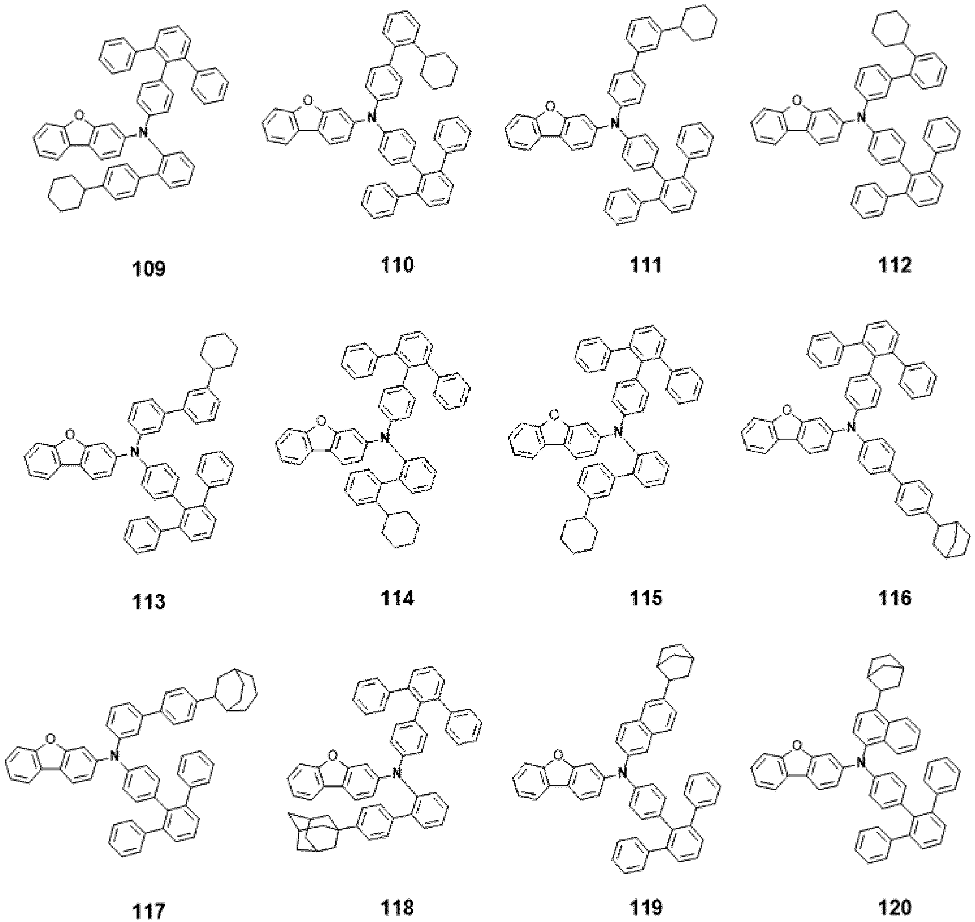

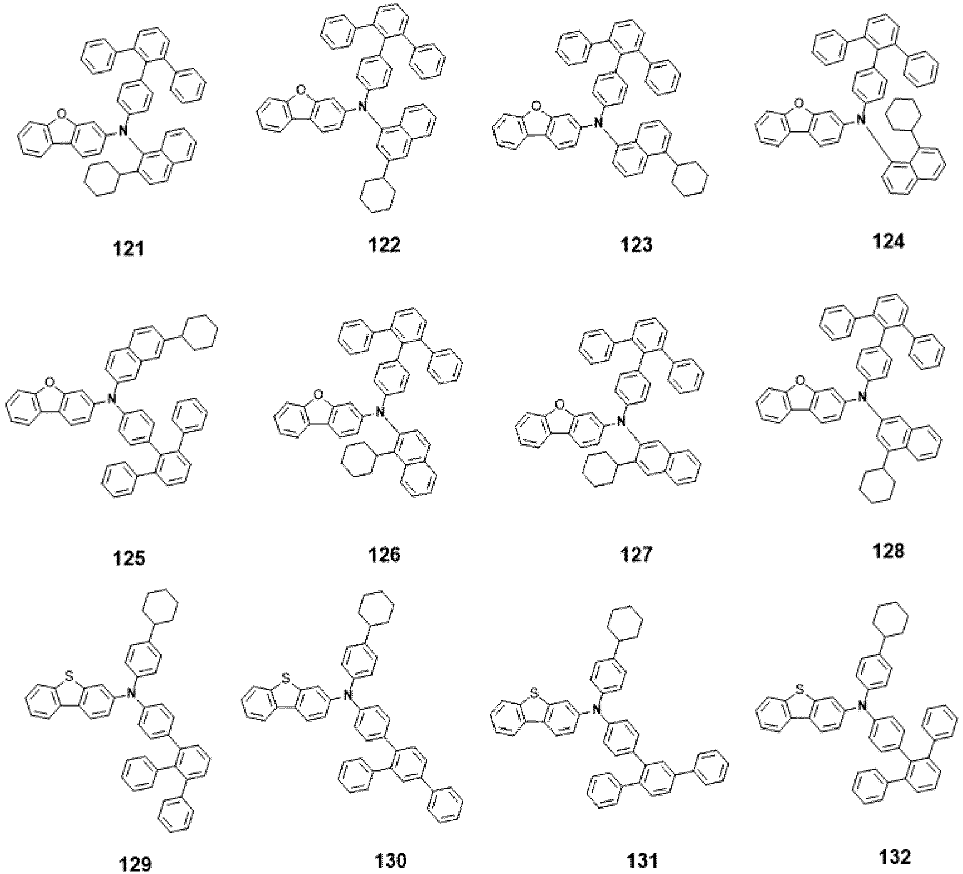

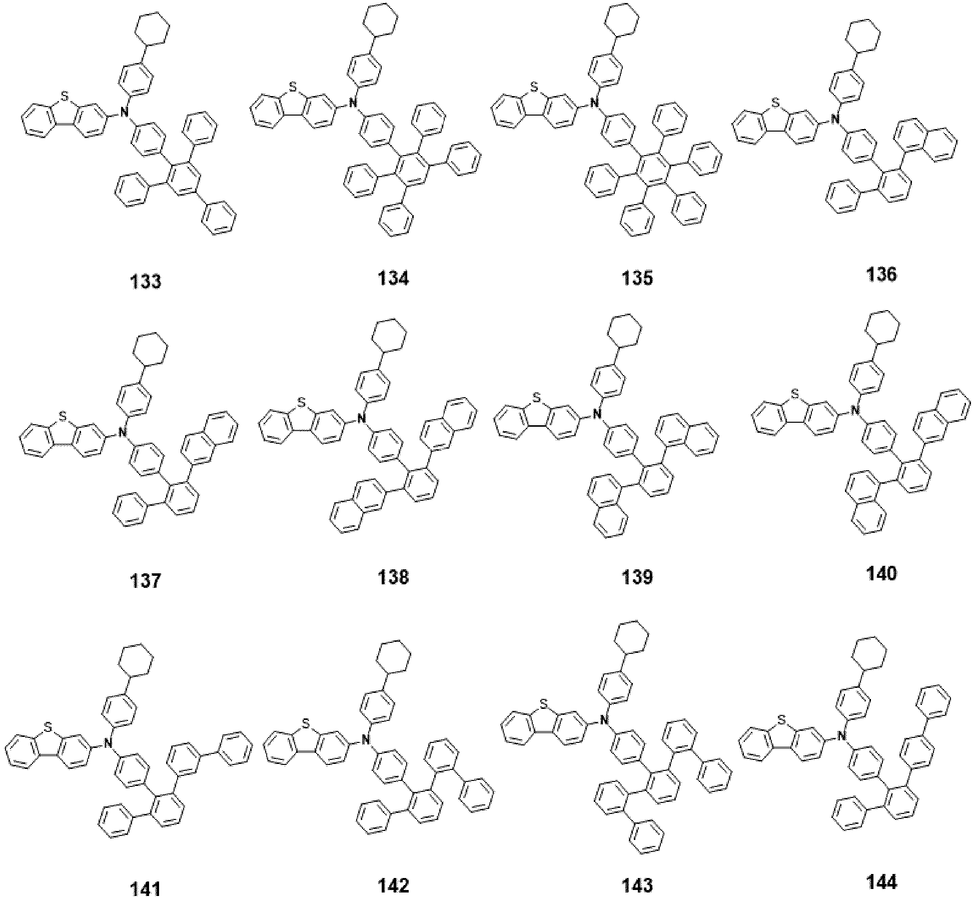

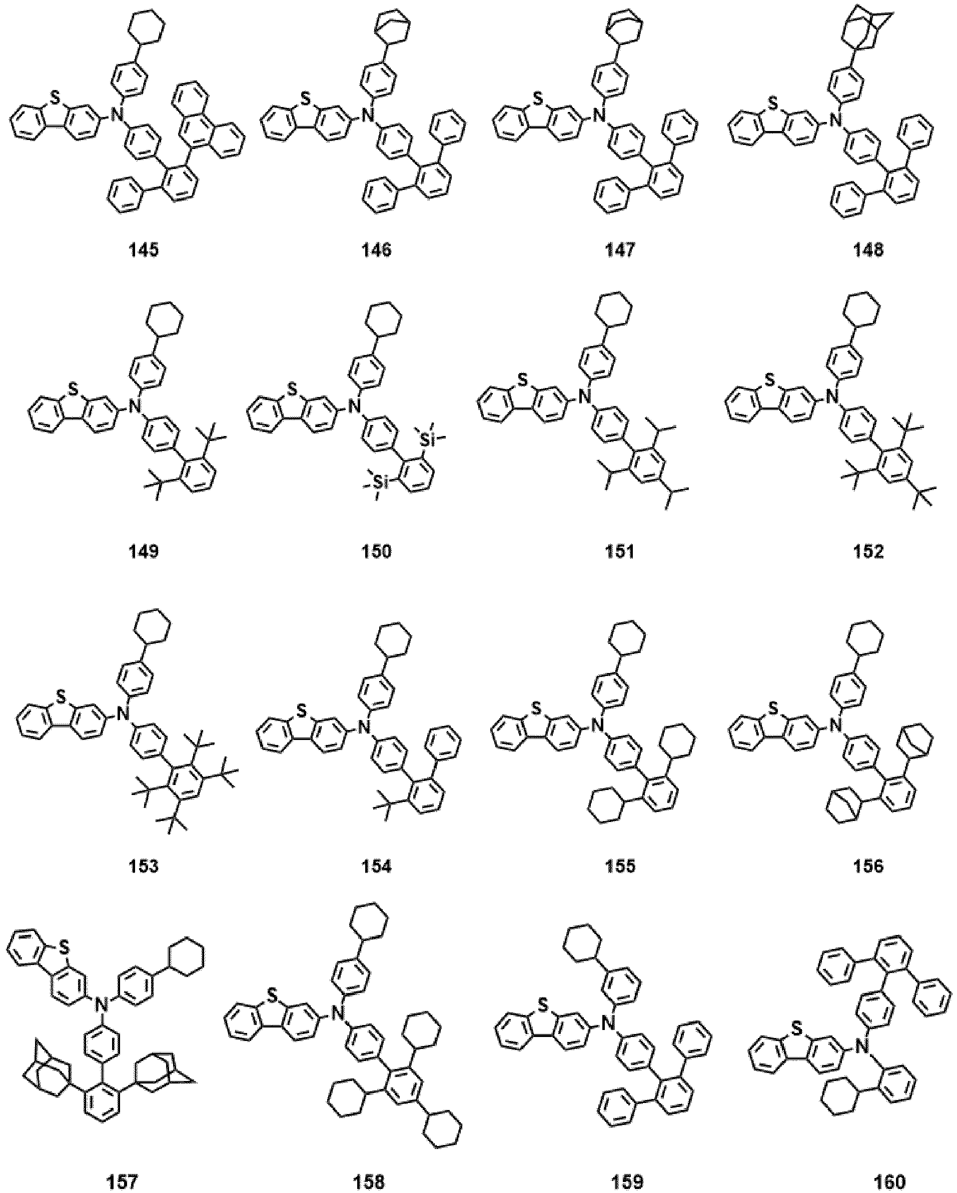

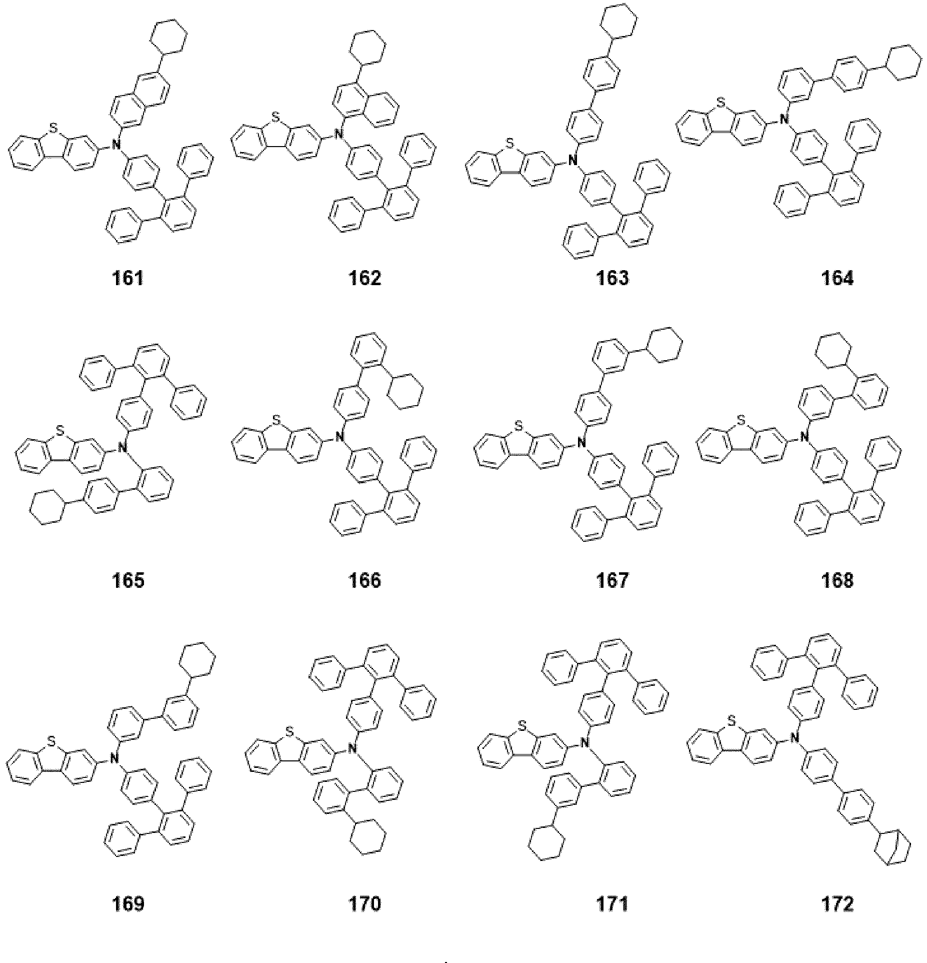

10. A light emitting device (ED) comprising:
a first electrode (EL1);
a second electrode (EL2) on the first electrode (EL1); and
at least one functional layer between the first electrode (EL1) and the second electrode (EL2) and comprising an amine compound according to any one of claims 1 to 9.

11. The light emitting device (ED) of claim 10, wherein:
the at least one functional layer comprises an emission layer (EML), a hole transport region (HTR) between the first electrode (EL1) and the emission layer (EML), and an electron transport region (ETR) between the emission layer (EML) and the second electrode (EL2); and
the hole transport region (HTR) comprises the amine compound according to any one of claims 1 to 9.

12. The light emitting device (ED) of claim 10 or 11, wherein:
the at least one functional layer comprises an emission layer (EML), a first hole transport layer (HTL1) between the first electrode (EL1) and the emission layer (EML), a second hole transport layer (HTL2) between the first hole transport layer (HTL1) and the emission layer (EML), and an electron transport region (ETR) between the emission layer (EML) and the second electrode (EL2); and
the first hole transport layer (HTL1) comprises the amine compound according to any one of claims 1 to 9.

13. The light emitting device (ED) of claim 12, wherein:
the at least one functional layer further comprises a third hole transport layer (HTL3) between the second hole transport layer (HTL2) and the emission layer (EML); and
the third hole transport layer (HTL3) comprises the amine compound according to any one of claims 1 to 9.

14. The light emitting device (ED) of claim 12 or 13, wherein the second hole transport layer (HTL2) comprises an amine derivative compound represented by Formula 5: 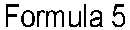 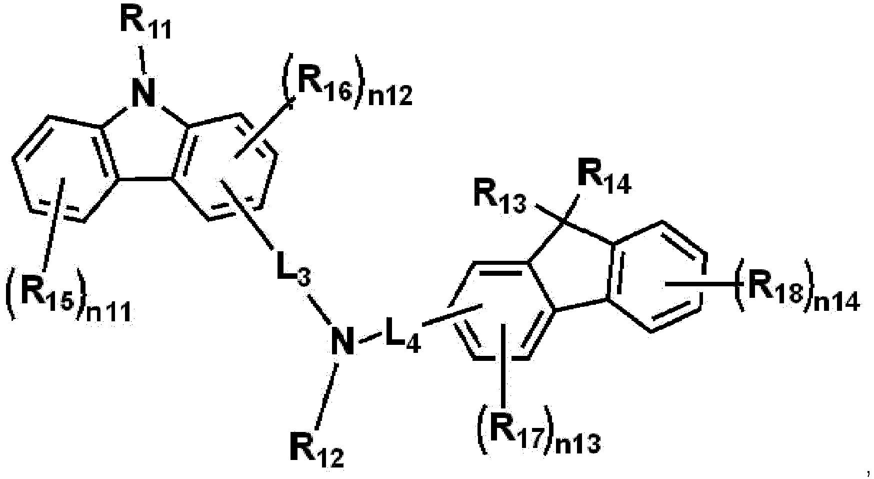 and
wherein, in Formula 5,
L₃ and L₄ are each independently a direct linkage, a substituted or unsubstituted arylene group having 6 to 30 ring-forming carbon atoms, or a substituted or unsubstituted heteroarylene group having 2 to 30 ring-forming carbon atoms,
R₁₁ to R₁₄ are each independently a substituted or unsubstituted alkyl group having 1 to 10 carbon group, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, and/or are bonded to an adjacent group to form a ring,
R₁₅ to R₁₈ are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or are bonded to an adjacent group to form a ring,
n11 and n14 are each independently an integer of 0 to 4, and
n12 and n13 are each independently an integer of 0 to 3.

15. The light emitting device (ED) of claim 14, wherein the amine derivative compound represented by Formula 5 is represented by Formula 6-1 or Formula 6-2: 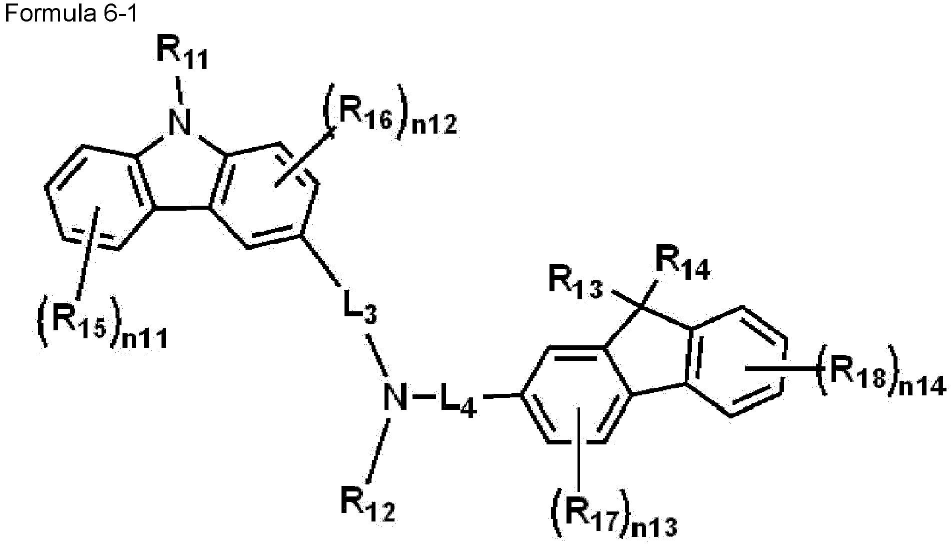 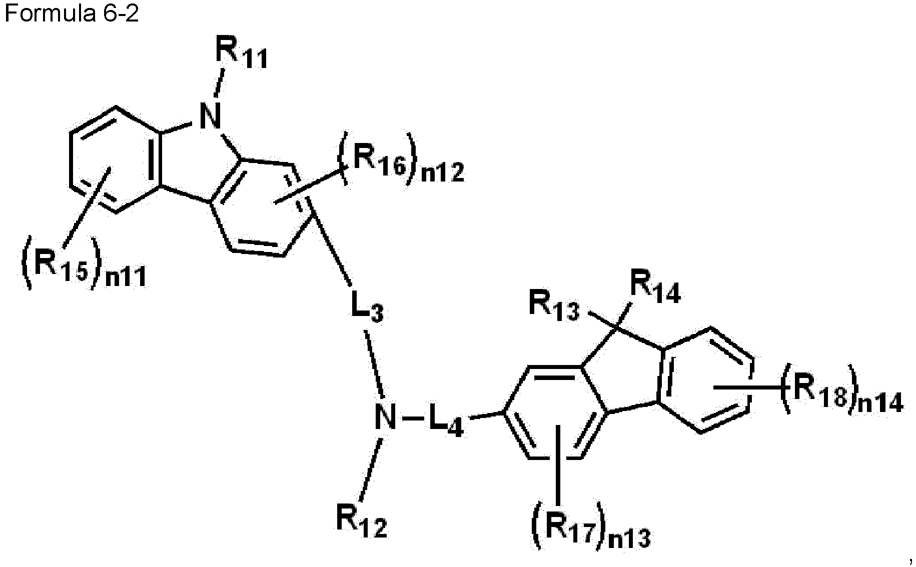 and
wherein, in Formula 6-1 and Formula 6-2, L₃, L₄, R₁₁ to R₁₄, R₁₅ to R₁₈, and n11 to n13 are the same as defined in Formula 5.
